# EUROPÄISCHE PATENTANMELDUNG

(11) **EP 1 844 805 A1**
(43) Veröffentlichungstag der Anmeldung: **17.10.2007**
(21) Anmeldenummer: 06007767.4
(22) Anmeldetag: 13.04.2006
(51) Int. Cl.: A61M 15/00

(54) **Inhalator**

(71) Anmelder: Boehringer Ingelheim Pharma GmbH & Co.KG, 55216 Ingelheim am Rhein (DE)
(72) Erfinder: Besseler, Jens, 44319 Dortmund (DE); Kunze, Hubert, 44227 Dortmund (DE); Moser, Achim, 65483 Sulzbach (DE); Thoemmes, Ralf, 47877 Willich (DE); Wuttke, Gilbert, 44149 Dortmund (DE); Hochrainer, Dieter, 57392 Schmallenberg (DE); Kladders, Heinrich, 45468 Mülheim-Ruhr (DE); Dworzak, Christoph, 3503 Biel (CH); Eckert, Josef, 97638 Mellrichstadt (DE); Lanci, Antonino, 3006 Bern (CH); Mast, Markus, 2502 Biel (CH); Mock, Elmar, 2013 Comlombier (CH); Klopfenstein, André, 2520 Le Neuveville (CH)
(74) Vertreter: Gesthuysen, von Rohr & Eggert

(57) **Zusammenfassung**

Es wird ein Inhalator (1) zur Inhalation einer Formulierung (2) aus Kapseln (3) vorgeschlagen, die jeweils eine Dosis der Formulierung enthalten. Die Kapseln werden vorzugsweise jeweils in einer Kapselkammer (4) dadurch entleert, daß sie von einem durch die Kapselkammer strömenden Luftstrom in Bewegung versetzt werden. Der Luftstrom kann durch das Einatmen eines Benutzers bzw. Patienten und/oder aktiv durch den Inhalator erzeugt werden. Zur einfachen Handhabung weist der Inhalator eine Einrichtung zur insbesondere selbsttätigen Befüllung, Entleerung und/oder Reinigung der Kapselkammer auf, wenn diese mehrfach verwendet wird. Alternativ weist der Inhalator eine Vielzahl von Kapselkammern auf, die jeweils vorzugsweise bereits eine Kapsel enthalten und vorzugsweise nur einmal verwendet werden.

## Beschreibung

Die vorliegende Erfindung betrifft einen Inhalator gemäß dem Oberbegriff des Anspruchs 1, 10 oder 23.

Die vorliegende Erfindung betrifft insbesondere einen Inhalator zur Ausgabe bzw. Inhalation einer vorzugsweise pulverförmigen Formulierung, also einen Pulverinhalator. Jedoch kann die Formulierung grundsätzlich auch in flüssiger Phase, als Dispersion oder in sonstiger fluidisierbarer Form vorliegen.

Bei der Formulierung handelt es sich insbesondere um ein therapeutisches Mittel bzw. Arzneimittel. Insbesondere enthält die Formulierung dementsprechend mindestens einen Wirkstoff oder besteht daraus. Die Formulierung dient also insbesondere der medizinischen Behandlung oder sonstigen therapeutischen Zwecken.

Bei der vorliegenden Erfindung ist die Formulierung in Kapseln aufgenommen, wobei jede Kapsel eine Dosis der Formulierung enthält. Die Formulierung ist also in die Kapseln vordosiert.

Bei der vorliegenden Erfindung sind unter dem Begriff "Kapsel" primär Behältnisse mit einer festen oder einer zumindest im wesentlichen starren Hülle zu verstehen, die insbesondere separat voneinander handhabbar und/oder öffenbar sind. In einem weiteren Sinn sind gemäß der vorliegenden Erfindung unter dem Begriff "Kapsel" vorzugsweise auch sonstige Behältnisse, Verpakkungen oder dgl. mit jeweils einer Dosis der Formulierung zu verstehen, die insbesondere separat voneinander handhabbar und/oder öffenbar sind.

Die EP 0 147 755 A2 offenbart einen Inhalator für die Inhalation pulverförmiger Arzneimittel aus länglichen Kapseln. Der Inhalator weist eine Kapselkammer auf, in die jeweils eine Kapsel manuell einführbar ist. Die Kapsel wird durch manuelle Betätigung einer Öffnungseinrichtung in der Kapselkammer längsseitig angestochen und dadurch geöffnet. Beim Inhalieren führt ein durch die Kapselkammer strömender Luftstrom dazu, daß die Kapsel in der Kapselkammer hin- und herbewegt wird, wobei das pulverförmige Arzneimittel ausgetragen und im Luftstrom dispergiert wird. Die vorliegende Erfindung benutzt insbesondere dieses Prinzip, ist jedoch aber auch bei sonstigen Techniken zum Austrag einer Formulierung einsetzbar.

Weiter ist ein Inhalator unter der Bezeichnung "Inhalator M" von der Boehringer Ingelheim Pharma GmbH & Co. KG, Ingelheim, Deutschland, bekannt, der entsprechend der EP 0 147 755 A2 arbeitet und einen drehbaren Träger mit sechs Kapselkammern aufweist, die manuell mit Kapseln befüllbar sind.

Die WO 2005/049121 A1 offenbart eine insbesondere in einen Pulverinhalator einsetzbare, tragbare Kapselvorrichtung zur Aufnahme einer Vielzahl von Kapseln. Die zylindrischen Kapseln sind aufrecht hintereinander von einer Schiene geführt oder kettenartig miteinander verbunden.

Der vorliegenden Erfindung liegt die Aufgabe zugrunde, einen Inhalator anzugeben, der eine einfache Handhabung, einen einfachen bzw. kompakten Aufbau und/oder eine Mehrfachbenutzung ohne manuelles Einsetzen neuer Kapseln gestattet.

Die obige Aufgabe wird durch einen Inhalator gemäß Anspruch 1,10 oder 23 gelöst. Vorteilhafte Weiterbildungen sind Gegenstand der Unteransprüche.

Einzelne Aspekte, Merkmale, Eigenschaften und Vorteile der vorliegenden Erfindung ergeben sich aus den Ansprüchen und der folgenden Beschreibung bevorzugter Ausführungsformen und -varianten anhand der Zeichnung. Es zeigt:
- Fig. 1: eine schematische Explosionsdarstellung eines Inhalators gemäß einer bevorzugten AusfUhrungsform;
- Fig. 2: einen schematischen, ausschnittsweisen Schnitt des Inhalators gemäß Fig. 1;
- Fig. 3a: eine perspektivische Darstellung eines Trägers des Inhalators gemäß Fig. 1;
- Fig. 3b: eine schematische Darstellung des Öffnens des Trägers gemäß Fig. 3a;
- Fig. 3c: einen schematischen, ausschnittsweisen Schnitt von Fig. 3b;
- Fig. 3d: eine schematische Ansicht einer weiteren Ausführungsform;
- Fig. 3e: eine schematische Ansicht einer weiteren Ausführungsform;
- Fig. 3f: einen schematischen, ausschnittsweisen Schnitt von Fig. 3e;
- Fig. 3g: einen schematischen, ausschnittsweisen Schnitt einer weiteren Ausführungsform;
- Fig. 4a: eine schematische Ansicht des Inhalators gemäß einer weiteren Ausführungsform;
- Fig. 4b: einen schematischen, ausschnittsweisen Schnitt des Inhalators gemäß Fig. 4a;
- Fig. 5a: eine schematische Ansicht eines Trägers des Inhalators gemäß einer weiteren Ausführungsform;
- Fig. 5b: eine schematische Vergrößerung von Fig. 5a;
- Fig. 5c: eine schematische, schnittartige Seitenansicht des Inhalators mit dem Träger gemäß Fig. 5a;
- Fig. 6a: eine schematische, teilgeschnittene Ansicht des Inhalators gemäß einer weiteren Ausführungsform;
- Fig. 6b: einen schematischen, ausschnittsweisen Schnitt des Inhalators gemäß Fig. 6a;
- Fig. 6c: eine schematische seitliche Funktionsdarstellung des Inhalators gemäß Fig. 6a;
- Fig. 7a: einen schematischen Schnitt des Inhalators gemäß einer weiteren Ausführungsform in einer Nicht-Gebrauchsstellung;
- Fig. 7b: einen schematischen Schnitt des Inhalators gemäß Fig. 7a in einer Zwischenstellung;
- Fig. 7c: einen schematischen Schnitt des Inhalators gemäß Fig. 7a in einer Gebrauchsstellung;
- Fig. 8a: eine schematische, schnittartige Ansicht des Inhalators gemäß einer weiteren Ausführungform;
- Fig. 8b: einen schematischen Schnitt des Inhalators gemäß Fig. 8a;
- Fig. 8c: einen weiteren schematischen Schnitt des Inhalators gemäß Fig. 8a;
- Fig. 9: einen schematischen Schnitt des Inhalators gemäß einer weiteren Ausführungsform;
- Fig. 10a: eine schematische Darstellung eines Trägers des Inhalators gemäß einer weiteren Ausführungsform;
- Fig. 10b: eine schematische Darstellung eines Trägers des Inhalators gemäß einer weiteren Ausführungsform;
- Fig. 11: eine schematische Ansicht des Inhalators gemäß einer weiteren Ausführungsform;
- Fig. 12: eine schematische Ansicht eines Fühnmgselements und einer davon gelösten Kapselkammer des Inhalators gemäß Fig. 11;
- Fig. 13: einen ausschnittsweisen, schematischen Schnitt des Inhalators gemäß Fig. 11;
- Fig. 14a: einen schematischen Schnitt des Inhalators gemäß einer weiteren Ausführungsform mit geöffneter Kapselkammer;
- Fig. 14b: einen schematischen Schnitt des Inhalators gemäß Fig. 14a mit geschlossener Kapselkammer;
- Fig. 14c: einen schematischen Schnitt des Inhalators gemäß einer weiteren Ausführungsform mit geschlossener Kapselkammer;
- Fig. 14d: eine perspektivische Ansicht des offenen Inhalators gemäß Fig. 14c;
- Fig. 15a: eine schematische Ansicht einer Befülleinrichtung und der Kapselkammer des Inhalators gemäß Fig. 14a mit geöffneter Kapselkammer;
- Fig. 15b: eine schematische Ansicht einer Befülleinrichtung und der Kapselkammer des Inhalators gemäß Fig. 14a mit geschlossener Kapselkammer;
- Fig. 15c: eine schematische Ansicht eines Kapselkammerteils der Öffnungseinrichtung bzw. der Kapselkammer des Inhalators gemäß Fig. 14a;
- Fig. 16a: einen schematischen, ausschnittsweisen Schnitt des Inhalators gemäß Fig. 14a mit geöffneter Kapselkammer;
- Fig. 16b: einen schematischen, ausschnittsweisen Schnitt des Inhalators gemäß Fig. 14a mit geschlossener Kapselkammer;
- Fig. 17a: eine schematische Darstellung einer geschlossenen Kapselkammer eines Inhalators gemäß einer weiteren Ausführungsform;
- Fig. 17b: eine schematische Darstellung der Kapselkammer des Inhalators gemäß Fig. 17b in geöffnetem Zustand;
- Fig. 18: einen schematischen Aufbau des Inhalators gemäß einer weiteren Ausführungsform;
- Fig. 19: einen schematischen Aufbau des Inhalators gemäß einer weiteren Ausführungsform;
- Fig. 20: einen schematischen Schnitt des Inhalators gemäß Fig. 19;
- Fig. 21: eine Ansicht des Inhalators gemäß einer weiteren Ausführungsform im Transport- bzw. Inhalationszustand;
- Fig. 22: eine Ansicht des Inhalators gemäß Fig. 21 in einem Zwischenstadium während der Betätigung;
- Fig. 23: eine ausschnittsweise, schnittartige Funktionsdarstellung des Inhalators gemäß Fig. 21 im Transport- bzw. Inhalationszustand;
- Fig. 24: eine ausschnittsweise, schnittartige Funktionsdarstellung des Inhalators gemäß Fig. 21 beim anfänglichen Drehen;
- Fig. 25: eine ausschnittsweise, schnittartige Funktionsdarstellung des Inhalators gemäß Fig. 21 beim weiteren Drehen;
- Fig. 26: eine ausschnittsweise, schnittartige Funktionsdarstellung des Inhalators gemäß Fig. 21 nach dem Drehen um 90°;
- Fig. 27: eine ausschnittsweise, schnittartige Funktionsdarstellung des Inhalators gemäß Fig. 21 kurz vor Vollendung der Drehbewegung;
- Fig. 28: eine schematische Darstellung eines Innenraums des Inhalators gemäß einer weiteren Ausführungsform;
- Fig. 29: eine schematische Ansicht des Inhalators gemäß einer weiteren Ausführungsform;
- Fig. 30: eine schematische, teilgeschnittene Darstellung des Inhalators gemäß Fig. 29;
- Fig. 31: eine andere, spitzartige Ansicht des Inhalators gemäß Fig. 29;
- Fig. 32: einen schematischen, schnittartigen Aufbau des Inhalators gemäß einer weiteren Ausführungsform;
- Fig. 33: einen schematischen Aufbau des Inhalators gemäß einer weiteren Ausführungsform;
- Fig. 34: einen schematischen Schnitt des Inhalators gemäß einer weiteren Ausführungsform;
- Fig. 35: einen anderen Schnitt des Inhalators gemäß Fig. 34;
- Fig. 36: einen schematischen Schnitt des Inhalators gemäß einer weiteren Ausführungsform;
- Fig. 37a: einen ausschnittsweisen, schematischen Schnitt des Inhalators gemäß Fig. 36 in einem ersten Zustand;
- Fig. 37b: einen ausschnittsweisen, schematischen Schnitt des Inhalators gemäß Fig. 36 in einem zweiten Zustand;
- Fig. 37c: einen ausschnittsweisen, schematischen Schnitt des Inhalators gemäß Fig. 36 in einem dritten Zustand;
- Fig. 38a: einen schematischen Schnitt des Inhalators gemäß einer weiteren Ausführungsform;
- Fig. 38b: einen schematischen Schnitt einer Kapselkammer des Inhalators gemäß Fig. 38a;
- Fig. 38c: einen ausschnittsweisen Schnitt des Inhalators gemäß Fig. 38a;
- Fig. 39: einen schematischen, ausschnittsweisen Aufbau eines Reservoirs des Inhalators gemäß einer weiteren Ausführungsform;
- Fig. 40: einen schematischen, ausschnittsweisen Aufbau eines Reservoirs des Inhalators gemäß einer weiteren Ausführungsform;
- Fig. 41: einen schematischen, ausschnittsweisen Aufbau eines Reservoirs des Inhalators gemäß einer weiteren Ausführungsform;
- Fig. 42: einen schematischen, ausschnittsweisen Aufbau eines Reservoirs des Inhalators gemäß einer weiteren Ausführungsform;
- Fig. 43: einen schematischen Schnitt eines Trägers oder einer Kapselkammer des Inhalators gemäß einer weiteren Ausführungsform;
- Fig. 44: einen schematischen Schnitt des Inhalators gemäß einer weiteren Ausführungsform;
- Fig. 45: einen schematischen Schnitt des Inhalators gemäß einer weiteren Ausführungsform in einem ersten Zustand und
- Fig. 46: einen schematischen Schnitt des Inhalators gemäß einer weiteren Ausführungsform in einem zweiten Zustand.

In den Figuren werden für gleiche oder ähnliche Teile die gleichen Bezugszeichen verwendet, auch wenn eine wiederholte Beschreibung weggelassen ist. Insbesondere ergeben sich dann auch die gleichen oder entsprechende Vorteile und Eigenschaften. Die einzelnen Figuren sind aus Darstellungs- oder Vereinfachungsgründen meistens nicht maßstabsgerecht.

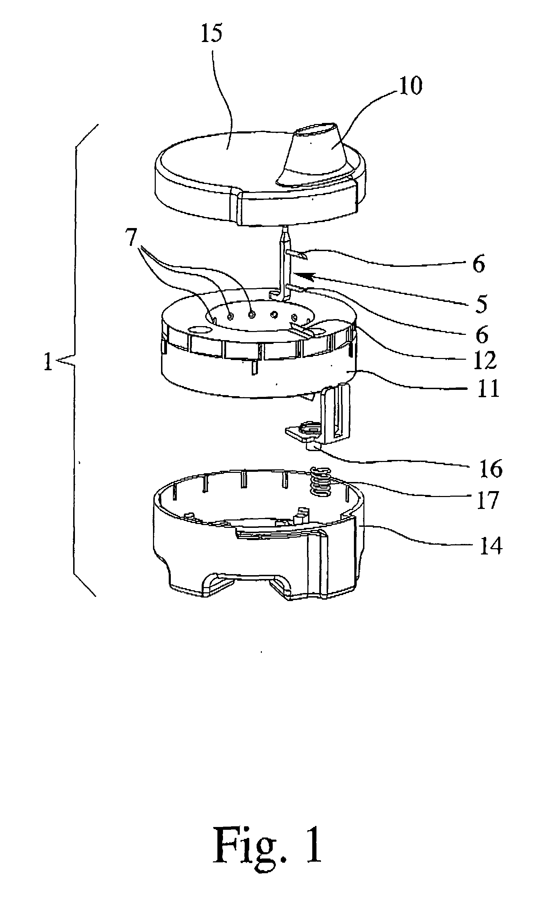

Fig. 1 zeigt in einer perspektivischen, explosionsartigen Darstellung schematisch den Aufbau eines vorschlagsgemäßen Inhalators 1 gemäß einer bevorzugten Ausführungsform, Der Inhalator 1 ist vorzugsweise tragbar ausgebildet und arbeitet insbesondere nur mechanisch.

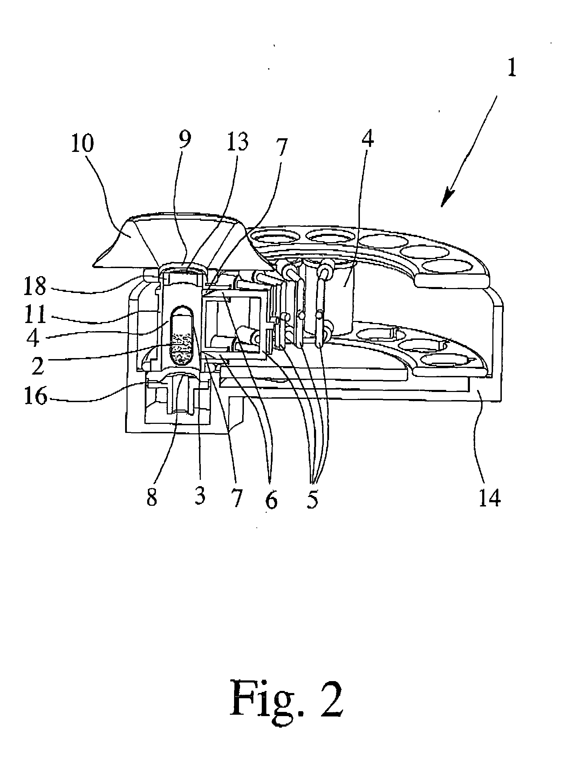

Fig. 2 zeigt in einem schematischen Schnitt den zusammengebauten Inhalator 1 zur Inhalation einer vorzugsweise pulverförmigen Formulierung 2 im eingangs genannten Sinne aus Kapseln 3.

In dem schematischen Schnitt ist eine Kapsel 3 innerhalb einer Kapselkammer 4 des Inhalators 1 gezeigt. Die Kapsel 3 ist noch verschlossen, also noch nicht geöffnet.

Die Kapseln 3 sind vorzugsweise länglich ausgebildet. Grundsätzlich können die Kapseln 3 jedoch auch jede sonstige geeignete Form aufweisen und beispielsweise kugelförmig ausgebildet sein.

Die Kapseln 3 können grundsätzlich aus jedem geeigneten Material hergestellt sein bzw. bestehen. Vorzugsweise wird Gelatine als Kapselmaterial verwendet. In diesem Fall kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird Gelatine im Gemisch mit PEG, bevorzugt PEG 3350, verwendet. Besonders bevorzugt enthält eine Gelatine-Kapsel 3 PEG in einem Anteil von 1 bis 10 % (Gew.-%), bevorzugt 3 bis 8 %. Besonders bevorzugte Gelatine-Kapseln 3 enthalten PEG in einem Anteil von 4 bis 6 %, wobei ein PEG-Anteil von etwa 5 % höchstbevorzugt ist. Im Falle Gelatine-haltiger Kapselmaterialien weisen die Kapseln 3 vorzugsweise eine Tews- oder Halogentrockner-Feuchte von weniger als 12 %, besonders bevorzugt von ≤ 10 % auf.

Werden Cellulosederivate als Kapselmaterial verwendet, so ist die Verwendung von Hydropropylmethylcellulose, Hydroxypropylcellulose, Methyleellulose, Hydroxymethylcellulose und Hydroxyethylcellulose bevorzugt. Besonders bevorzugt wird in diesem Fall Hydropropylmethylcellulose (HPMC), besonders bevorzugt HPMC 2910 als Kapselmaterial eingesetzt. Im Falle der Verwendung von Cellulosederivaten als Kapselmaterialien liegt der Grad der Tews- oder Halogentrockner-Feuchte vorzugsweise bei weniger als 8 %, besonders bevorzugt bei weniger als 5 %. Höchstbevorzugt werden Inhalationskapseln 3 aus Cellulosederivaten vor Befüllung mit einem Tiotropiumhaltigen Inhalationspulver auf eine Tews- oder Halogentrockner-Feuchte von weniger als 4 %, besonders bevorzugt weniger als 2 %, getrocknet.

Als Kunststoffmaterial für die Kapseln 3 kommen alle pharmazeutisch akzeptablen Kunststoffe in Frage, die spritz- oder blasformtechnisch sowie tiefziehtechnisch durch Thermoformen verarbeitet werden können, und/oder Kunststoffe, für deren Verarbeitung zur Kapselkappe oder zum Kapselkörper kein Formtrennmittel notwendig ist, das ein Anhaften der Inhaltsstoffe an die Kapselwand bewirken kann. Der Kunststoff sollte auch keine ausgeprägte Adhäsion für pharmazeutisch-chemische Stoffe, insbesondere für Partikel mit lungengängiger Größe aufweisen. Die bevorzugte Shorehärte D der Materialien liegt vorzugsweise zwischen 10 und 85, bevorzugt zwischen 55 und 75, besonders bevorzugt zwischen 60 bis 70. Das Material sollte auch so sein, daß eine Kunststoffkapsel 3 entlang der Längsachse einer Kraft von vorzugsweise bis zu 20 N standhält. Außerdem sollte die Wand der Kapsel 3 eine Durchlässigkeit für Wasserdampf von weniger als 1,3 x 10⁻¹⁴ kg/(m² s Pa), bevorzugt von 1,5 x 10⁻¹⁶ kg/(m² s Pa), aufweisen. Die Schmelzviskosität MFR (melt flow rate) liegt bevorzugt zwischen 40 und 65 g/10 Minuten, bevorzugt bei 45 bis 49 g/10 Minuten und besonders bevorzugt bei 52 g/10 Minuten.

Die Kapseln 3 können jeweils aus einem Kapselkörper und einer Kapselkappe bestehen, wie insbesondere in der WO 00/07572 A2 offenbart. Daher wird hiermit explizit auf den Inhalt der WO 00/07572 A2 in vollem Umfang Bezug genommen. Bei diesem zweiteiligen Aufbau wird insbesondere Kunststoff als Kapselmaterial eingesetzt. Insbesondere bestehen der Kapselkörper und die Kapselkappe aus dem gleichen Material. Sie werden so miteinander verbunden, daß ein stabiler, abgeschlossener Hohlraum von definiertem Volumen gebildet wird. Besonders bevorzugt wird hierbei Kunststoff, insbesondere Polyethylen, eingesetzt. Die Kapsel 3 kann Rastelemente aufweisen, die die Kapselkappe fest mit dem Kapselkörper verbinden.

Zum Öffnen weist der Inhalator 1 eine Öffnungseinrichtung 5 auf, insbesondere mehrere Öffnungseinrichtungen 5, die jeweils einer Kapselkammer 4 bzw. einer Kapsel 3 zugeordnet sind. Insbesondere erfolgt beim Darstellungsbeispiel ein seitliches Öffnen der Kapsel 3. Hierzu ist die vorzugsweise längliche Kapsel 3 jeweils beispielsweise von mindestens einem Anstechelement 6 - beim Darstellungsbeispiel jeweils zwei Anstechelementen 6 - der jeweiligen Öffnungseinrichtung 5 längsseitig bzw. seitlich und/oder quer zur Hauptströmungsrichtung anstechbar und dadurch öffenbar. Die Kapselkammer 4 weist vorzugsweise entsprechende Anstechöffnungen 7 für die Anstechelemente 6 auf. Vorzugsweise sind die Anstechelemente 6 in die Anstechöffnungen 7 vorgerückt und verschließen diese. Diese Stellung nimmt die Öffnungseinrichtung 5 insbesondere nach dem Öffnen der Kapsel 3 während der Inhalation ein.

Der Kapselkammer 4 sind ein Einlaß 8 und ein Auslaß 9 zugeordnet, die insbesondere axial bzw. stirnseitig an die vorzugsweise längliche bzw. zylindrische Kapselkammer 4 angeschlossen sind. Vorzugsweise entsprechen die geometrischen Verhältnisse den Angaben in der EP 0 147 755 A2, die diesbezüglich als ergänzende Offenbarung genannt wird.

Bei der Inhalation strömt Luft oder sonstiges Gas durch den Einlaß 8 in die Kapselkammer 4, durch diese hindurch und aus dem vorzugsweise gegenüberliegenden Auslaß 9 wieder hinaus. Dieser Luft- bzw. Gasstrom kann durch das Einatmen beim Inhalieren und/oder durch einen dem Inhalator 1 zugeordneten, nicht dargestellten Druckerzeuger, wie eine Luftpumpe, ein Druckgasreservoir oder dgl., erzeugt werden.

Der Luftstrom durch die Kapselkammer 4 bewirkt insbesondere durch den Bemoulli-Effekt, daß die Kapsel 3 sich in der Kapselkammer 4 insbesondere axial hin- und herbewegt bzw. vibriert oder schwingt. Dies bewirkt oder unterstützt den Austrag der Formulierung 2 aus der geöffneten Kapsel 3 in den Luftstrom - insbesondere in Form von sehr feinen Partikeln - und ein Dispergieren der Formulierung in den Luftstrom, mit dem die Formulierung 2 über den Auslaß 9 ausgetragen und insbesondere über ein sich anschließendes Mundstück 10 an einen nicht dargestellten Benutzer bzw. Patienten ausgegeben wird. Das Ausgeben bzw. Dispergieren der Formulierung 2 erfolgt insbesondere wie in der EP 0 147 755 A2 beschrieben, die diesbezüglich als ergänzende Offenbarung eingeführt wird. Jedoch kann die Formulierung 2 grundsätzlich auch auf jede sonstige geeignete Weise aus der Kapsel 3 ausgetragen werden.

Bei der vorliegenden Ausführungsform weist der Inhalator 1 mehrere Kapselkammern 4 auf, die jeweils mit einer Kapsel 3 befüllt sind und insbesondere jeweils nur einmal verwendet werden. Es erfolgt insbesondere kein Nachfüllen oder Auswechseln der Kapseln 3. Dies ermöglicht eine werksseitige Konfektionierung des Trägers 11 mit den Kapseln 3, so daß ein manuelles Einsetzen oder Handhaben von Kapseln 3 für den Benutzer bzw. Patienten nicht erforderlich ist.

Der vorschlagsgemäße Inhalator 1 weist vorzugsweise nur eine einzige Öffnungseinrichtung 5 auf. Bedarfsweise kann der Inhalator 1 aber auch mehrere Öffnungseinrichtungen 5 aufweisen, insbesondere so daß jeder Kapselkammer 4 eine separate Öffnungseinrichtung 5 zugeordnet ist oder den Kapselkammem 4 bzw. Kapseln 3 separate bzw. unterschiedliche Anstechelemente 6 oder dergleichen zugeordnet sind, wie in Fig. 2 angedeutet. In diesem Fall sind die Öffnungseinrichtungen 5 vorzugsweise über eine Steuerkurve, Kulisse oder sonstige Einrichtung in erforderlicher Weise steuer- oder betätigbar. Beim Darstellungsbeispiel werden die Öffnungseinrichtungen 5 bzw. die Anstechelemente 6 mit den Kapselkammern 4 bzw. den Trägern 11 mitbewegt, und sind vorzugsweise nicht davon lösbar.

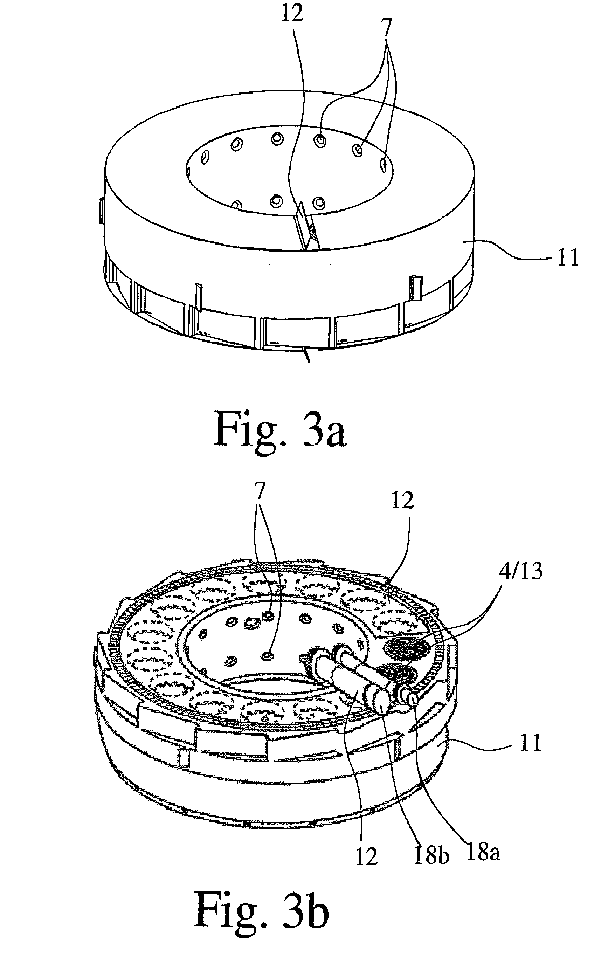

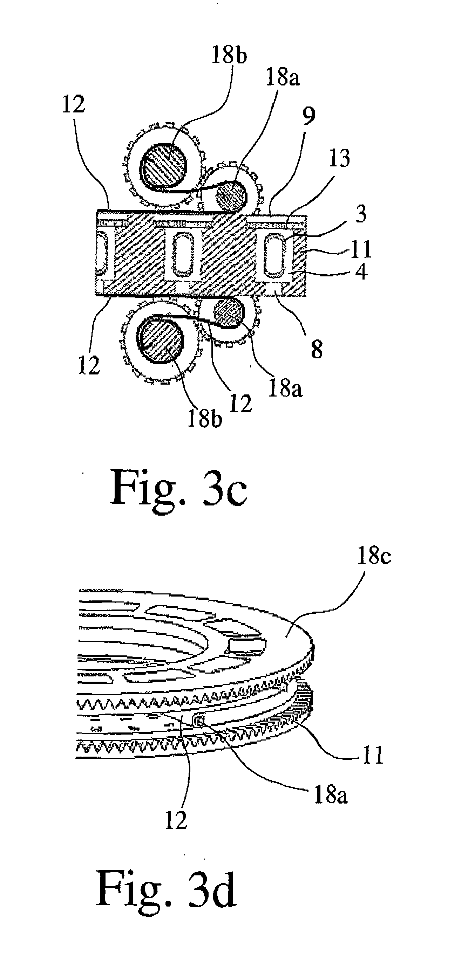

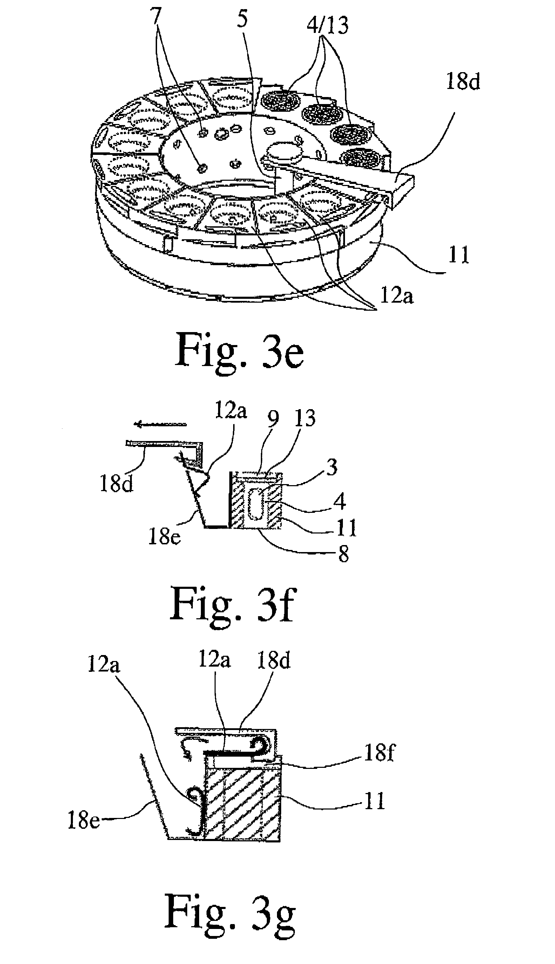

Vorzugsweise sind die Kapseln 3 und die Kapselkammern 4 ringförmig angeordnet. Insbesondere sind die Kapselkammern 4 an oder von einem gemeinsamen, vorzugsweise ringförmigen Träger 11 gebildet (vgl. Fig. 1 und Fig. 3 a). Der Träger 11 ist bedarfsweise auswechselbar. In diesem Fall ist der Inhalator 1 mehrfach - also mit mehreren Trägern 11-verwendbar.

Bei der vorliegenden Ausführungsform sind eine Vielzahl von Kapseln 3 im Träger 11 aufgenommen und entsprechend vielen Kapselkammern 4 gebildet.

Jede Kapsel 3 enthält vorzugsweise eine Dosis der Formulierung 2. Da der Träger 11 eine Vielzahl von Kapseln 3 und damit eine entsprechende Anzahl von Dosen enthalten kann, kann der Inhalator 1 beispielsweise für eine Woche oder mehrere Wochen oder sogar für einen Monat die Versorgung eines Benutzers bzw. Patienten mit der Formulierung 2, also einem Medikament oder dgl., sicherstellen.

Die Kapselkammern 4 sind vorzugsweise verschlossen, insbesondere flüssigkeitsdicht und besonders bevorzugt auch gasdicht. Beispielsweise ist der Träger 11 hierzu mit einer ggf. durchgehenden Abdeckung 12 auf beiden Stirnseiten versehen, so daß einerseits die Einlässe 8 und andererseits die Auslässe 9 - ggf. auch darin eingesetzte bzw. angeordnete Gitter 13 - überdeckt und verschlossen sind. Bei der Abdeckung 12 handelt es sich insbesondere um eine geeignete Versiegelung, Folie oder dgl.

Beim Darstellungsbeispiel ist die Abdeckung 12 jeweils vorzugsweise streifenartig bzw. durchlaufend ausgebildet. Jedoch können bedarfsweise auch separate Abdeckungen für die einzelnen Kapselkammern 4 bzw. Einlässe 8 und/oder Auslässe 9 vorgesehen sein. Beispielsweise ist auch eine segmentartige Ausbildung der Abdeckung 12 möglich.

Vorzugsweise sind die Anstechöffnungen 7 auch zunächst verschlossen, beispielsweise durch nicht dargestellte Wandungsabschnitte Pfropfen, Abdekkungen, ggf. auch durch die Abdeckung(en) 12 oder dgl.

Die Kapselkammern 4 mit den darin angeordneten Kapseln 3 sind dementsprechend vorzugsweise hermetisch abgeschlossen. Dies gestattet eine lange Lagerung, da die Kapseln 3 optimal gegen Umwelteinflüsse - insbesondere gegen starke Änderungen der Luftfeuchtigkeit oder Verschmutzung - geschützt sind.

Beim Darstellungsbeispiel weist der Inhalator 1 ein Gehäuseunterteil 14 insbesondere zur Aufnahme des Trägers 11 und ein Gehäuseoberteil 15 insbesondere mit dem Mundstück 10 zur Abdeckung auf

Im zusammengebauten Inhalator 1 ist der Träger 11 schrittweise derart weiter bewegbar, daß jeweils die für die nächste Inhalation vorgesehene Kapsel 3 bzw. Kapselkammer 4 in eine Inhalationsposition - hier unter das Mundstück 10 - bewegbar ist. Bei der Bewegung in diese Position - hier durch Rotation des Trägers 11 - erfolgt vorzugsweise ein einzelweises Öffnen der sich in die Inhalationsposition bewegenden Kapselkammer 4, beispielsweise durch Abziehen der Abdeckung(en) 12 vom jeweiligen Einlaß 8 und Auslaß 9. Dies erfolgt vorzugsweise selbsttätig durch eine entsprechende Mechanik, Einrichtung oder dgl. im Inhalator 1. Alternativ kann das Öffnen des Einlasses 8 und des Auslasses 9 auch erst in der Inhalationsposition erfolgen.

Das vorzugsweise einzelweise Öffnen der Kapselkammern 4 kann beispielsweise durch sukzessives Abziehen, Abschälen, Aufrollen oder Aufwickeln der Abdeckung(en) 12 erfolgen. Jedoch kann die Abdeckung 12 bzw. jeweilige Kapselkammer 4 auch auf jede sonstige geeignete Weise geöffnet werden. Beispielsweise ist auch möglich, die Abdeckung 12 - insbesondere bei der Ringordnung wie bei der ersten Ausführungsform - radial nach innen oder außen und/oder in axialer Richtung zu ziehen, zu drücken, zu schälen oder in sonstiger Weise zu bewegen, zu entfernen oder aufzutrennen.

Bedarfsweise kann die Abdeckung 12 nach dem Entfernen bzw. Ablösen wieder angeklebt werden, beispielsweise am Träger 11 oder an einem sonstigen geeigneten Teil des Inhalators 1. Beispielsweise kann die entfernte Abdekkung 12 auch einem Verschließen der Anstechöffnungen 7 dienen, um die gewünschte Luftführung bei der Inhalation sicherzustellen. Hierzu werden die jeweiligen Anstechöffnungen 7 beispielsweise durch die abgezogene Abdekkung 12 überklebt.

Beim Darstellungsbeispiel weist der Inhalator 1 ein den Einlaß 8 bildendes oder sich daran anschließendes Anschlußstück 16 auf, das insbesondere durch Federkraft - hier durch eine axial wirkende Feder 17 - gegen die Kapselkammer 4 in der Inhalationsposition luftzuführungsseitig vorgespannt und/oder anlegbar ist. Ggf. kann das Anschlußstück 16 bei entsprechender Ausgestaltung auch gleichzeitig das Öffnen der jeweiligen Anschlußkammer 11 bewirken.

Auslaßseitig ist in entsprechender oder einer sonstigen geeigneten Weise das Mundstück 10 mit dem Auslaß 9 oder ein Auslaßkanal bzw. Anschlußstück an die jeweilige Kapselkammer 4 in der Inhalationsposition anschließbar, vorzugsweise anlegbar, insbesondere so daß beim Einatmen ein ausreichender Luftstrom durch die Kapselkammer 4 erzeugt bzw. gesaugt wird, um die Kapsel 3 in der Kapselkammer 4 in gewünschter Weise in Bewegung zu versetzen und insbesondere dadurch die Ausgabe der Formulierung 2 zu bewirken oder unterstützen.

Während der Inhalation bzw. Ausgabe der Formulierung 2 verschließt die Öffnungseinrichtung 5 mit ihren Anstechelementen 6 vorzugsweise die Anstechöffnungen 7, wie in Fig. 2 gezeigt, um die gewünschte Luftströmung vom Einlaß 8 zum Auslaß 9 zu gewährleisten. Nach dem Inhalieren wird die Öffnungseinrichtung 5 aus den Anstechöffnungen 7 vollständig herausgezogen, so daß der Träger 11 weiter gedreht und damit die nächste Kapselkammer 4 in die Inhalationsposition bewegt werden kann.

Die Anstechöffnungen 7 können jedoch auch in sonstiger geeigneter Weise nach dem Anstechen bzw. Öffnen der Kapsel 3 wieder, zumindest temporär für die Inhalation, verschlossen werden. Beispielsweise können die Anstechöffnungen 7 jeweils durch ein nicht dargestelltes, zugeordnetes Septum selbstverschließend ausgebildet oder - zumindest im Bereich der Inhalationsposition nach dem Anstechen der Kapsel 3 - durch ein geeignetes Verschlußelement, wie einen Stopfen, eine anpressbare Dichtung oder dgl., verschlossen werden.

Zur Weiterbewegung des Trägers 11, zur koordinierten Betätigung der Öffnungseinrichtung 5 und/oder zum Öffnen der jeweiligen Kapselkammer 4 ist vorzugsweise eine geeignete, insbesondere gemeinsame Mechanik oder dgl. vorgesehen, die hier nicht dargestellt ist. Insbesondere erfolgt eine manuelle Betätigung des Inhalators 1.

Bei entsprechender Ausgestaltung der Mechanik ergibt sich eine besonders einfache Handhabung. Insbesondere ist ein manuelles Einführen einzelner Kapseln 3 in die jeweilige Kapselkammer 4 nicht erforderlich. Des weiteren kann bei entsprechender Gestaltung die Betätigung eines einzigen Betätigungselements zur Ausführung aller Funktionen - Weiterbewegen der nächsten Kapselkammer 4 mit Kapsel 3 in die Inhalationsposition, Öffnen der Kapselkammer 4 und Öffnen der Kapsel 3 - genügen.

Ein Vorteil des vorschlagsgemäßen Inhalators 1 liegt darin, daß leere bzw. benutzte Kapseln 3 und benutzte Kapselkammern 4 nicht ausgegeben und entsorgt werden müssen, sondern vom Inhalator 1 aufgenommen werden bzw. in diesem verbleiben. Dies gestattet eine sehr einfache Handhabung und einen universellen Einsatz.

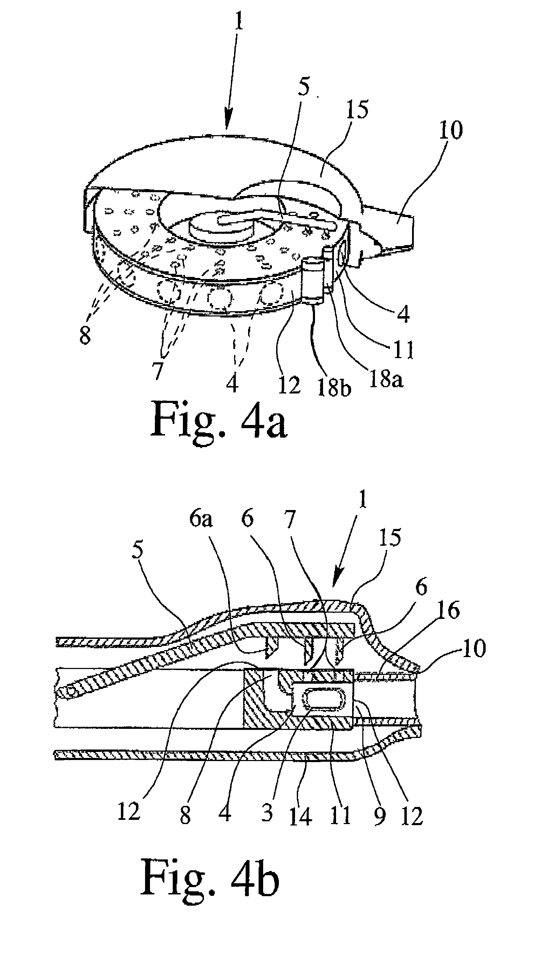

Bei der vorliegenden Ausführungsform sind die Kapseln 3 und/oder Kapselkammern 4 vorzugsweise quer zur Bewegungsrichtung und/oder parallel zueinander und/oder zumindest im wesentlichen axial ausgerichtet. Gemäß Fig. 4, 5 oder 6 können die Kapseln 3 und/oder Kapselkammern 4 jedoch auch radial ausgerichtet sein. Dies gestattet insbesondere eine geringe Bauhöhe des Inhalators 1. Bei Bedarf können die Kapselkammern 4 und/oder Kapseln 3 aus der radialen Lage auch in die axiale Lage zur Inhalation bzw. in der Inhalationsposition geschwenkt werden, insbesondere bei entsprechender Ausbildung des Trägers 11.

Die voranstehenden Erläuterungen gelten für die Kapseln 3 entsprechend, wenn diese zunächst nicht in Kapselkammern 4 angeordnet sind, sondern beispielsweise erst nacheinander einer Kapselkammer 4 zugeführt werden.

Generell können an Stelle der Abdeckung 12 auch sonstige Verschlüsse oder dgl., wie Stopfen, Deckel, Schleusen, eine vorzugsweise drehbare Blende, Schieber, Hülse oder dgl., eingesetzt werden, um die Kapselkammern 4 einzeln oder gruppenweise oder gemeinsam zu verschließen.

Alternativ oder zusätzlich ist es möglich, die Kapselkammern 4 einzeln oder gruppenweise oder den Träger 11 insgesamt mit einer Umverpackung, wie einem Blister, eine Folieneinschweißung oder dgl. zu versehen. Dies ist einer langen Lagerfähigkeit zuträglich.

Bei der vorliegenden Ausführungsform ist der Träger 11 vorzugsweise aus einem relativ festen bzw. starren und/oder diffusionsdichtem Material, insbesondere einem geeigneten Kunststoff, hergestellt Die Anstechöffnungen 7 sind vorzugsweise bereits im Träger 11 gebildet und zum Erhalt der gewünschten Lagerstabilität bzw. zum hermetischen Abschluß der Kapselkammern 4 ebenso wie die Einlässe 8 und Auslässe 9 vorzugsweise abgedeckt. Hierzu kann wiederum eine nicht dargestellte, durchgehende bzw. gemeinsame oder separate Abdeckung vorgesehen sein, wobei die Anstechöffnungen 7 dann vorzugsweise jeweils nur sukzessive - also immer nur für die jeweilige Kapselkammer 4 - geöffnet werden. An Stelle einer Ausbildung der Anstechöffnungen 7 bei der Herstellung des Trägers 11 ist es grundsätzlich auch möglich, die Anstechöffnungen 7 erst durch die Anstechelemente 6 oder dgl. beim Öffnen der einzelnen Kapseln 3 zu bilden. Hierzu können die Anstechelemente 6 beispielsweise entsprechend geschwächte oder dünne Wandungsabschnitte des Trägers 11 durchdringen. Alternativ ist es grundsätzlich auch immer möglich, die Kapsel 3 durch den Einlaß 8 und/oder Auslaß 9 der jeweiligen Kapselkammer 4 hindurch oder vor dem Einführen in die Kapselkammer 4 zu öffnen. Insbesondere ist auch ein axiales bzw. längsseitiges Öffnen der Kapseln 3 möglich.

Bei den Darstellungsbeispielen ist vorzugsweise ein Öffnen der Kapseln 3 jeweils an zwei Stellen vorgesehen, um ein möglichst gutes bzw. vollständiges Entleeren der Kapsel 3 zu ermöglichen bzw. sicherzustellen. Grundsätzlich ist es jedoch auch möglich, die Kapseln 3 jeweils nur an einer Stelle bzw. in einem Bereich zur Entleerung zu öffnen. Dies hängt insbesondere von der Größe, Form und Lage der jeweiligen Öffnung, der Kapselform, der Kammerform, der möglichen Kapselbewegung und dgl. ab.

Weiter ist es grundsätzlich auch möglich, daß sich die Kapseln 3 während der Entleerung nicht bewegen, sondern beispielsweise durch einen entsprechenden Luftstrom durch die jeweils geöffnete Kapsel 3 hindurch oder durch sonstige Ausgabe oder beispielsweise vollständiges Öffnen der Kapsel 3 die jeweilige Dosis der Formulierung 2 entnommen bzw. ausgetragen wird.

Die optionalen Gitter 13 verhindern, daß beispielsweise durch das Öffnen bzw. Anstechen entstandene Kapselteile vom Luftstrom mitgerissen und durch das Mundstück 10 mit ausgegeben werden können. Derartige Kapselteile können von den Gittern 13 zurückgehalten werden. Alternativ oder zusätzlich können die Gitter 13 auch dazu dienen, die Kapseln 3 in den Kapselkammern 4 zu halten.

Die Gitter 13 sind vorzugsweise von insbesondere ringförmigen Einsätzen 18 gehalten, die auslaßseitig an den Kapselkammern 4 angeordnet oder in diese eingesetzt sind. Beim Darstellungsbeispiel führen die Einsätze 18 vorzugsweise gleichzeitig zu einer gewünschten auslaßseitigen Reduzierung des Strömungsquerschnitts, um die gewünschten Strömungsverhältnisse zu erreichen, so daß die bevorzugte Längsbewegung der Kapsel 3 in der Kapselkammer 4 während der Inhalation bzw. beim Durchströmen von Luft bzw. Gas erzeugt wird.

Alternativ ist es auch möglich, ein durchgehendes Gitter für mehrere Kapselkammern 4 oder alle Kapselkammern 4 auslaßseitig vorzusehen.

Nachfolgend werden Ausführungsvarianten und weitere Ausführungsformen näher erläutert. Hierbei wird insbesondere nur auf wesentliche Unterschiede gegenüber der ersten Ausruhrungsform oder neue Aspekte eingegangen. Die bisherigen Ausführungen gelten insbesondere entsprechend oder ergänzend.

Fig. 3b zeigt eine bevorzugte Variante, zum Öffnen der Abdeckung 12. Die vorzugsweise als Folie ausgebildete Abdeckung 12 wird hierbei über eine erste Rolle 18a abgepeelt bzw. abgezogen oder abgerollt und vorzugsweise auf einer zweiten Rolle 18b aufgewickelt. Die beiden Rollen 18a und b sind insbesondere über eine Rutschkupplung oder Verzahnung miteinander verbunden und/oder über eine Verzahnung - vorzugsweise während der oder durch die Weiterbewegung des Trägers 11 bzw. der Kapselkammern 4 - antreibbar bzw. drehbar. Die Rollen 18a und b sind in geeigneter Weise im Inhalator 1 gelagert.

Fig. 3c zeigt in einem ausschnittsweisen Schnitt von Fig. 3b das Öffnen der Kapselkammem 4 bzw. des Trägers 11 auf beiden Seiten, daß vorzugsweise in entsprechender Weise jeweils mit mindestens einer Rolle 18a, vorzugsweise zwei Rollen 18a und b erfolgt.

Alternativ kann an Stelle der ersten Rolle 18a ein Abziehen bzw. Abpeelen auch über eine feststehende Rolle, Kante oder dergleichen erfolgen.

Fig. 3d zeigt in einer ausschnittsweisen schematischen Darstellung eine weitere Ausführungsform. Die Abdeckung 12 wird hier nicht auf der zweiten Rolle 18b aufgewickelt, sondern vorzugsweise über die erste Rolle 18a auf einem gegenläufigen Ring 18c abgelegt.

Fig. 3e veranschaulicht eine andere Ausführungsform. Hier ist die Abdeckung 12 vorzugsweise aus Einzelstücken bzw. -segmenten 12a gebildet, die mittels eines Öffnungselements 18d abziehbar sind. Das Öffnungselement 18d ist vorzugsweise mit der Öffnungseinrichtung 5 derart gekoppelt, daß beim Zurückziehen der Anstechelemente 6 die jeweilige Kapselkammer 4 geöffnet und dadurch für die Inhalation bereit gemacht wird.

Der schematische Schnitt gemäß Fig. 3f veranschaulicht das Prinzip. Das Öffnungselement 18d ist vorzugsweise hakenartig ausgebildet und hakt sich vorzugsweise in eine optionale Ausnehmung und Durchbrechung des jeweiligen Abdeckungsstücks 12a ein, das als nächstes abgezogen werden soll. Im von dem in Fig. 3e gezeigten Zustand - Verlauf der in Fig. 3f durch den Pfeil angedeuteten Bewegung wird - ausgehend das jeweilige Abdeckungsstück 12a vorzugsweise radial nach innen abgezogen und dadurch die zugeordnete Kapselkammer 4 geöffnet. Dieser Zustand ist in Fig. 3f dargestellt. Spätestens beim Zurückbewegen des Öffnungselements 18b in seine in Fig. 3e dargestellte Ausgangsposition kann sich das abgezogene Abdeckungsstück 12a vom Öffnungselement 18d bzw. dessen Haken lösen und vorzugsweise in einen in Fig. 3f angedeuteten, optionalen Auffangbehälter 18e oder dergleichen fallen bzw. darin abgelegt werden.

Fig. 3g zeigt in einem vergleichbaren Schnitt eine weitere Ausführungsform. Hier ist das Öffnungselement 18d insbesondere schaufelförmig ausgebildet. Vorzugsweise greift das Öffnungselement 18d mit zwei schaufelförmigen Enden in vorzugsweise radiale Nuten 18f im Träger 11 seitlich unter das jeweilige Abdeckungsstück 12a und peelt oder dieses insbesondere von außen her ab. Die abgepeelten bzw. abgezogenen Abdeckungsstücke 12a werden vorzugsweise wiederum vom Auffangbehälter 18e aufgenommen.

Bedarfsweise kann die Abdeckung 12 auch einstückig ausgebildet sein. In diesem Fall erfolgt das Öffnen - vorzugsweise durch entsprechende Ausbildung des Öffnungselements 18d - derart, daß die Abdeckung 12 in einzelne Abdeckungsstücke 12a zerschnitten oder in sonstiger Weise aufgetrennt wird, sofern die Abdeckung 12 nicht als fortlaufendes Band, Streifen oder dergleichen insbesondere aufgewickelt oder in sonstiger geeigneter Weise aufgenommen wird. Zur Erleichterung der Bildung einzelner bzw. voneinander separierbarer Abdeckungsstücke 12a ist die Abdeckung 12 bedarfsweise geschlitzt, perforiert und/oder mit sonstigen Sollbruchstellen oder dergleichen versehen. Vorzugsweise sind separate bzw. getrennte Abdeckungen 12 bzw. Abdeckungsstücke 12a für die Einlaßseite einerseits und die Auslaßseite andererseits der Kapselkammern 4 vorgesehen. Jedoch ist es grundsätzlich auch möglich eine gemeinsame Abdeckung 12 oder gemeinsame Abdeckungsstükke 12a einzusetzen, die zumindest den jeweiligen Einlaß 8 und Auslaß 9 einer Kapselkammer 4 abdecken.

Fig. 4a zeigt in einer schematischen Darstellung eine weitere Ausführungsform des vorschlagsgemäßen Zerstäubers 1. Die Kapseln 3 und Kammern 4 sind hier vorzugsweise radial ausgerichtet. Die Abdeckung 12 ist umfangsseitig angeordnet und wird vorzugsweise entsprechend am Umfang abgepeelt bzw. abgezogen, insbesondere wiederum mittels der Rollen 18a und b oder in sonstiger geeigneter Weise.

Die Öffnungseinrichtung 5 dient hier vorzugsweise auch einem zuluft- bzw. einlaßseitigen Öffnen der Kapselkammern 4. Die gemeinsame, beispielsweise hebel, stangen- oder fingerartige Öffnungseinrichtung 5 weist hierzu beispielsweise ein zusätzliches Anstechelement 6a auf, das die beispielsweise axialseitige Abdeckung 12 durchstechen kann, um den Einlaß 8 der jeweiligen Kapselkammer 4 freizugeben bzw. zu öffnen. Die Anstech- und/oder Öffnungsbewegung erfolgt bei dieser Ausführungsform vorzugsweise axial. Fig. 4b zeigt eine noch einlaß- und auslaßseitig geschlossene Kapselkammer 4 in der Inhalierposition benachbart zu dem sich hier vorzugsweise radial erstreckenden Mundstück 10.

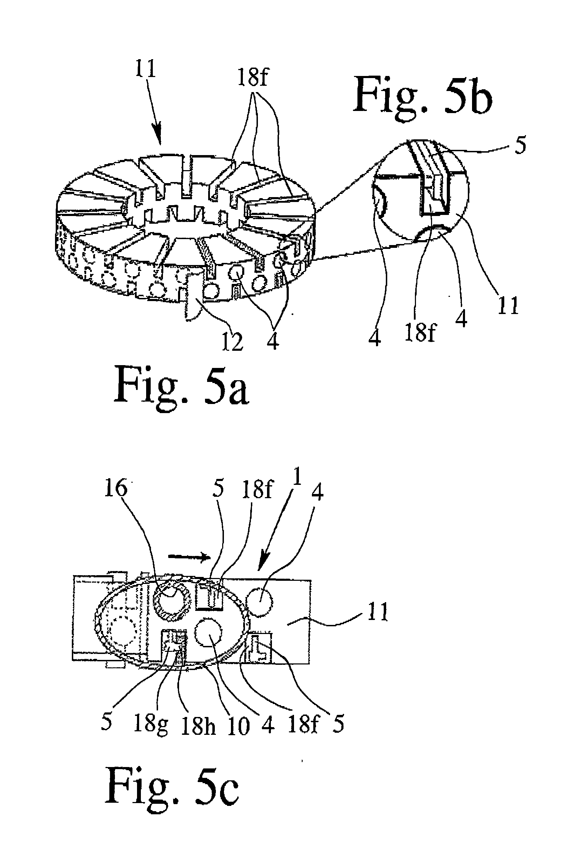

Fig. 5a bis 5c veranschaulichen eine weitere Ausführungsform. Fig. 5a zeigt in einer perspektivischen Ansicht den Träger 11 mit radial ausgerichteten, abwechselnd in zwei Axialebenen angeordneten bzw. gegeneinander versetzten Kapseln 3 und Kapselkammern 4. Vorzugsweise ist hier jeder Kapselkammer 4 eine insbesondere hebelartige oder stangenartige Öffnungseinrichtung 5 zugeordnet, die insbesondere ähnlich wie die in Fig. 4a und b dargestellte Öffnungseinrichtung 5 ausgebildet ist. Um eine geringe axiale Bauhöhe zu ermöglichen, weist der Träger 4 vorzugsweise auf beiden Flach- bzw. Stirnseiten radiale Nuten 18f zwischen den Kapselkammern 4 auf, in die sich die Öffnungseinrichtungen 5 erstrecken, wie in der ausschnittsweisen Vergrößerung mit Fig. 5b dargestellt.

Das Anstechen bzw. Öffnen der jeweiligen Kapselkammer 4 erfolgt vorzugsweise wiederum in axialer Richtung. Die Betätigung der einzelnen Öffnungseinrichtungen 5 erfolgt vorzugsweise über eine Steuerkurve, Kulisse 18g oder dergleichen. Besonders bevorzugt ist diese am Mundstück 10 angeordnet oder davon gebildet, wie dem schematischen Schnitt gemäß Fig. 5c zu entnehmen ist.

Das Mundstück 10 kann radial herausgezogen und insbesondere um 180° gedreht werden. Mit der Drehbewegung wird der vorzugsweise ringförmige Träger 11 um eine Position - also zur nächsten Kapselkammer 4 - weiter gedreht. Weiter wird der von dem Mundstück gebildete Strömungskanal bzw. Auslaß 9 durch die Drehung in die entsprechende Axialebene der für die nächste Inhalation vorgesehenen Kapselkammer 4 bewegt. Die Radialbewegung des Mundstücks 10 bewirkt vorzugsweise die insbesondere zwangsgeführte Betätigung bzw. Bewegung der Öffnungseinrichtung 5, beispielsweise durch Eingriff eines Vorsprungs 18h in die hier vorzugsweise radial verlaufende Kulisse 18g. Das Anstechen bzw. Öffnen kann je nach Bedarf beim Herausziehen oder Einschieben des Mundstücks 10 erfolgen.

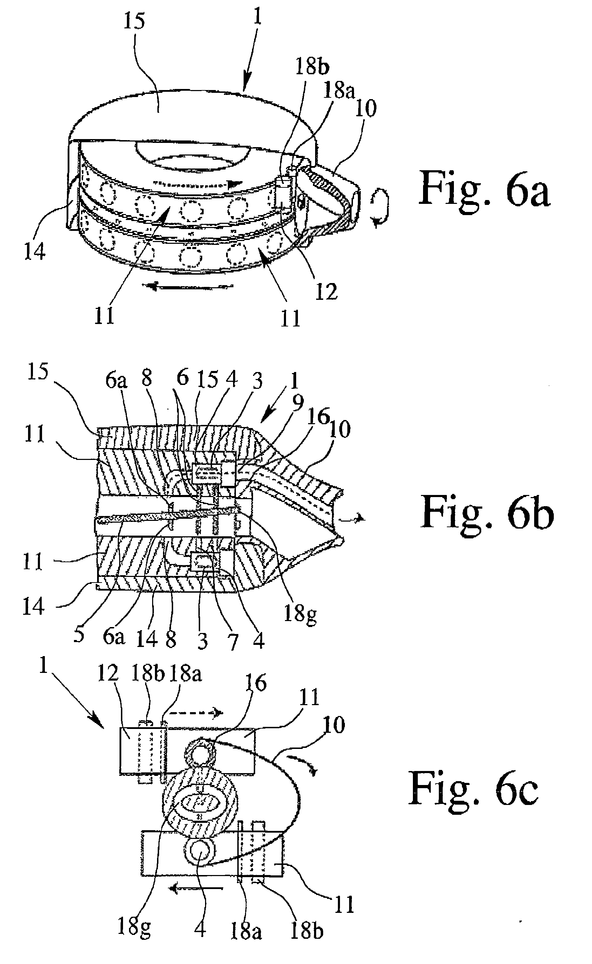

Fig. 6a bis 6c veranschaulichen eine weitere Ausführungsform des vorschlagsgemäßen Inhalators 1. Die Kapselkammern 4 sind hier vorzugsweise in zwei gegenläufigen, ringförmigen Trägern 11 mit insbesondere radialer Ausrichtung untergebracht, wie insbesondere in Fig. 6a gezeigt. Das Peelen bzw. Abziehen der Abdeckungen 12 erfolgt dann vorzugsweise entsprechend in entgegengesetzten Richtungen, insbesondere peripher bzw. tangential wie bei den schon beschriebenen Ausführungsformen.

Wenn das Mundstück 10 um 180° gedreht wird, bewegen bzw. drehen sich die Träger 11 abwechselnd. Hierbei werden jeweils die beiden Abdeckungen 12 am Umfang abgepeelt bzw. abgezogen, vorzugsweise aufgewickelt, wie bereits erläutert. Über eine Kurvenscheibe, Kulisse 18g oder dergleichen - insbesondere am Mundstück 10 - wird die vorzugsweise zentrale bzw. gemeinsame, insbesondere zwischen den beiden Trägern 11 angeordnete Öffnungseinrichtung 5 betätigt, um die Kapseln 3 und die Einlässe 8 der Kapselkammern 4 abwechselnd im oberen und unteren Träger 11 zu öffnen bzw. anzustechen.

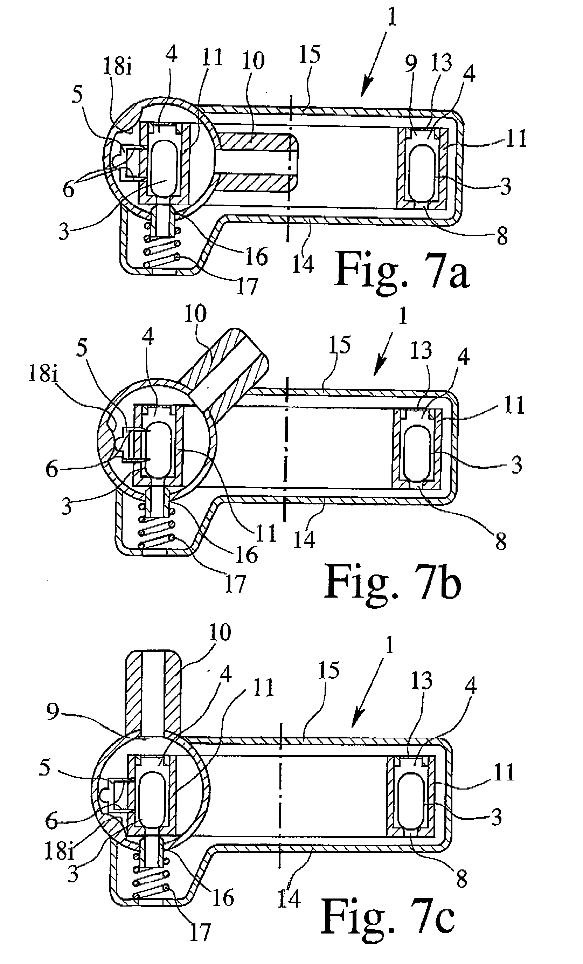

Fig. 7a bis 7c zeigen eine weitere Ausführungsform des vorschlagsgemäßen Inhalators 1 schematisch im Schnitt. Hier ist das Mundstück 10 quer, insbesondere senkrecht, zur Ringebene drehbar bzw. klappbar. In diesem Fall umschließt das Mundstück 10 bzw. dessen Verbindungsabschnitt den Träger 11 im Bereich der Inhalationsposition vorzugsweise zumindest im wesentlichen vollständig mit vorzugsweise ringförmiger Außenkontur. Fig. 7a zeigt das Mundstück 10 in der in die Mitte geklappten Nicht-Gebrauchsposition, Fig. 7b eine Zwischenstellung und Fig. 7c das Mundstück 10 in der ausgeklappten, insbesondere axialen Gebrauchsposition.

Vorzugsweise weist der Inhalator 1 eine derartige Mechanik, wie die Steuerkurve 18i oder dergleichen auf, so daß das Aus- bzw. Hochklappen des Mundstücks 10 zu der gewünschten Betätigung des Inhalators 1 - insbesondere Weitertransport des Trägers 11, Öffnen der Kapsel 3 und/oder Öffnen der Kapselkammer 4 - führt. Bedarfsweise kann der Weitertransport des Trägers 11 zusätzlich oder alternativ auch beim Einklappen bzw. durch das Einklappen des Mundstücks 10 erfolgen.

Das Mundstück 10 kann je nach Bedarf um 90° oder sogar bis zu 270° schwenkbar bzw. klappbar sein. Vorzugsweise rastet das Mundstück 10 sowohl in seiner Gebrauchsstellung als auch in seiner Nicht-Gebrauchsstellung ein.

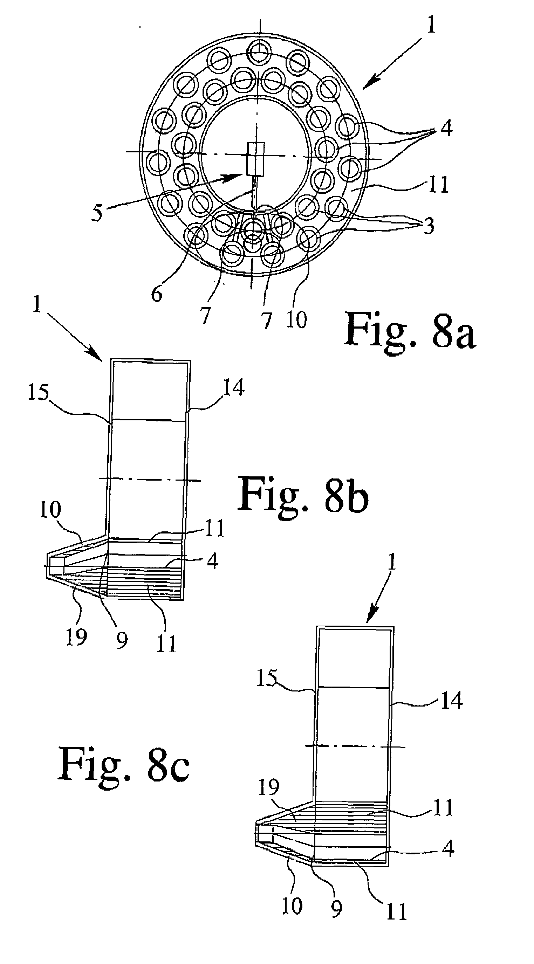

Fig. 8a bis 8c zeigen in sehr schematischen Darstellungen eine weitere Ausführungsform. Hier sind die Kapseln 3 und Kapselkammern 4 mehrreihig, insbesondere in zwei konzentrischen Ringen mit zwei unterschiedlichen Radien mit axialer Ausrichtung angeordnet, bilden also eine Doppelringanordnung. Die inneren und äußeren Kapselkammern 4 sind vorzugsweise derart versetzt, daß die vorzugsweise innerhalb des Rings angeordnete Öffnungseinrichtung 5 mit den Anstechelementen 6 zwischen den inneren Kapselkammern 4 hindurch die äußeren Kapselkammern 4 radial anstechen kann. Die Anstechkanäle bzw. Öffnungen sind vorzugsweise entsprechend angeordnet, wie in Fig. 8a angedeutet. Die Doppelringanordnung gestattet einen besonders kompakten Aufbau des Inhalators 1 bei einer hohen Anzahl von Kapselkammern 4 und Kapseln 3.

Das Mundstück 10 ist vorzugsweise derart ausgebildet, daß abwechselnd oder wahlweise aus einer auf dem inneren Kreis oder auf dem äußeren Kreis liegenden Kapselkammer 4 und Kapsel 3 inhaliert werden kann. Hierzu weist das Mundstück 10 insbesondere einen drehbaren Einsatz 19 auf, wie in den Schnittdarstellungen gemäß Fig. 8b und 8c veranschaulicht. An Stelle des drehbaren Einsatzes 19 kann ggf. auch das Mundstück 10 insgesamt drehbar ausgebildet sein, insbesondere wobei ein Drehen des Mundstücks 10 auch ein Weiterdrehen des Trägers 11 bewirken kann. Alternativ können im Mundstück 10 auch zwei unterschiedlich verlaufende Ansaugkanäle gebildet sein, wobei nur über einen Kanal entweder aus einer äußeren oder einer inneren Kapselkammer 4 inhaliert wird.

Auch bei der Doppelringanordnung kann bedarfsweise eine durchgehende bzw. gemeinsame Abdeckung für alle Kapselkammem 4 bzw. alle Einlässe 8 einerseits und alle Auslässe 9 andererseits auf den beiden Flach- bzw. Stirnseiten des Trägers 11 vorgesehen sein. Jedoch ist es beispielsweise auch möglich, daß die Abdeckungen jeweils nur einer Gruppe von Kapselkammern 4 bzw. Einlässen 8 oder Auslässen 9 dienen, insbesondere zwei konzentrische, vorzugsweise streifen- oder ringförmige Abdeckungen auf jeder Flachseite vorgesehen sind, um einerseits die inneren und andererseits die äußeren Kapselkammem 4 abzudecken.

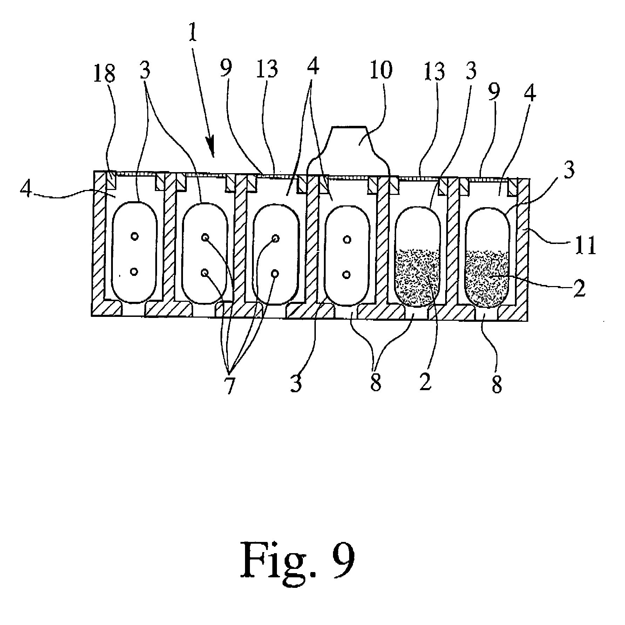

Fig. 9 zeigt in einem schematischen Schnitt eine weitere Ausführungsform des vorschlagsgemäßen Inhalators 1. Die Kapselkammern 4 sind hier vorzugsweise in einer geraden Reihe nebeneinander bzw. hintereinander angeordnet bzw. im Träger 11 gebildet, bedarfsweise aber auch mit unterschiedlich ausgerichteten und/oder mehrseitig angeordnet.

Das Mundstück 10 ist entlang des Trägers 11 verschiebbar und über den einzelnen Kapselkammern 4 bzw. deren Auslässen 9 positionierbar, um eine Inhalation der Formulierung 2 aus den jeweiligen Kapselkammern 4 bzw. Kapseln 3 zu ermöglichen. In der Fig. 9 sind aus Vereinfachungsgründen sonstige Teile oder Komponenten, wie die Öffnungseinrichtung 5, Mittel zur Öffnung der vorzugsweise verschlossenen Kapselkammern 4 oder dg1., nicht dargestellt.

Die Einlässe 8 und Auslässe 9 der jeweiligen Kapselkammer 4 können bei entsprechender Kanalführung bedarfsweise auch an einer gemeinsamen, beispielsweise zu dem Mundstück 10 gewandten Seite des Trägers 11 oder zumindest relativ nah benachbart enden. Dies erleichtert die Verwendung einer gemeinsamen Abdeckung für den Einlaß 8 und Auslaß 9 und oder ermöglicht eine Vereinfachung des Öffnens des Einlasses 8 und Auslasses 9 der jeweiligen Kapselkammer 4, da beispielsweise eine gemeinsame Einrichtung zum Öffnen eingesetzt werden kann. Des weiteren ist dies einem kompakten Aufbau des Inhalators 1 zuträglich.

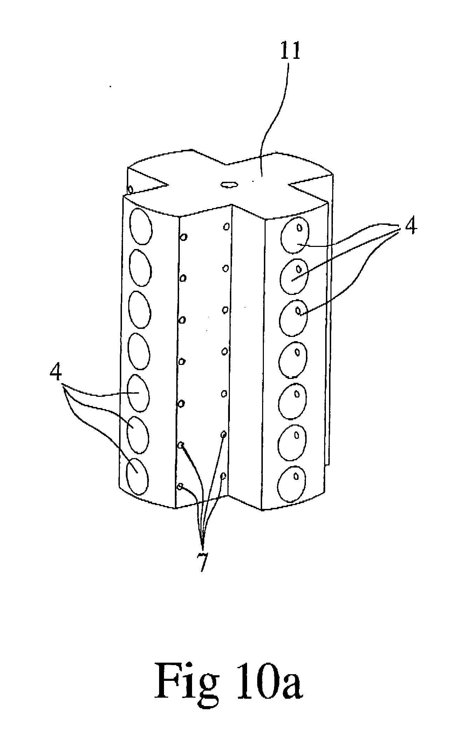

Fig. 10a zeigt in einer sehr schematischen Darstellung eine weitere Ausführungsvariante des Trägers 11 mit geöffneten Kapselkammern 4 im nicht befüllten Zustand, also ohne Kapseln 3, Abdeckungen 12 oder dgl. Die Kapselkammern 4 bzw. Kapseln 3 sind bei dieser Ausführungsvariante in vorzugsweise mehreren Reihen bzw. mehreren Gruppen angeordnet und insbesondere hinsichtlich ihrer Strömungsrichtungen bzw. Längsrichtungen der jeweiligen Kapselkammern 4 bzw. Kapseln 3 unterschiedlich ausgerichtet.

Bei der Ausführungsvariante gemäß Fig. 10a ist das nicht dargestellte Mundstück 10 bzw. eine sonstige Ausgabeeinrichtung vorzugsweise sowohl in axialer Richtung des Trägers 11 verschiebbar als auch relativ zum Träger 11 verschwenkbar. Insbesondere ist hierzu der Träger 11 im nicht dargestellten Inhalator 1 drehbar gelagert bzw. eingebaut.

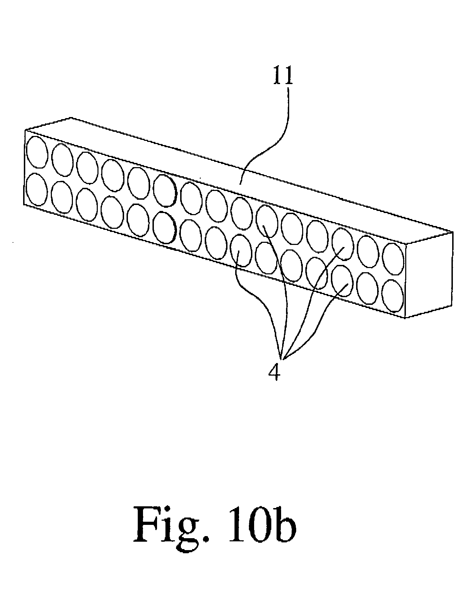

Fig. 10b zeigt eine Ausführungsform, bei der der Träger 11 mehrreihig ausgebildet ist, insbesondere mindestens zwei vorzugsweise parallele Reihen aufweist.

Bei den Darstellungsbeispielen ist der Träger 11 insbesondere prismenförmig bzw. stabförmig, länglich oder kreuzförmig ausgebildet. Jedoch ist grundsätzlich auch jede sonstige Form möglich.

Beispielsweise kann der Träger 11 auch zylindrisch ausgebildet sein. Die Kapselkammern 4 können beispielsweise auch über eine Mantelfläche einer quasi beliebigen Form des Trägers 11 - beispielsweise über eine Zylindermantelfläche - verteilt und/oder wahlweise in Schichten, schraubenlinienförmig oder in sonstiger Weise - angeordnet sein.

Bei den voranstehend beschriebenen Ausführungsformen sind mehrere Kapselkammem 4 des Inhalators 1 in einem gemeinsamen, vorzugsweise starren bzw. festen Träger 11 oder mehreren Trägern 11 gebildet. Jedoch können die Kapselkammern 4 auch beweglich zueinander und insbesondere auch getrennt voneinander ausgebildet sein.

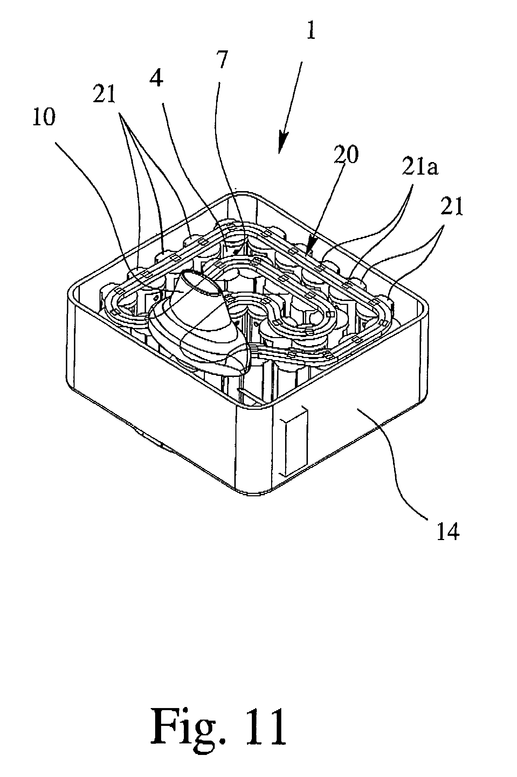

Fig. 11 zeigt in einer nur schematischen Ansicht im teilweise geöffneten bzw. teilweise weggeschnittenen Zustand einen vorschlagsgemäßen Inhalator 1 gemäß einer weiteren Ausführungsform. Nachfolgend werden wieder lediglich wesentliche Unterschiede oder einzelne Besonderheiten erläutert, wobei die bisherigen Ausführungen und Erläuterungen insbesondere entsprechend oder ergänzend gelten.

Bei der vorliegenden Ausführungsform sind die Kapselkammern 4 ebenfalls vorzugsweise nur einmalig verwendbar, insbesondere also jeweils mit einer Kapsel 3 vorkonfektioniert bzw. werksseitig befüllt. Jedoch sind die Kapselkammern 4 nicht fest bzw. starr miteinander verbunden, insbesondere nicht in einem gemeinsamen Träger 11 gebildet, sondern vorzugsweise lose bzw. als separate oder bewegliche, gegebenenfalls voneinander trennbare Einheiten ausgebildet. Insbesondere bilden die Kapselkammern 4 ein Band, eine Kette oder eine sonstige Anordnung.

Die Kapselkammern 4 sind bei dieser Ausführungsform vorzugsweise entlang einer Führung 20, insbesondere einer Schiene, einem Kanal oder dgl., verschiebbar bzw. bewegbar im Inhalator 1 geführt.

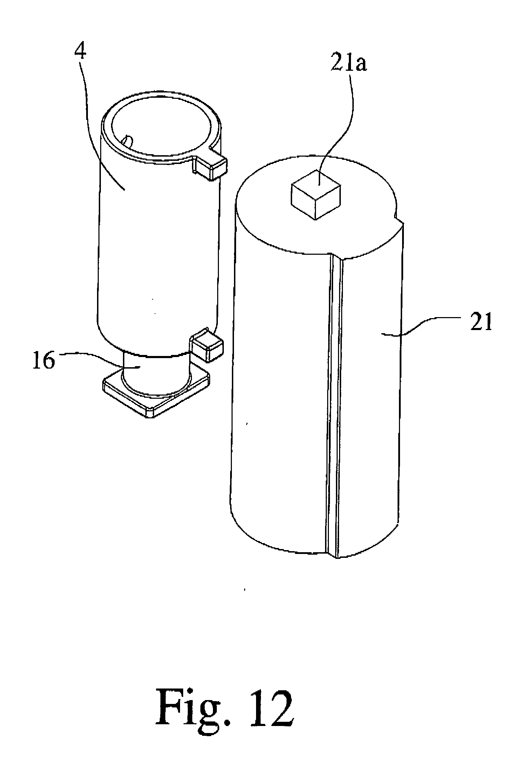

Die Kapselkammern 4 können grundsätzlich unmittelbar von der Führung 20 aufgenommen werden. Beim Darstellungsbeispiel sind die einzelnen Kapselkammern 4 vorzugsweise jedoch in insbesondere gondelartigen Führungselementen 21 aufgenommen und von diesen zur Inhalation vorzugsweise lösbar wie in Fig. 12 angedeutet. Die Führungselemente 21 werden vorzugsweise mit einer bestimmten Drehlage von der Führung 20 geführt (beispielsweise durch Formschluß von Eingriffselementen 21a) und sind bedarfsweise miteinander, beispielsweise gelenkig, rastend, lösbar oder unlösbar verbunden.

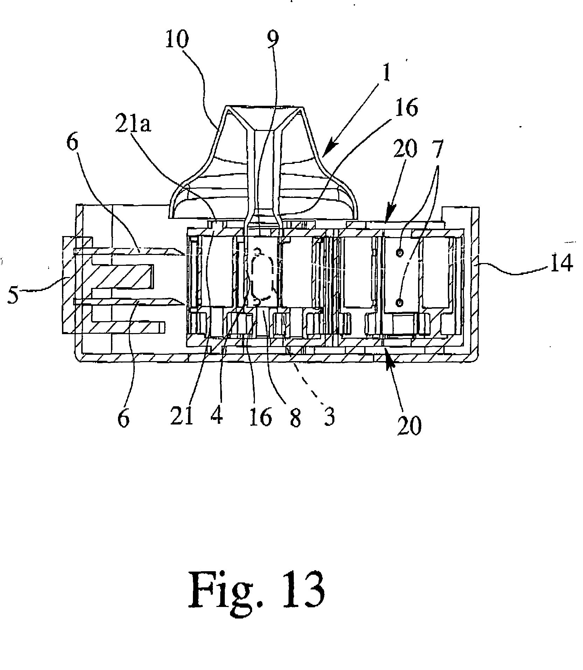

Fig. 13 zeigt in einem ausschnittsweisen, schematischen Schnitt einen möglichen Aufbau des Inhalators 1. Die Kapselkammern 4 bzw. Führungselemente 21 sind vorzugsweise oben und/oder unten von der Führung 20 - insbesondere über die Eingriffselemente 21a - geführt.

Beim Darstellungsbeispiel sind die Kapselkammern 4 den Führungselementen 21 zur Inhalation entnehmbar. Fig. 13 zeigt eine Kapselkammer 4 in der Inhalationsposition unmittelbar unterhalb des Mundstücks 10 mit an die Kapselkammer 4 angeschlossenem Auslaß 9 bzw. Mundstück 10. Diese Kapselkammer 4 wurde insbesondere durch die Öffnungseinrichtung 5 oder eine sonstige Mechanik dem zugeordneten Führungselement 21 - das beim Darstellungsbeispiel gemäß Fig, 13 auf der linken Seite dargestellt ist - entnommen, um die Kapselkammer 4 einlaßseitig und auslaßseitig zu öffnen, die in der Kapselkammer 4 befindliche Kapsel 3 insbesondere durch Anstechen zu öffnen und/oder die Kapselkammer 4 einlaßseitig und/oder auslaßseitig an die entsprechende Luft- bzw. Gasführung anzuschließen.

Fig. 13 zeigt die in der Inhalationsposition befindliche Kapselkammer 4 im inhalationsbereiten Zustand. Der Einlaß 8 und der Auslaß 9 sind geöffnet. Die Kapsel 3 ist geöffnet also insbesondere bereits angestochen. Beim Inhalieren wird ein Luftstrom erzeugt, der durch die Kapselkammer 4 führt und den gewünschten Austrag der Formulierung 2 aus der Kapsel 3 - also einen Dispergieren der Formulierung 2 in den Luftstrom und damit insbesondere eine Ausgabe der Formulierung 2 als Sprühnebel oder Partikelnebel - bewirkt.

Das Weiterbewegen der Kapselkammern 4 bzw. Führungselemente 21 entlang der Führung 20 erfolgt vorzugsweise durch Betätigen, insbesondere Drehen des Mundstücks 10, vorzugsweise um die Inhalationsrichtung bzw. Inhalationsachse, also um eine in der Zeichnungsebene von Fig. 13 von unten nach oben verlaufende Achse. Jedoch kann das Weiterfördern der Kapselkammern 4 bzw. Führungselemente 21 alternativ oder zusätzlich auch beispielsweise durch Schwenken oder Klappen des Mundstücks 10 und/oder einer zugeordneten, nicht dargestellten Abdeckung des Mundstücks 10 um eine sonstige Achse bewirkt werden. Ein entsprechender Mechanismus ist nicht dargestellt, für den Fachmann jedoch ohne weiteres realisierbar. Alternativ oder zusätzlich kann auch ein separates Betätigungselement, insbesondere zum Weiterbefördern der Kapselkammern 4 bzw. der Führungselemente 21, vorgesehen sein.

Insbesondere ist folgender Ablauf bzw. folgende Bedienung möglich. Das Mundstück 10 wird bei geschlossener (nicht dargestellter) Abdeckkappe beispielsweise um 90° gedreht. Hierdurch werden die Kapselkammern 4 bzw. Führungselemente 21 um eine Position weiter bewegt. Beim Weiterdrehen des Mundstücks 10 wird die nächste Kapselkammer 4 unter das Mundstück 10 bewegt, insbesondere aus dem zugehörigen Führungselement 21 herausbewegt, und die darin befindliche Kapsel 3 geöffnet bzw. angestochen. Vorzugsweise erst jetzt und erst, nachdem das Mundstück 10 diese Drehposition erreicht hat, beispielsweise nach 180° bezüglich der Ausgangsdrehstellung, kann die nicht dargestellte Abdeckkappe zum Freigeben des Mundstücks 10 aufgeklappt werden.

Beim Aufklappen der Abdeckkappe des Mundstücks 10 fährt die Öffnungsseinrichtung 5 zurück in die Inhalationsposition, insbesondere werden hierbei die Anstechelemente 6 aus der Kapsel 3 zurückgezogen. Nunmehr kann die Inhalation erfolgen.

Nach dem Inhalieren wird die Abdeckkappe wieder geschlossen. Hierdurch wird die Öffnungseinrichtung gegebenenfalls erst vollständig aus der Kapselkammer 4 herausgezogen und die Kapselkammer 4 mit der entleerten Kapsel 3 wieder freigegeben. Weiter kann hierdurch die Kapselkammer 4 wieder von dem zugeordneten Führungselement 21 aufgenommen werden. Bedarfsweise kann dies jedoch auch erst bei der anschließenden Weiterbewegung der Kapselkammern 4, Führungselemente 21 bzw. Kette erfolgen.

Das Herausbewegen der für die nächste Inhalation vorgesehenen Kapselkammer 4 aus dem jeweiligen Führungselement 21 in die Inhalierposition kann beispielsweise durch die Öffnungseinrichtung 5 - insbesondere gleichzeitig mit dem Einführen der Anstechelemente 6 in die Kapselkammer 4 oder unmittelbar danach - erfolgen.

Je nach Gestaltung der Kapselkammern 4 und Führungselemente 21 bzw. der Anschlüsse, Verschlüsse, Abdeckungen oder dgl. der Kapselkammern 4 ist die genannte Entnahme bzw, das Lösen der Kapselkammern 4 von dem jeweiligen Führungselement 21 zum Inhalieren nicht erforderlich. Vielmehr kann auch ein entsprechendes Öffnen bzw. Anschließen der Kapselkammer 4 oder des Führungselements 21 in der Inhalierposition ohne Entnahme der jeweiligen Kapselkammer 4 realisiert werden, um die gewünschte Luftzuführung und Luftabführung beim Inhalieren zu ermöglichen.

Durch entsprechende Ausbildung der Führung 20, einer sonstigen Führungseinrichtung, der Kapselkammern 4 und/oder der Führungselemente 21 wird eine gewünschte Ausrichtung und Positionierung der Kapselkammern 4 sichergestellt oder ermöglicht, insbesondere um das Einführen der Anstechelemente 6 durch die Anstechöffnungen 7 hindurch in die für die nächste Inhalation vorgesehene Kapselkammer 4 zu ermöglichen.

Die Kapselkammern 4 bzw. Führungselemente 21 können je nach Bedarf beispielsweise schneckenförmig, mäanderförmig, konzentrisch, mehrreihig oder in sonstiger geeigneter Weise geführt bzw. angeordnet und ggf. auch gegenläufig bewegbar sein.

Bei der Ausführungsform gemäß Fig. 11 bis 13 sind die Kapselkammern 4 bzw. Führungselemente 21 vorzugsweise gelenkig, rastend, lösbar oder unlösbar oder in sonstiger geeigneter Weise miteinander verbunden. Alternativ können die Kapselkammern 4 bzw. Führungselemente 21 oder Kapseln 3 auch nicht miteinander verbunden, sondern beispielsweise nur lose von der Führung 20 geführt oder in sonstiger geeigneter Weise im Inhalator 1 aufgenommen sein.

Der Antrieb zum Bewegen der Kapselkammern 4 bzw. Führungselemente 21 bzw. der davon gebildeten Kette oder eines davon gebildeten Bandes oder dgl. kann wahlweise von innen, außen, unten oder oben, beispielsweise über ein Drehkreuz, ein Ritzel oder dgl., erfolgen. Der Antrieb kann wahlweise unmittelbar an den Kapselkammern 4 oder Führungselementen 21 angreifen. Alternativ können die Kapselkammern 4 oder Führungselemente 21 auch an einem vorzugsweise gemeinsamen bzw, durchgehenden oder umlaufenden Antriebsmittel, wie einer Kette, einem Band oder dgl., angeordnet sein, das seinerseits angetrieben wird.

Die voranstehenden Erläuterungen und Ausführungen gelten entsprechend auch für die Kapseln 3, wenn diese nicht in Kapselkammern 4, sondern in sonstigen Verpackungen oder lose im Inhalator 1 aufgenommen sind, so daß für die eigentliche Inhalation ggf. noch eine Überführung in eine Kapselkammer 4 erfolgen muß. Nachfolgend werden einige derartige Ausfiihrungsformen beispielhaft erläutert.

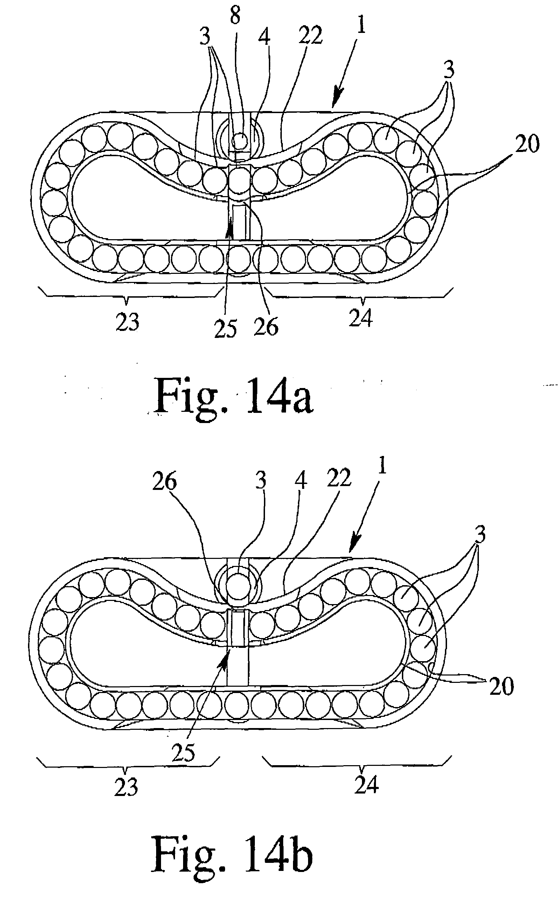

Fig. 14a und b zeigen eine Ausführungsform des vorschlagsgemäßen Inhalators 1, der vorzugsweise nierenförmig, insbesondere mit einer Verjüngung oder Einschnürung 22 in einem mittleren (Gehäuse-)Bereich, ausgebildet ist.
Fig. 14a und 14b zeigen den Inhalator 1 in schematischen Schnitten in verschiedenen Funktionszuständen.

Der Inhalator 1 weist vorzugsweise einen ersten Aufnahmeraum 23 (Kapselreservoir) für unbenutzte bzw. noch gefüllte Kapseln 3 und einen zweiten Aufnahmeraum 24 für benutzte bzw. bereits entleerte Kapseln 3 auf. Die Kapseln 3 sind vorzugsweise schneckenförmig, linienförmig, umlaufend und/oder hintereinander im ersten und/oder zweiten Aufnahmeraum 23, 24 aufgenommen, bzw. durch diese oder den Inhalator 1 förderbar. Es ist insbesondere je auch eine mehrreihige, mäanderförmige oder sonstige Aufnahme möglich.

Die Kapseln 3 sind vorzugsweise mittels eines flexiblen Verbindungs- oder Antriebsmittels - beispielsweise eines Bandes, eines Kunststoffstreifens, eines Blisterstreifens oder dgl. - miteinander verbunden und/oder bewegbar. Die Kapseln 3 können jedoch auch in sonstiger Weise, beispielsweise lose oder wälzlagerartig aufgenommen sein und/oder von einer Führung 20, wie in der zweiten Ausführungsform dargestellt, geführt sein.

Der Inhalator 1 weist vorzugsweise nur eine einzige Kapselkammer 4 auf. Die Kapselkammer 4 ist zu ihrer Befüllung und/oder Entleerung mit Kapseln 3 öffenbar, insbesondere längsseitig bzw. quer zur Durchströmungsrichtung bei der Inhalation. Zur insbesondere selbsttätigen Befüllung bzw. Entleerung der Kapselkammer 4 mit Kapseln 3 weist der Inhalator 1 eine Einrichtung 25 auf, auch Befülleinrichtung genannt.

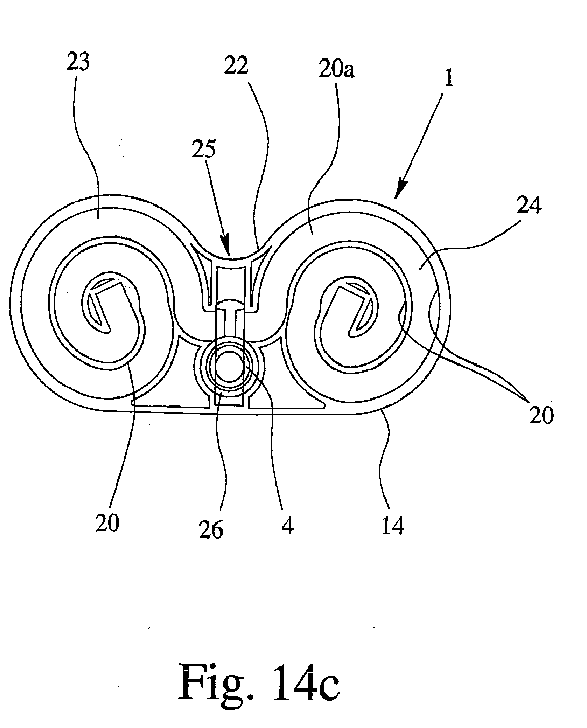

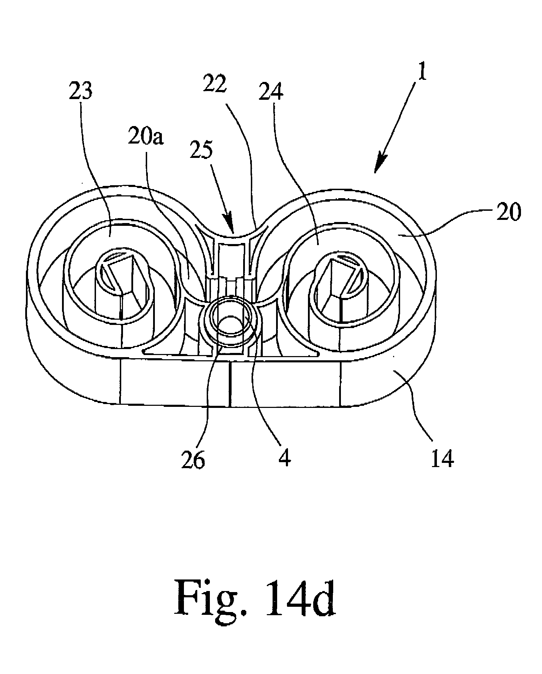

Fig. 14c und 14d zeigen eine sehr ähnliche Ausführungsform des vorschlagsgemäßen Inhalators 1, so daß nachfolgend nur Unterschiede gegenüber der vorangehenden Ausführungsform erläutert werden,

In Fig. 14c und 14d ist die Kapselkammer 4 im geschlossenen Zustand dargestellt. Kapseln 3 sind nicht gezeigt.

Die Führung 20 bildet vorzugsweise wiederum eine Laufbahn 20a für die Kapseln 3, die jedoch nicht umlaufend, sondern schneckenförmig ausgebildet ist.
Fig. 14c und 14d veranschaulichen die vorzugsweise nierenförmige Ausbildung des Zerstäubers 1 insbesondere durch die vorzugsweise im Bereich der Gehäusemitte angeordnete Verjüngung 22. Dies gestattet insbesondere ein sehr angenehmes bzw. ergonomisches Greifen bzw. Halten des Inhalators 1.

Das nicht dargestellte Gehäuseoberteil 15 des Inhalators 1 trägt vorzugsweise das Mundstück 10, das sich insbesondere nach oben in Verlängerung an die Kapselkammer 4 anschließt.

Insbesondere sind die Kapseln 3 im ersten Aufnahmeraum 23 und/oder zweiten Aufnahmeraum 24 schneckenförmig oder in jeder sonstigen geeigneten Weise aufnehmbar.

Fig. 14a der vorangehenden Ausführungsform zeigt die Kapselkammer 4 im geöffneten Zustand. Ein Kapselkammersegment bzw. -teil 26, das insbesondere einen Zentralabschnitt der Kapselkammer 4 bildet, ist aus der Kapselkammer 4 heraus bewegt bzw. heraus gezogen.

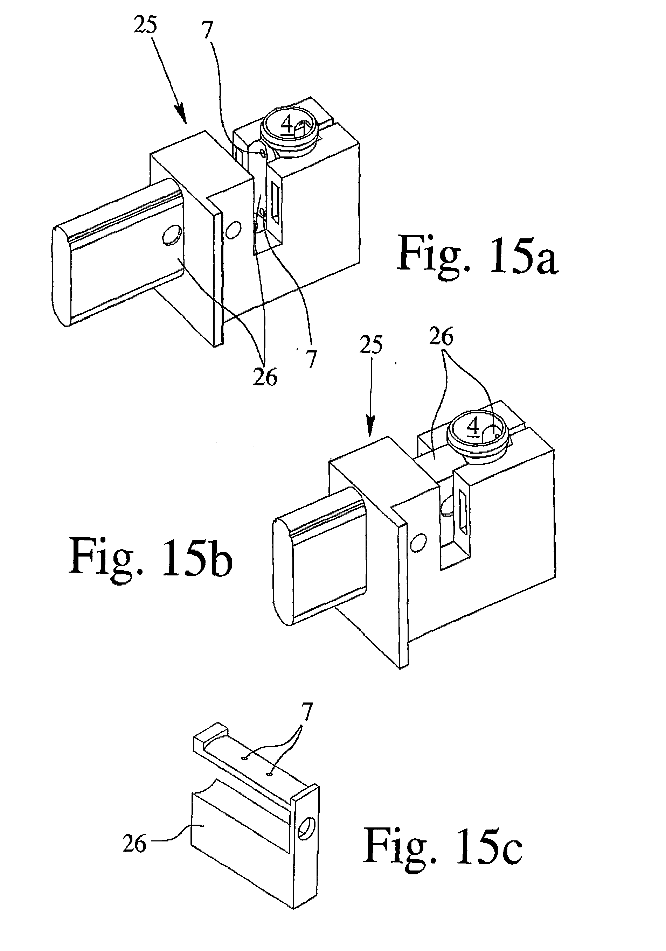

Fig. 15a und b veranschaulichen in schematischen Darstellungen eine mögliche Ausbildung der Einrichtung 25. Insbesondere ist die hier vorzugsweise schieberartige Ausbildung des Kapselkammerteils 26 zu erkennen. Das Kapselkammerteil 26 ist beim Darstellungsbeispiel radial zur Zentralachse der Kapselkammer 4 bzw. Hauptströmungsrichtung in der Kapselkammer 4 und/oder quer zur Hauptbewegungsrichtung der Kapseln 3 bzw. Längsachsen der Kapseln 3 bewegbar.

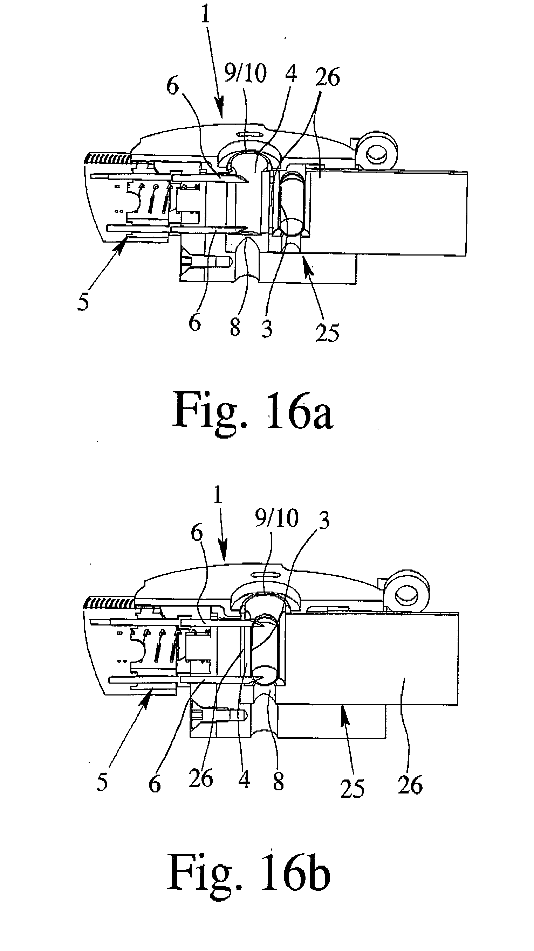

Fig. 16a zeigt die Einrichtung 25 im geöffneten Zustand, also mit ausgerücktem Kapselkammerteil 26. Fig. 15b zeigt die Einrichtung 25 im geschlossenen Zustand, also mit geschlossener Kapselkammer 4. Fig. 15c veranschaulicht in einer perspektivischen Ansicht eine mögliche Ausbildung des Kapselkammerteils 26.

Das Kapselkammerteil 26 ist insbesondere in die Führung 20 oder einen Führungs- bzw. Bewegungsbereich oder eine hier vorzugsweise umlaufende Bahn der Kapseln 3 ausrückbar, so daß das ausgerückte Kapselkammerteil 26 direkt mit der für die nächste Inhalation vorgesehenen Kapsel 3 bei entsprechender Weiterbewegung der Kapseln 3 von dem ersten Aufnahmeraum 23 in den zweiten Aufnahmeraum 24 bestückbar bzw. befüllbar ist.

Anschließend fördert die Einrichtung 25 die aufgenommene Kapsel 3 in die Kapselkammer 4, insbesondere durch entsprechende Verschiebung oder sonstige Bewegung des Kapselkammerteils 26. Hierdurch wird die Kapselkammer 4 mit der Kapsel 3 befüllt und geschlossen, wie in Fig. 14b dargestellt.

Das Öffnen, insbesondere Anstechen, der Kapsel 3 kann wahlweise durch die Einrichtung 25, eine hier nicht dargestellte, separate Öffnungseinrichtung 5 oder in sonstiger geeigneter Weise erfolgen. Insbesondere kann das Öffnen direkt bei der Förderung bzw. Überführung der Kapsel 3 zu der oder in die Kapselkammer 4 oder erst in der Kapselkammer 4 - also erst nach dem Schließen der Kapselkammer 4 - erfolgen. Im letzteren Fall ist es auch möglich, die Kapsel 3 erst unmittelbar vor dem Inhalieren zu öffnen, beispielsweise durch entsprechende Betätigung des Mundstücks 10, einer dem Mundstück 10 zugeordneten, nicht dargestellten Abdeckkappe oder dgl.

Nach dem Entleeren der Kapsel 3 - also nach der Inhalation bzw. Ausgabe der Formulierung 2 - wird die geleerte bzw. benutzte Kapsel 3 wieder mittels der Einrichtung 25 der Kapselkammer 4 entnommen (Entleerung der Kapselkammer 4). Diese Entnahme und das Öffnen der Kapselkammer 4 erfolgen hier durch entsprechendes Herausbewegen des Kapselkammerteils 26 zusammen mit der entleerten Kapsel 3. Wenn sich das Kapselkammerteil 26 wieder in der Ausrückposition befindet, erfolgt ein Weiterbewegen der Kapseln 3 durch einen nicht dargestellten Mechanismus bzw. Antrieb (beispielsweise ein Drehen oder Schwenken des Mundstücks 10 oder Betätigen eines sonstigen, nicht dargestellten Betätigungselements des Inhalators 1). Hierdurch wird die leere Kapsel 3 in den zweiten Aufnahmeraum 24 weitergefördert.

Bei der vorliegenden Ausführungsform erfolgt eine mehrfache Verwendung der Kapselkammer 4. Dies gestattet einen besonders kompakten, raumsparenden Aufbau des Inhalators 1 oder - bei gleicher Baugröße - die Aufnahme einer größeren Anzahl von Kapseln 3.

Das automatische Befüllen und Entleeren der Kapselkammer 4 mit den Kapseln 3 gestattet eine sehr einfache und insbesondere hygienische Handhabung. Das Öffnen der einzelnen Kapseln 3 kann - wie bereits angesprochen - wahlweise beim Transport bzw. beim Befüllen der Kapselkammer 4 erfolgen. Hierzu kann die entsprechende Öffnungseinrichtung ggf. auch ein feststehendes Öffnungselement, wie eine Schneide oder dgl., im Bewegungsweg aufweisen, so daß ein zwangsweises Öffnen - beispielsweise längsseitiges Aufschneiden - der Kapseln 3 beim Befüllen der Kapselkammer 4 erfolgt. Das Öffnen kann jedoch auch bereits bei der Entnahme oder Zuförderung der Kapseln 3 aus dem ersten Aufnahmeraum 23 oder einem sonstigen Reservoir 31 erfolgen. Beispielsweise kann auch jede Kapsel 3 bedarfsweise einzelweise verpackt und/oder in einem sonstigen geeigneten Behältnis aufgenommen sein, wobei dann ein vorzugsweise einzelweises Öffnen der Verpackung bzw. des Behältnisses bzw. Entnehmen der Kapseln 3 und ggf, gleichzeitiges Öffnen der Kapseln 3 erfolgt.

Fig. 16a und b zeigen in ausschnittsweisen, schematischen Schnitten des Inhalators 1 eine mögliche Realisierung der Öffnungseinrichtung 5 und/oder der Befülleinrichtung 25. Fig. 16a zeigt die Kapselkammer 4 im geöffneten Zustand, Fig. 16b im geschlossenen Zustand.

Beim Darstellungsbeispiel wird die Kapsel 3 beim Schließen der Kapselkammer 4 vorzugsweise direkt geöffnet bzw. angestochen. Dies erfolgt dadurch, daß die Anstechelemente 6 bereits in die leere bzw. geöffnete Kapselkammer 4 hineinragen. Beim Hineinbewegen bzw. Schließen können die Anstechelemente 6 die Anstechöffnungen 7 im Kapselkammerteil 26 durchdringen und direkt die Kapsel 3 öffnen bzw. anstechen. Zur Inhalation werden anschließend die Anstechelemente 6 aus der Kapsel 3 zurückgezogen, beispielsweise durch Öffnen des Mundstücks 10 oder eine sonstige Betätigung.

An Stelle des gezeigten Aufbaus der Kapselkammer 4 sind jedoch auch sonstige Konstruktionen einsetzbar, die ein Öffnen der Kapselkammer 4, insbesondere längsseitig, bedarfsweise aber auch in der Äquatorialebene, einlaß- oder auslaßseitig oder in sonstiger Weise ermöglichen.

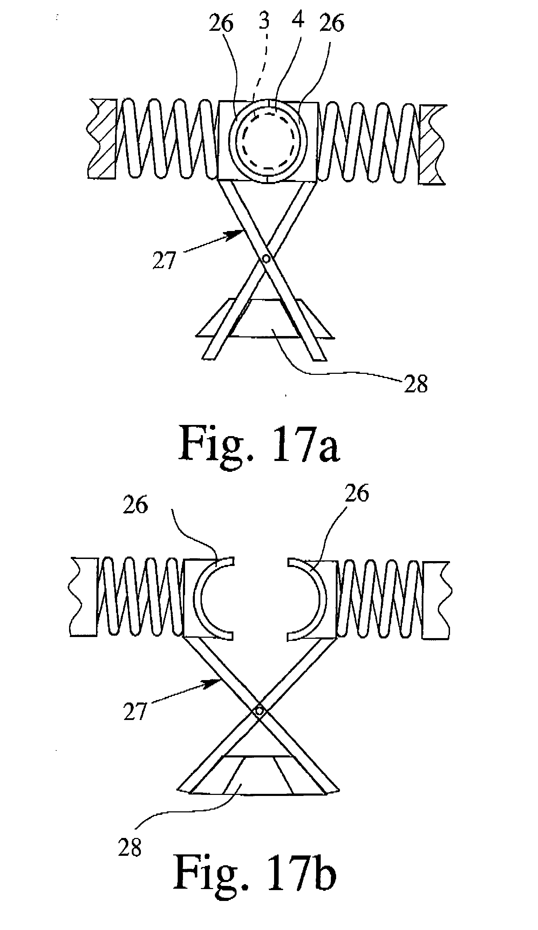

Fig. 17a und b zeigen in schematischen, schnittartigen Darstellungen eine andere Ausführungsform der öffenbaren Kapselkammer 4. Die Kapselkammer 4 ist hier insbesondere längsgeteilt bzw. aus mindestens zwei Segmenten oder Teilen 26 aufgebaut, die beispielsweise durch eine zangenartige Mechanik 27 auseinander und wieder zusammen bewegbar sind. Fig. 17a zeigt die Kapselkammer 4 im geschlossenen Zustand. Fig. 17b zeigt die Kapselkammer 4 im geöffneten Zustand, also mit voneinander abgerückten Teilen 26. Vorzugsweise erfolgt das Öffnen gegen Federkraft, beispielsweise mittels eines Steuerschiebers 28, der die Zangenmechanik 27 aufspreizt und so die Kapselkammer 4 öffnen kann.

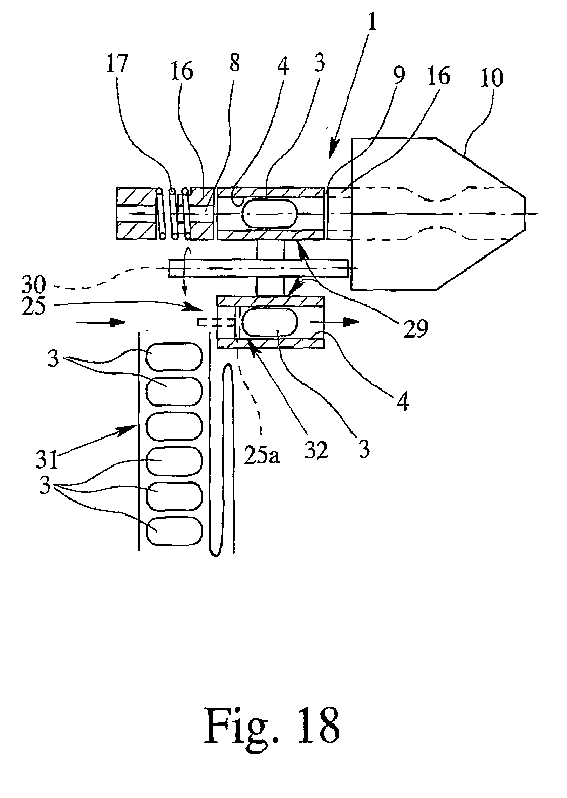

Fig. 18 zeigt eine weitere Ausführungsform des vorschlagsgemäßen Inhalators 1 in einer nur sehr schematischen ausschnittsweisen und teilschnittartigen Darstellung. Der Inhalator 1 weist vorzugsweise mehrere - mindestens zwei - Kapselkammern 4 auf, die insbesondere revolverartig an einer Dreh- bzw. Fördereinrichtung oder Schwenkeinrichtung 29 angeordnet bzw. von dieser gebildet sind. Die Kapselkammern 4 sind vorzugsweise um eine parallel zur Längserstreckung oder Hauptdurchströmungsrichtung verlaufende Schwenkachse 30 schwenkbar. Bei dieser Ausführungsform werden die an der Schwenkeinrichtung 29 angeordneten oder davon gebildeten Kapselkammern 4 mehrfach verwendet.

Bei zwei Kapselkammern 4 ist insbesondere abwechselnd eine Kapselkammer 4 in die Inhalationsposition zum Anschluß von Einlaß 8 und Auslaß 9 schwenkbar. Die jeweils andere Kapselkammer 4 nimmt dann vorzugsweise eine Befüllposition ein, in der diese Kapselkammer 4 mit der für die nächste Inhalation vorgesehenen Kapsel 3 befüllbar ist. Die zuvor bereits entleerte Kapsel 3 wird dabei oder zuvor aus der Kapselkammer 4 entfernt, insbesondere ausgeschoben, wie in Fig. 18 angedeutet.

Vorzugsweise erfolgt das Befüllen und Entleeren der Kapselkammer 4 mit Kapseln 3 durch die Einrichtung 25, die insbesondere ein Förderelement 25a aufweist, wie in Fig. 18 angedeutet. Die Befüll- und Entleerrichtung ist durch Pfeile P angedeutet. Insbesondere erfolgt das Befüllen und Entleeren der Kapselkammer 4 in Längs- bzw. axialer Richtung und/oder durch das vorzugsweise schieberartige Förderelement 25a, das ggf. gleichzeitig die Kapselkammer 4 bei Ausschieben oder Entnehmen der entleerten bzw. benutzten Kapsel 3 reinigen kann. Jedoch sind auch andere konstruktive Lösungen und/oder Befüll- bzw. Entleerungsrichtungen möglich.

Vorzugsweise weist der Inhalator 1 eine Einrichtung 32 zur Reinigung seiner mehrfach verwendbaren Kapselkammer(n) 4 auf. Diese Reinigungseinrichtung 32 kann bedarfsweise von der Einrichtung 25 zur Befüllung und Entleerung der Kapselkammer 4 bzw. deren Förderelement 25a gebildet sein, wie in Fig. 18 angedeutet, das beispielsweise als Schieber oder dgl. in die Kapselkammer 4 zur Befüllung bzw. Entleerung einführbar und mit einer Bürste oder einem sonstigen Reinigungselement versehen ist, um die Kapselkammer 4 entsprechend vorzugsweise bei jeder Befüllung oder Entleerung zu reinigen.

Nach dem in Fig. 18 angedeuteten Entleeren der Kapselkammer 4 wird diese wieder mit der nächsten Kapsel 3 befüllt. Vorzugsweise wird hierzu die nächste Kapsel 3 mittels des Förderelements 25a in die sich in der Befüllposition befindende (in Fig. 18 die untere) Kapselkammer 4 geschoben bzw. eingeführt. Beim Darstellungsbeispiel wird die Reinigungseinrichtung vorzugsweise also von der Einrichtung 25 bzw. deren Förderelement 25a gebildet.

Insbesondere im Falle der Schwenkeinrichtung 29 besteht beispielsweise bei Ausbildung von drei Kapselkammern 4 die Möglichkeit, daß die Kapselkammern 4 abwechselnd drei Positionen einnehmen, nämlich die Befüllposition, die Inhalationsposition, und eine (zusätzliche) Reinigungsposition, Durch entsprechendes Weiterdrehen wandert jede Kapselkammer 4 von einer Position zur nächsten Position. In der Reinigungsposition kann dann mittels der bereits angesprochenen Reinigungseinrichtung die benutzte Kapsel 3 aus der jeweiligen Kapselkammer 4 entfernt werden und eine Reinigung der jeweiligen Kapselkammer 4 erfolgen.

Die noch unbenutzten Kapseln 3 werden vorzugsweise einem in Fig. 18 nur schematisch angedeuteten Kapselreservoir 31 entnommen. Dieses Kapselreservoir 31 kann die Kapseln 3 beispielsweise als Schüttung oder in sonstiger geeigneter Weise, insbesondere als Stapel, Band, Kette oder in diskreten Aufnahmen, Halterungen oder Räumen, enthalten bzw. aufnehmen, Bedarfsweise ist in das Kapselreservoir 31 auch eine Fördereinrichtung oder dgl. integriert und/oder von der Einrichtung 25 zur Befüllung der Kapselkammer 4 (siehe Fig. 15a und b sowie Fig. 16a und b) gebildet.

Beim Darstellungsbeispiel erfolgt die Entnahme der Kapseln 3 aus dem Kapselreservoir 31 vorzugsweise einzelweise. Insbesondere kann die Entnahme über eine Schleuse oder dgl. erfolgen, so daß die noch unbenutzten, im Kapselreservoir 31 befindlichen Kapseln 3 weiterhin möglichst hermetisch abgeschlossen bleiben, insbesondere gegen Umwelteinflüsse geschützt werden.

Das Kapselreservoir 31 kann insbesondere magazinartig ausgebildet sein. Es ist entweder in den Inhalator 1 integriert oder an diesen ankoppelbar. Bedarfsweise ist das Kapselreservoir 31 auch auswechselbar und/oder nachfüllbar.

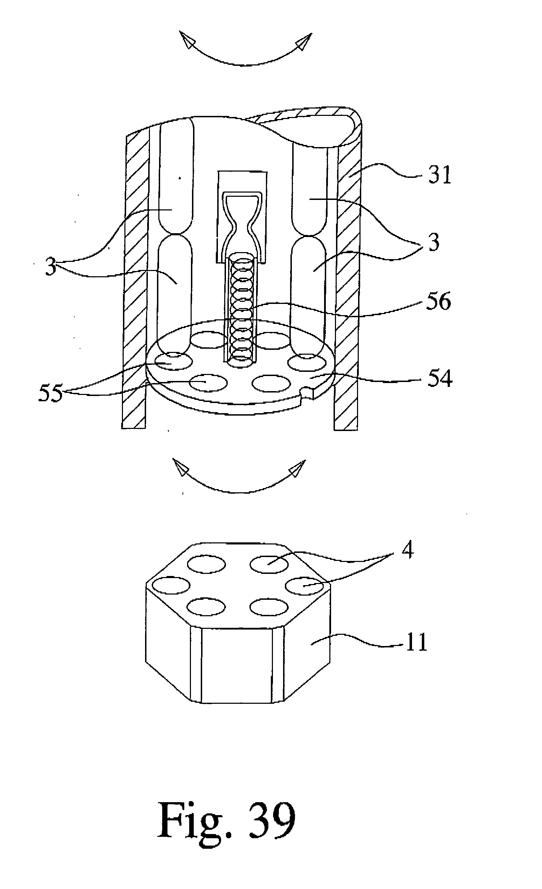

Das Kapselreservoir 31 kann beispielsweise rohrförmig ausgebildet sein, wobei die Kapseln 3 beispielsweise parallel zur Längsachse des Kapselreservoirs 31 ausgerichtet bzw. aufgenommen sein können, wie später beispielhaft anhand Fig. 39 bis 42 erläutert.

Die Entnahme bzw. Ausgabe der Kapseln 3 kann einzeln nacheinander oder ggf. auch gruppenweise erfolgen. Dies kann beispielsweise durch eine Drehmechanik, eine Schiebervorrichtung, eine Federmechanik oder in sonstiger geeigneter Weise erfolgen, wie später beispielhaft anhand Fig. 39 bis 42 erläutert.

Die Entnahmevorrichtung bzw. Schleuse oder dgl. ist vorzugsweise jeweils wieder schließbar, um die noch im Kapselreservoir 31 befindlichen Kapseln 3 zu schützen. Zusätzlich oder alternativ ist im Kapselreservoir 31 vorzugsweise ein Trockenmittel vorgesehen, insbesondere um die Kapseln 3 vor übermäßiger Feuchtigkeit zu schützen.

Alternativ oder zusätzlich können die Kapseln 3 auch mit einer zusätzlichen Umverpackung versehen sein. Insbesondere sind die Kapseln 3 einzeln ver- bzw. eingepackt.

Zusätzlich oder alternativ kann auch das Kapselreservoir 31 selbst mit einer Umverpackung versehen sein, die beispielsweise erst beim Einsetzen des Kapselreservoirs 31 in den Inhalator 1 oder erst bei der erstmaligen Entnahme einer Kapsel 3 geöffnet wird.

Die benutzten bzw. entleeren Kapseln 3 werden vorzugsweise von dem Inhalator 1 aufgenommen, beispielsweise in einen zweiten Aufnahmeraum wie bei der vorhergehenden Ausführungsform. Jedoch können die benutzten bzw. entleerten Kapseln 3 bedarfsweise auch ausgegeben werden. Dies gestattet einen kompakteren Aufbau des Inhalators 1.

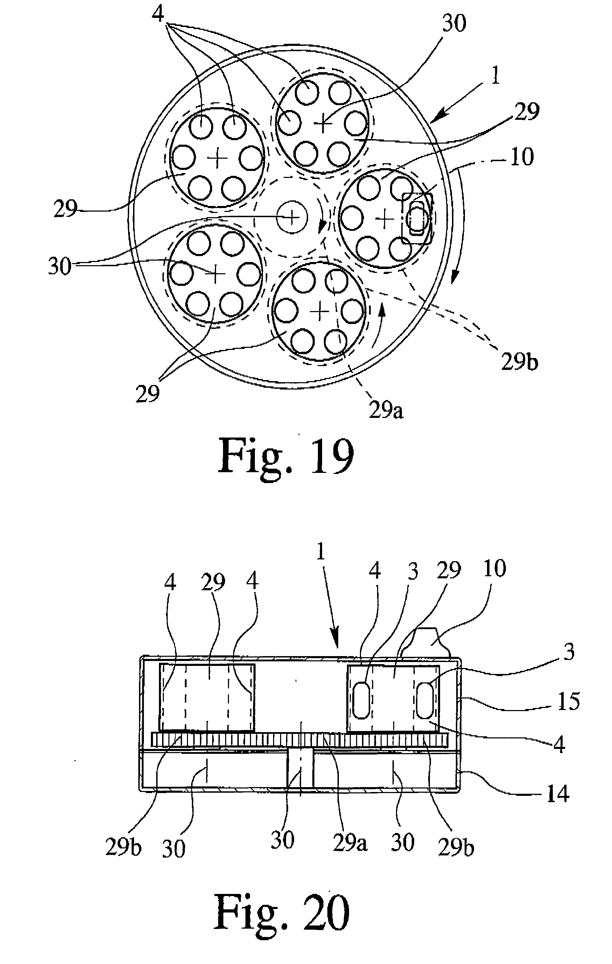

Fig. 19 zeigt eine weitere Ausführungsform des vorschlagsgemäßen Inhalators 1 in einer nur schematischen, offenen Draufsicht. Fig. 20 zeigt den Inhalator 1 in einem schematischen Schnitt. Der Inhalator 1 weist vorzugsweise mehrere Dreh- bzw. Fördereinrichtungen oder Schwenkeinrichtungen 29 mit jeweils mehreren Kapsekammern 4 auf. Die Schwenkachsen 30 der Schwenkeinrichtung 29 verlaufen vorzugsweise parallel zueinander. Die Schwenkeinrichtungen 29 sind vorzugsweise in einer gemeinsamen Ebene oder alternativ versetzt, übereinander oder überlagert angeordnet.

Die Kapselkammern 4 sind vorzugsweise jeweils nur einmalig verwendbar und insbesondere jeweils bereits mit einer Kapsel 3 vorkonfektioniert bzw. befüllt. Die Kapselkammern 4 sind vorzugsweise verschlossen, insbesondere durch nicht dargestellte Abdeckungen 12, und einzelweise öffenbar.

Die Längsrichtungen bzw. Durchströmungsrichtungen der Kapseln 3 und Kapselkammern 4 verlaufen vorzugsweise parallel zueinander und parallel zu den Schwenkachsen 30. Alternativ können diese jedoch auch quer und insbesondere radial zu den Schwenkachsen 30 verlaufen.

Die Schwenkeinrichtungen 29 sind wahlweise gemeinsam drehbar oder unabhängig voneinander. Im erstgenannten Fall können diese beispielsweise peripher über ihren Außenumfang mit der jeweils benachbarten Schwenkeinrichtung 29 in Eingriff stehen oder beispielsweise in der Art eines Planetengetriebes, insbesondere über ein Sonnenrad 29a und Zahnräder 29b der Schwenkeinrichtungen 29, wie in Fig. 19 und dem schematischen Schnitt gemäß Fig. 20 angedeutet, oder auf sonstige geeignete Weise, beispielsweise mit einem umlaufenden Band, Riemen oder dgl., getrieblich verbunden sein.

Zusätzlich ist die Anordnung der Schwenkeinrichtungen 29 vorzugsweise relativ zum Mundstück 10 drehbar, so daß nacheinander die einzelnen Schwenkeinrichtungen 29 in die Inhalierposition bringbar sind, um die jeweilige Kapselkammer 4 und die darin enthaltene Kapsel 3 öffnen und anschließend die Formulierung 2 aus der geöffneten Kapsel 3 ausgeben zu können. Je nach Ausgestaltung und Bedarf ist es möglich, daß erst alle Kapselkammern 4 einer Schwenkeinrichtung 29 hintereinander für die Inhalation benutzt werden und dann zur nächsten Schwenkeinrichtung 29 gewechselt wird oder daß zunächst eine Kapselkammer 4 jeder Schwenkeinrichtung 29 zur Inhalation und erst danach jeweils eine weitere Kapselkammer 4 jeder Schwenkeinrichtung 29 zur Inhalation verwendet wird.

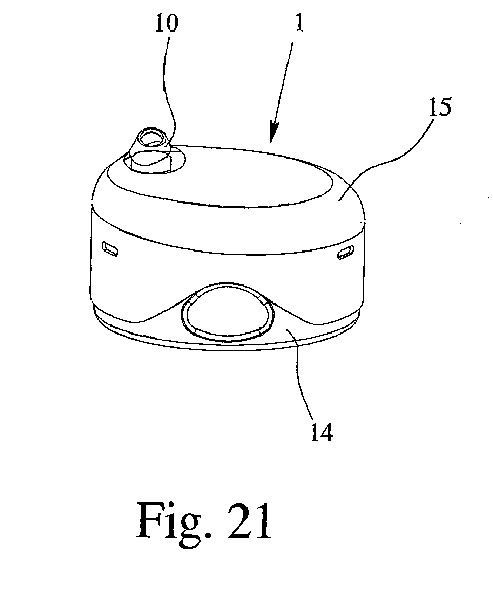

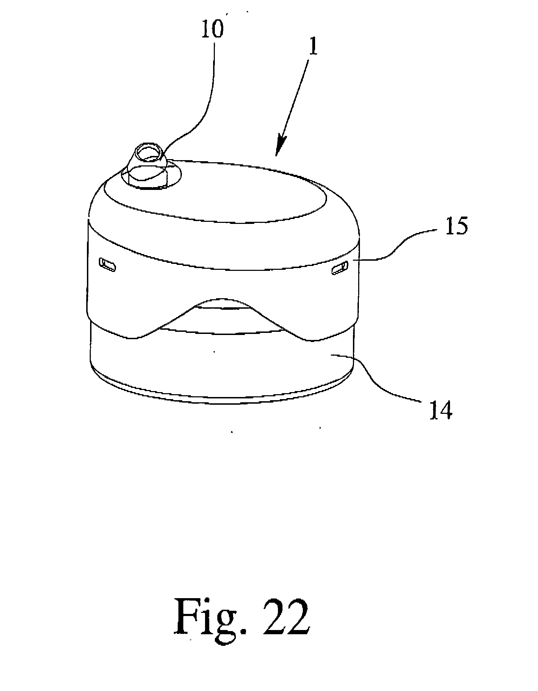

Fig. 21 und 22 zeigen eine weitere Ausfübrungsform des vorschlagsgemäßen Inhalators 1. in Fig. 21 befindet sich der Inhalator 1 im Transportzustand oder Inhalationszustand. In Fig. 22 ist eine Zwischenphase dargestellt, in der das Gehäuseoberteil 15 insbesondere um 90° gegenüber dem Gehäuseunterteil 14 verdreht und vorzugsweise gleichzeitig axial angehoben ist. Generell kann es sich bei dem Gehäuseoberteil 15 und Gehäuseunterteil 14 um beliebige, vorzugsweise äußere Teile des Inhalators 1 handeln. Vorzugsweise über- bzw. umgreift das Oberteil 15 das Unterteil 14 jedoch topfartig oder umgekehrt.

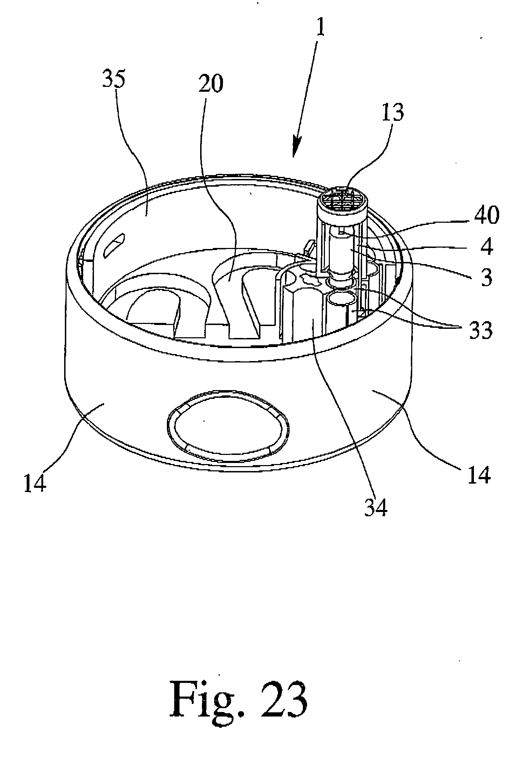

Fig. 23 zeigt in einer teilweise geschnittenen Darstellung schematisch den inneren Aufbau des geöffneten Inhalators 1. Der Inhalator 1 weist vorzugsweise nur eine einzige Kapselkammer 4 auf. Die in Fig. 23 nur angedeuteten Kapseln 3 sind vorzugsweise von einem nicht dargestellten Band, beispielsweise einem Blisterband, einer Kette oder dgl. aufgenommen, beispielsweise in vorzugsweise hülsenartigen Aufnahmen 33 aufgenommen und/oder mittels einer Fördereinrichtung 34, wie einem Transportrad, förderbar.

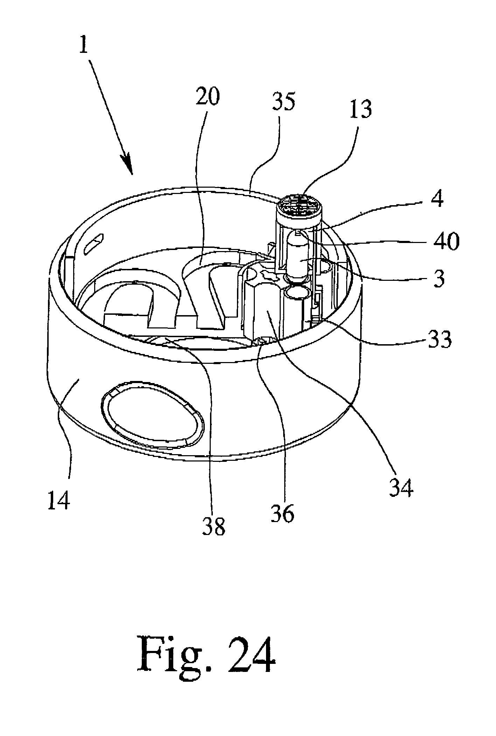

Zum Antrieb weist der Inhalator 1 insbesondere eine im Inhaltatorgehäuse drehbare Steuerhülse 35 mit einem zugeordneten Getriebe 36 (Fig. 24 bis 27) auf. Die Steuerhülse 35 ist vorzugsweise mit der Führung 20, insbesondere in Form einer Bahn oder Nut, für die Kapseln 3 versehen und trägt vorzugsweise die Fördereinrichtung 34, die Kapselkammer 4 und/oder die nicht dargestellte Öffnungseinrichtung 5.

Der Inhalator 1 weist ferner ein nicht dargestelltes Kammerteil 26 auf, das hier insbesondere als Unterteil der geschnitten dargestellten Kapselkammer 4 und/oder stößelartig ausgebildet ist und ein Element der Einrichtung 25 zur Befüllung und Entleerung der Kapselkammer 4 mit Kapseln 3 darstellt.

Nachfolgend wird die Funktionsweise des Inhalators 1 anhand der Fig. 23 bis 27 erläutert, die den Inhalator 1 jeweils in ähnlichen schematischen Darstellungen in unterschiedlichen Zuständen zeigen. Aus Vereinfachungsgründen ist das Oberteil 15 weggelassen.

Im Transport- bzw. Ruhestand gemäß Fig. 23 befindet sich die letzte, bereits entleerte Kapsel 3 noch in der Kapselkammer 4.

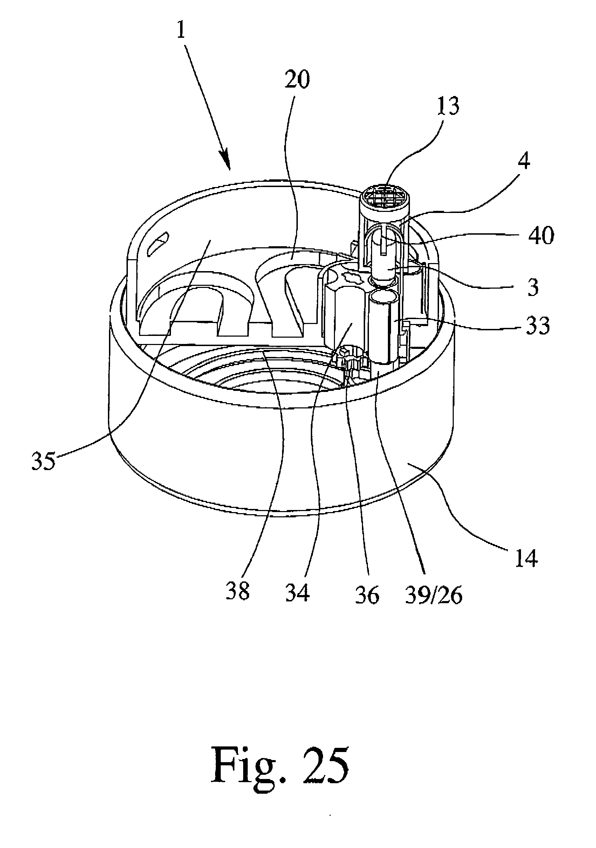

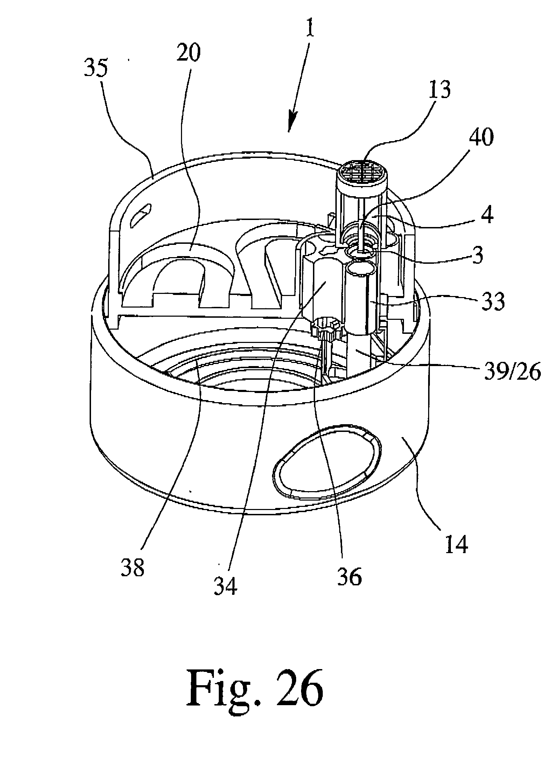

Die beiden Gehäusehälften bzw. Teile 14 und 15 werden gegeneinander verdreht. Insbesondere aufgrund einer Steuerkurve oder einer sonstigen getrieblichen Verbindung führt das Verdrehen der Steuerhülse 35 zum Gehäuseunterteil 14 dazu, daß die Steuerhülse 35 axial vom Gehäuseunterteil 14 angehoben wird. Das Kapselkammerteil 26 ist auf einer Kreisbahn verschieblich, jedoch axial von einer Führung 38 am Gehäuseunterteil 14 gehalten. Die Axial- bzw. Hubbewegung der Steuerhülse 35 führt dazu, daß das Kapselkammerteil 26 von der Kapselkammer 4 axial abrückt, insbesondere mit einem Stößel 39 (Fig. 25, 26) aus der für die noch in der Kapselkammer 4 befindliche Kapsel 3 vorhandenen Aufnahme 33 zurückgezogen wird. Des weiteren erfolgt bei dieser anfänglichen Drehbewegung das Öffnen - insbesondere Anstechen - der nächsten, insbesondere noch in einem Band bzw. der Führung 20 bzw. in der nächsten Aufnahme 33 befindlichen Kapsel 3.

Fig. 25 zeigt einen weitergedrehten Zustand. Der Stößel ist weiter aus der Aufnahme 33 für die leere Kapsel 3 im Band 44 ausgefahren. Ein Rückholelement 40, das ggf. auch vom Gitter 13 gebildet sein oder dieses bilden kann und/oder ggf käfigartig ausgebildet ist, greift an der Kapsel 3 an und schiebt diese zurück in die leere Aufnahme 33. Weiter wird die nicht dargestellte Öffnungseinrichtung 5 mit ihren Anstechelementen 6 oder dgl. aus der nachfolgenden, bereits geöffneten bzw. angestochenen Kapsel 3 zurückgezogen.

Die Drehbewegung bzw. Verdrehung des Gehäuseoberteils 15 zum Gehäuseunterteil 14 wird fortgeführt. Fig. 26 zeigt einen Zustand, bei dem die Steuerhülse 35 bereits um etwa 90° oder 180° zum Gehäuseunterteil 14 gedreht ist. Der Stößel 39 bzw. das Teil 26 ist komplett aus der Aufnahme 33 zurückgezogen. Die leere Kapsel 3 ist aus der Kapselkammer 4 wieder zurück in die Aufnahme 33 gefördert. Die Anstechelemente 6 sind wieder vollständig aus der nachfolgenden Kapsel 3 und Aufnahme 33 zurückgezogen.

Nun fördert die Fördereinrichtung 34 die Kapseln 3 bzw. Aufnahmen 33 um einen Schritt weiter, insbesondere die nächste, bereits geöffnete Kapsel 3 unter oder in die offene Kapselkamnner 4. Dies erfolgt insbesondere durch Weiterfördern des von den Kapseln 3 oder Aufnahmen 33 gebildeten Bandes 44, beispielsweise durch Drehen des dargestellten Transportrads oder dgl. Jedoch sind hier auch sonstige konstruktive Lösungen möglich.

Der Antrieb der Fördereinrichtung 34 erfolgt insbesondere durch das Getriebe 36, wobei für die vorgesehene, schrittweise Weiterbewegung der Kapseln 3 bzw. des Bandes entsprechende Einrichtungen, wie Sperrklinken, Federelemente oder dgl. eingesetzt werden können, um die gewünschte, insbesondere schrittweise Förderung abgestimmt auf die anderen Abläufe zu erreichen.

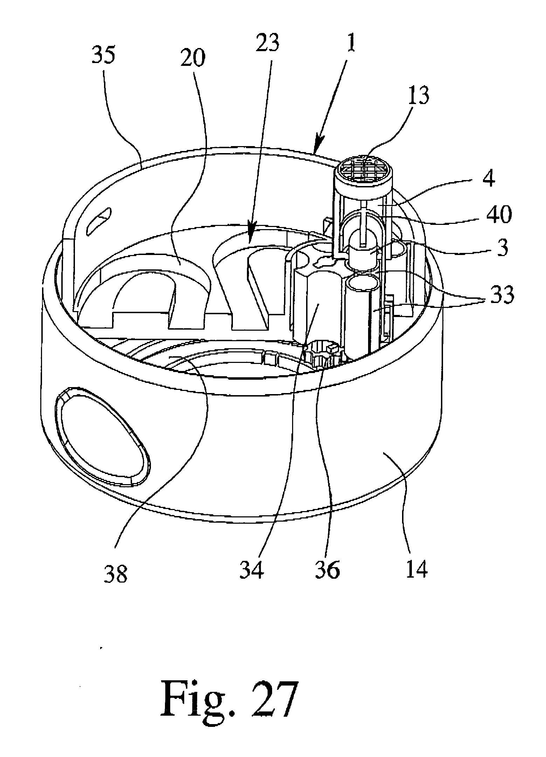

Beim Weiterdrehen fährt der Stößel 39 wieder in die nächste Aufnahme 33 ein und fördert bzw. schiebt dadurch die nächste, bereits geöffnete Kapsel 3 in die Kapselkammer 4, wie in Fig. 27 angedeutet. Diese Axialbewegung wird insbesondere wieder dadurch erreicht, daß vorzugsweise aufgrund der nicht dargestellten Steuerkurve die Steuerhülse 35 bzw. die davon getragene Kapselkammer 4 axial an das Gehäuseunterteil 14 bzw. Kapselkammerteil 26 mit dem Stößel 39 herangeführt wird.

Schließlich erreicht der Inhalator 1 beim Weiterdrehen wieder den in Fig. 23 gezeigten Zustand, in dem eine geöffnete Kapsel 3 vollständig in die Kapselkammer 4 eingeführt ist. Nunmehr kann die Inhalation erfolgen. Die Formulierung 2 wird dann - wie bereits beschrieben - mit dem durch die Kapselkammer 4 strömenden Luftstrom ausgetragen.

Nach dem Inhalieren ist keine weitere Betätigung erforderlich, jedoch möglich. Vielmehr erfolgt eine weitere Betätigung vorzugsweise erst unmittelbar vor der nächsten Inhalation bzw. Ausgabe der nächsten Dosis.

Beim Darstellungsbeispiel ergibt sich eine sehr einfache Betätigung. Insbesondere ist nur ein Drehen der Teile 14, 15 relativ zueinander erforderlich. Vorzugsweise ist beim Darstellungsbeispiel jeweils ein Drehen um 180° oder 360° erforderlich, um den voranstehend beschriebenen Ablauf einmal zu durchlaufen, also die Ausgabe der nächsten Dosis zu ermöglichen.

Ein besonderer Aspekt der vorliegenden Ausführungsform liegt darin, daß der Drehbewegung eine Hub- bzw. Axialbewegung - insbesondere aufgrund einer Zwangsführung - überlagert ist. Dies gestattet bei einfacher Handhabung auch komplexe bzw. kompliziertere Bewegungsabläufe im Inhalator 1. Insbesondere gestattet die axiale Vergrößerung des Inhalators 1 im Verlauf der Betätigung mehr Platz zur Ausführung entsprechender Vorgänge, wie das Auswechseln der Kapsel 3 in der Kapselkammer 4. Jedoch sind auch andere konstruktive Lösungen möglich.

Der vorschlagsgemäße Inhalator 1 ist vorzugsweise mit einer nicht dargestellten Abdeckkappe für das Mundstück 10 versehen. Um eine besonders einfache Bedienung sicherzustellen, kann vorgesehen sein, daß die Abdeckkappe erst dann geöffnet werden kann, wenn eine vollständige Betätigung des Inhalators 1 - insbesondere ein vollständiges Verdrehen der beiden Teile 14 und 15 gegeneinander - erfolgt ist. Die Abdeckkappe schützt den Inhalator 1, insbesondere die Kapselkammer 4 und die sonstigen Luftführungen vor unerwünschter Verschmutzung oder dgl.

Ein weiterer Vorteil des vorschlagsgemäßen Inhalators 1 ist dessen einfacher Aufbau aus wenigen Teilen.

Vorzugsweise sind im Inhalator 1 sowohl ein erster Aufnahmeraum 23 (Fig. 27) für die noch nicht benutzten Kapseln 3 bzw. das Band 44 oder die Aufnahmen 33 mit den noch nicht benutzten Kapseln 3 und ein nicht dargestellter zweiter Aufnahmeraum 24 für die benutzten Kapseln 3 bzw. das Band 44 mit den benutzten Kapseln 3 gebildet. Alternativ kann jedoch beispielsweise auch ein gemeinsames Kapselreservoir 31 sowohl für die noch unbenutzten als auch für die benutzten Kapseln 3 vorgesehen sein. Dies gestattet eine Verringerung der Baugröße des Inhalators 1 bei gleicher Kapazität. Im letztgenannten Fall kann das Band 33 auch als Endlosband oder -bahn ausgebildet sein, obwohl dies nicht erforderlich ist.

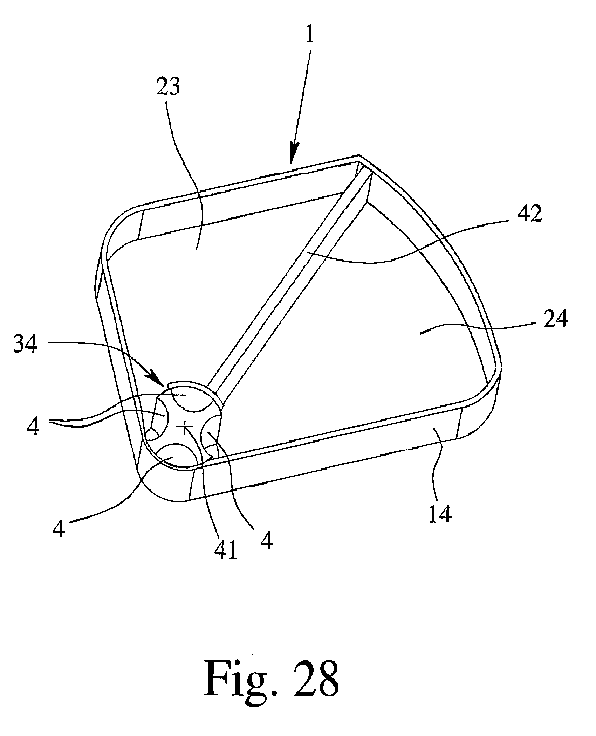

Fig. 28 zeigt schematisch den Aufbau einer weiteren Ausführungsform des vorschlagsgemäßen Inhalators 1. Der Inhalator 1 weist mehrere Kapselkammem 4 auf, die von der Fördereinrichtung 34 bzw. einem Antriebsrad 41 mit gebildet sind. Die vorzugsweise bandartig oder lose vorliegenden, nicht dargestellten Kapseln 3 werden durch Drehen des Antriebsrads 41 weitergefördert und nacheinander in die vorzugsweise zunächst seitlich offenen Kapselkammern 4 aufgenommen. Beispielsweise wird das Antriebsrad 41 jeweils in Schritten von 90° gedreht (beim Darstellungsbeispiel sind vier Kapselkammern 4 am Antriebsrad 41 gebildet, die in 90° Schritten gleichmäßig verteilt sind). So wird nacheinander eine der Kapselkammern 4 mit einer Kapsel 3 in die beispielsweise in der Gehäuseecke befindliche Inhalationsposition gebracht bzw. bewegt und insbesondere von einer Wandung, wie dem Gehäuseunterteil 14, seitlich geschlossen.

Das Öffnen bzw. Anstechen der Kapseln 3 erfolgt vorzugsweise bei der oder durch die Förderung, insbesondere Drehung des Antriebsrads 41, so daß die nächste Kapsel 3 selbsttätig geöffnet wird, insbesondere bevor sie die Inhalationsposition erreicht.

Ein auch unabhängig von der Ausbildung des Antriebsrads 41 mit Kapselkammern 4 realisierbarer Aspekt der vorliegenden Erfindung liegt darin, die Kapseln 3 vorzugsweise bei ihrer Förderung selbsttätig zu öffnen. Dies kann durch entsprechende Ausbildung der Öffnungseinrichtung 5, der Befülleinrichtung 25, der Schwenkeinrichtung 29, der Fördereinrichtung 34 und/oder des Antriebsrads 41 oder sonstiger Komponenten des Inhalators 1 realisiert werden und erleichtert die Bedienung für den Benutzer bzw. Patienten.

Beim Darstellungsbeispiel weist der Inhalator 1 einen ersten Aufnahmeraum 23 für die noch unbenutzten, also noch gefüllten Kapseln 3 und einen zweiten Aufnahmeraum 24 für die bereits benutzten, also entleerten Kapseln 3 auf. Gemäß einem besonders bevorzugten - auch unabhängig von dem voranstehend beschriebenen Funktionsaufbau des vorliegenden Inhalators 1 - realisierbaren Aspekt ist zwischen den beiden Aufnahmeräumen 23 und 24 vorzugsweise eine bewegbare, flexible und/oder elastische Trennwand 42 vorgesehen. Dies gestattet einen besonders kompakten Aufbau durch entsprechende Verschiebung, Verformung oder sonstige Modifikationen der Trennwand 42, da der für die Aufnahmeräume 23 und 24 insgesamt zur Verfügung stehende Raum optimal ausgenutzt werden kann, und zwar vorzugsweise zunächst im wesentlichen nur für den ersten Aufnahmeraum 23 mit noch gefüllten Kapseln 3. Mit zunehmender Benutzung wird der Aufnahmeraum 23 durch Bewegung, Verschiebung und/oder Verformung der Trennwand 42 verkleinert und im Gegenzug der zweite Aufnahmeraum 24 zur Aufnahme der benutzten, leeren Kapseln 3 vergrößert. Bedarfsweise kann die Trennwand 42 auch als elastisches Band, verschiebbares Segment, Folie o.dgl. ausgebildet sein.

Die bewegbare und/oder verformbare Trennwand 42 kann auch zur Abtrennung von zwei Aufnahmeräumen 23, 24 für Kapselkammern 4 - insbesondere unbenutzten Kapselkammern 4 einerseits und benutzten Kapselkammern 4 andererseits - in entsprechender Weise dienen.

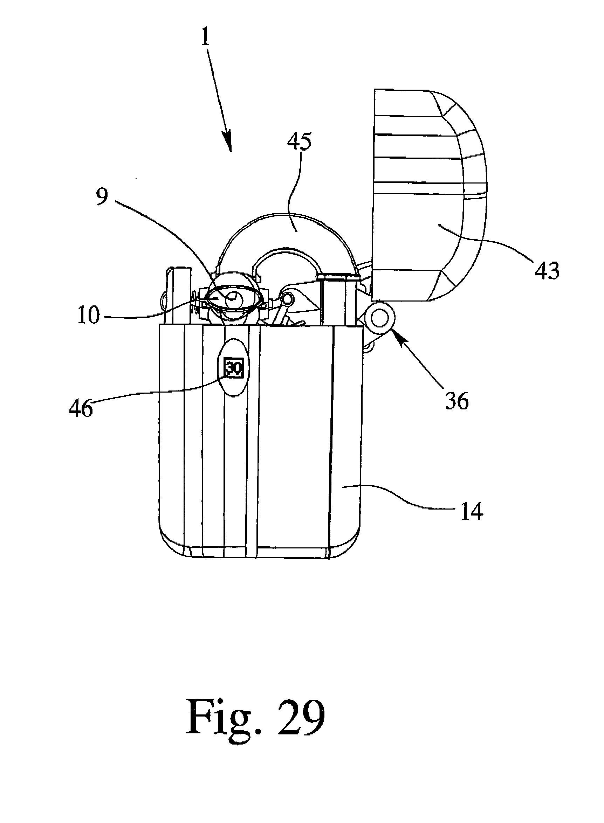

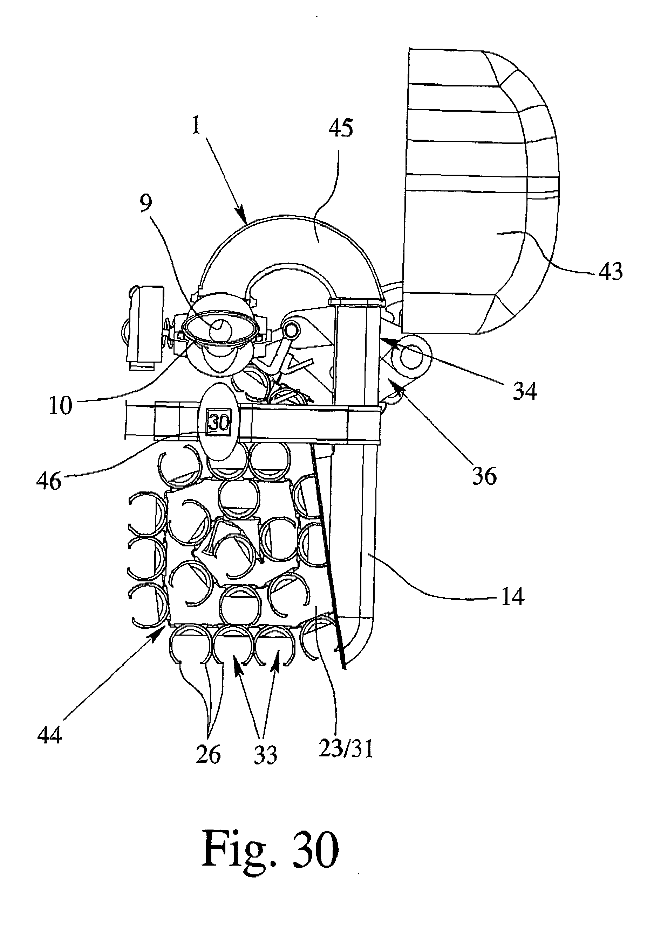

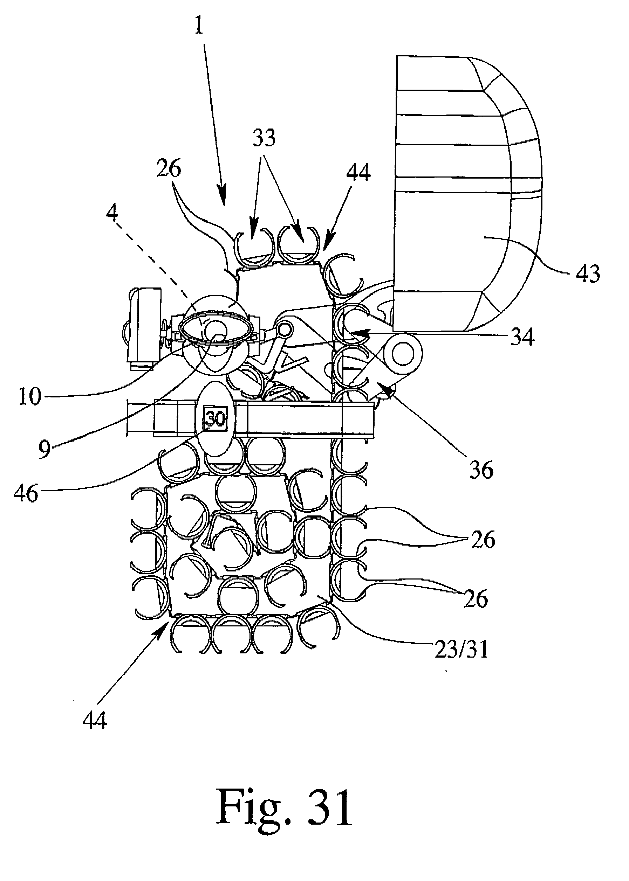

Fig. 29 zeigt in eine weitere Ausführungsform des vorschlagsgemäßen Inhalators 1 mit geöffneter Abdeckkappe 43 und ausgeklapptem Mundstück 10. Fig. 30 zeigt eine vergrößerte, teilschnittartige Ansicht, Fig. 31 zeigt eine andere vergrößerte, teilschnittartige Ansicht.

Die nicht dargestellten Kapseln 3 sind vorzugsweise in einer Kette oder einem Band 44 aufgenommen oder bilden diese bzw. dieses, die bzw. das in einem ersten Aufnahmeraum 23 bzw. Kapselreservoir 31 vorzugsweise schneckenartig aufgenommen ist. Das Band 44 weist Aufnahmen 33 für die einzelnen Kapseln 3 auf, die vorzugsweise von Kapselkammersegmenten bzw. -teilen 26 gebildet sind bzw. diese bilden.

Das Band 44 mit den Kapseln 3 ist in den Bereich des Mundstücks 10 insbesondere über einen Kanal 45 geführt. Dort ist die im einzelnen nicht dargestellte Kapselkammer 4 angeordnet. Die Förderung erfolgt mittels der Fördereinrichtung 34, die vorzugsweise durch Öffnen und Schließen der Abdeckkappe 43 betätigt wird. Beispielsweise ist der nachfolgende Ablauf möglich.

Ausgehend vom geschlossenen Zustand wird die Abdeckkappe 43 aufgeklappt. Zunächst muß noch keine Aktion erfolgen. Ab einem bestimmten Schwenkwinkel erfolgen eine Weiterförderung des Bandes 44 bzw. der Kapseln 3 und vorzugsweise ein Öffnen, insbesondere Anstechen, der nächsten Kapsel 3 mittels der hier nicht dargestellten Öffnungseinrichtung 5. Das Öffnen der Kapseln 3 erfolgt vorzugsweise also selbsttätig. Der Antrieb bzw. die Betätigung erfolgt insbesondere über das hier vorzugsweise als Hebelmechanik ausgebildete, der Abdeckkappe 43 zugeordnete Getriebe 36.

Die nächste Aufnahme 33 wird im weiteren Verlauf der Förderung mit der geöffneten Kapsel 3 zu der in Fig. 31 angedeuteten, sich insbesondere quer zur Zeichenebene und/oder Förderrichtung erstreckenden Kapselkammer 4 gefördert, so daß die Aufnahme 44 mit ihrem Kapselkammersegment bzw. -teil 26 die Kapselkammer 4 schließt. Hierbei kann das beispielsweise aus einem elastischen oder relativ starren Kunststoffmaterial bestehende Kapselkammerteil 26 elastisch oder inelastisch verformt werden. Dies gestattet beispielsweise eine Aufweitung, um die gewünschte Kapselkammergröße zu erreichen.

Alternativ oder zusätzlich kann dies einem vollständigen Schließen der Kapsekammer 4 bzw. einer guten Abdichtung der Kapselkammer 4 zuträglich sein.

Nachdem die Abdeckkappe 43 ausreichend weit geöffnet ist, beispielsweise mehr als 90°, insbesondere mehr als 130°, und dementsprechend die nächste Kapsel 3 geöffnet und in die Kapselkammer 4 gefördert und die Kapselkammer 4 geschlossen wurde, kann das Mundstück 10 ausgeklappt werden und die Inhalation erfolgen. Dieser Zustand ist in Fig. 30 dargestellt.

Nach der Inhalation wird das Mundstück 10 wieder eingeklappt und die Abdeckkappe 43 wieder geschlossen. Das Schließen der Abdeckkappe 43 bewirkt ein Abführen der gebrauchten bzw. entleerten Kapsel 3 in den Aufnahmeraum 23 bzw. das Kapselreservoir 31. Gemäß einem besonders bevorzugten, auch unabhängig realisierbaren Aspekt wird die gebrauchte Kapsel 3 hierbei komprimiert und/oder zerschnitten oder in sonstiger Weise zerkleinert, um den erforderlichen Lagerraum im Inhalator 1 und damit auch die mögliche Baugröße des Inhalators 1 zu minimieren. Alternativ oder zusätzlich wird die Aufnahme 33 bzw. das benutzte Kapselband 44 oder dgl. komprimiert, zerschnitten, abgetrennt oder in sonstiger Weise zur Minimierung des erforderlichen Stauraums behandelt.

Wenn der Inhalator 1 zur Aufnahme gebrauchter Kapseln 3, Kapselkammern 4 und/oder Teilen oder Segmenten 26 davon ausgebildet ist, ist vorzugsweise eine Trennung des dafür vorgesehenen zweiten Aufnahmeraums 24 oder dergleichen gegenüber sonstigen Teilen, Abschnitten oder Bereichen des Inhalators 1, insbesondere gegenüber dem ersten Aufnahmeraum 23, vorgesehen und vorzugsweise durch die Trennwand 42 gebildet, die insbesondere auch durch eine Folie gebildet sein kann. Alternativ oder zusätzlich kann eine entsprechende Trennung auch noch nicht entleerten Kapseln 3 bzw. noch nicht gebrauchten Kapselkammern 4 bzw. dem ersten Aufnahmeraum 23 zugeordnet sein und insbesondere einem Trockenschutz dienen.

Falls mehrere Kapselkammern 4 vorgesehen sind, können auch diese nach ihrer jeweiligen Verwendung komprimiert und/oder zerschnitten, aufgetrennt oder in sonstiger Weise behandelt werden, um möglichst platzsparend im Inhalator 1 aufgenommen bzw. gespeichert zu werden.

Der vorschlagsgemäße Inhalator 1 ist vorzugsweise mit einer Zähleinrichtung 46 versehen, die beispielsweise die bereits benutzten Kapseln 3 oder die noch unbenutzten Kapseln 3 zählen und anzeigen kann. Die Zähleinrichtung 46 kann beispielsweise mit dem Getriebe 36, der Abdeckkappe 43, der Fördereinrichtung 34, dem Band 44 oder dem Mundstück 10 und/oder einem nicht dargestellten Sensor zur Detektion einer Inhalation gekoppelt und ggf. davon angetrieben bzw. betätigt werden.

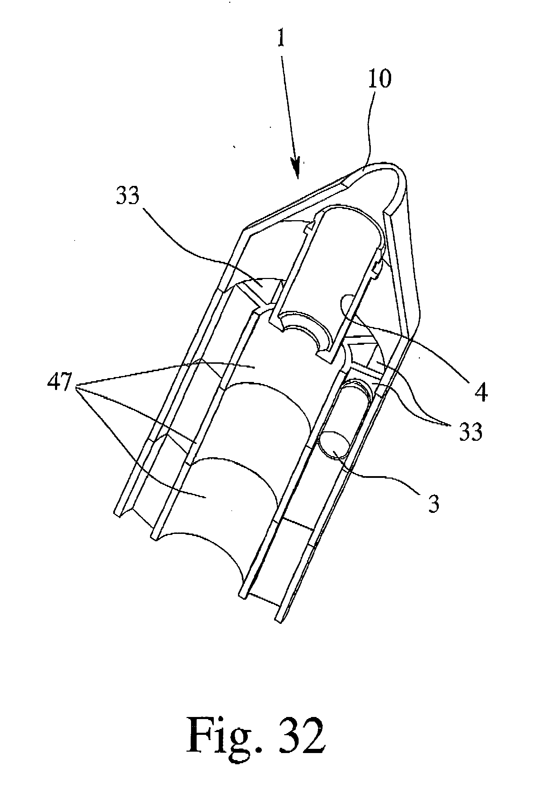

Fig. 32 zeigt in einer schematischen, schnittartigen Darstellung eine weitere Ausführungsform des vorschlagsgemäßen Inhalators 1 mit drehbaren Ringen oder Segmenten 47. Bei Drehen der Ringe oder Segmente 47 werden darin - beispielsweise in segmentartigen Räumen oder Aufnahmen 33 - befindliche Kapseln 3 (in Fig. 32 ist beispielhaft nur eine Kapsel 3 dargestellt) nacheinander einer zentralen Kapselkammer 4 zugeführt und dabei angestochen. Nach der Inhalation werden die benutzten Kapseln 3 wieder in den Ringen oder Segmenten 47 bzw. Aufnahmen 33 aufgenommen oder ausgegeben.

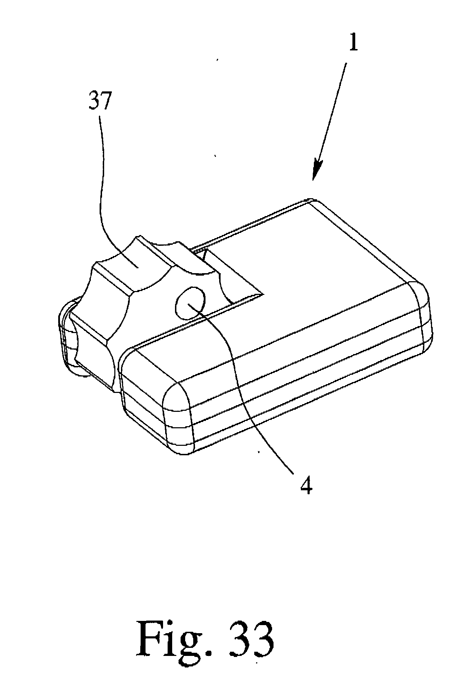

Fig. 33 zeigt ein Schema des Inhalators 1 gemäß einer weiteren Ausführungform. Der Inhalator weist hier ein vorzugsweise manuell drehbares Stellrad 37 mit der Kapselkammer 4 auf Vorzugsweise ist die Kapselkammer 4 manuell mit einer nicht dargestellten Kapsel 3 befüllbar und anschließend zum Öffnen der Kapsel 3 und zur Inhalation in das Inhalatorgehäuse drehbar.

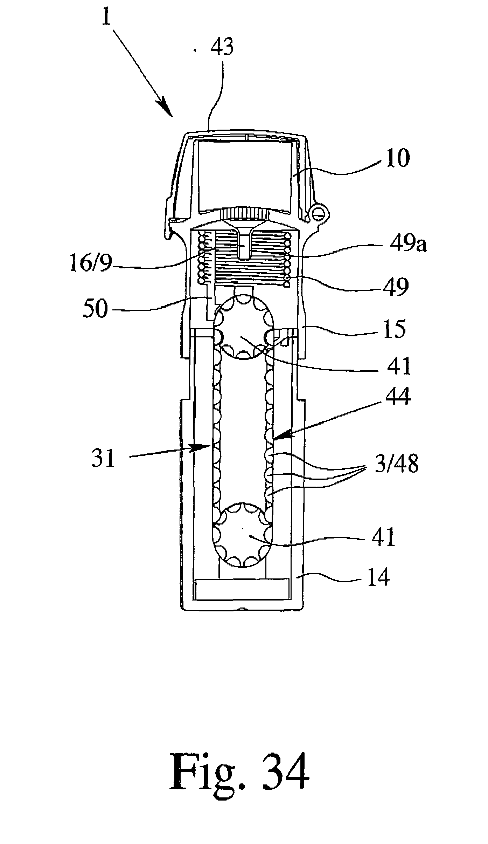

Fig. 34 zeigt in einem schematischen Schnitt eine weitere Ausführungsform des vorschlagsgemäßen Inhalators 1 mit einem endlosen Band 44, das um Antriebsräder 41 umläuft. Das Band 44 kann Aufnahmen 33 für nicht dargestellte Kapseln 3 aufweisen. Vorzugsweise sind die Kapseln 3 im Sinne der vorliegenden Erfindung hier durch Blistertaschen 48 oder sonstige Aufnahmen für die Formulierung 2 gebildet. Die Kapseln 3 sind in diesem Fall insbesondere unlösbar miteinander verbunden,

Durch axiales Zusammenschieben des Gehäuseunterteils 14 und des Gehäuseoberteils 15 des Inhalators 1 - insbesondere gegen die Kraft einer Feder 49 - wird das Band 44 um einen Schritt bzw. eine Position oder Blistertasche 48 weiterbewegt, um die nächste Kapsel 3 bzw. Blistertasche 48 in die Inhalierposition unterhalb des Mundstücks 10 zu bewegen, so daß diese Kapsel 3 bzw. Blistertasche 48 bei der nächsten Inhalation durch Austrag der darin enthaltenen Formulierung 2 entleerbar ist. Das Öffnen, insbesondere Anstechen, erfolgt vorzugsweise durch die nicht dargestellte Öffnungseinrichtung 5, insbesondere indem ein angedeuteter Stoßdorn 49a o.dgl. in die Blistertasche 48 kurz vor Erreichen der zusammen geschobenen Position eintaucht.

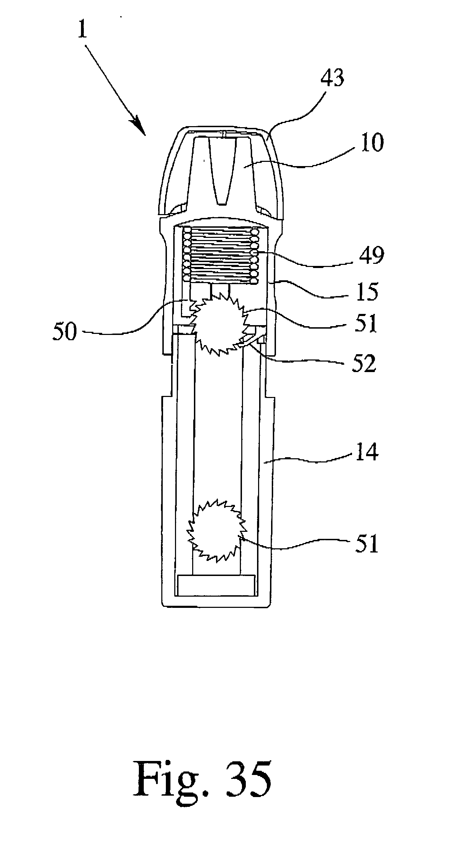

Fig. 35 zeigt in einem anderen Schnitt eine mögliche Realisierung des Antriebs. Ein Eingriffshaken 50 ist beim Zusammenschieben und Auseinanderbewegen der Gehäuseteile 14 und 15 tangential an einem Zahnkranz 51 eines Antriebsrads 41 hin- und herbewegbar, so daß das Antriebsrad 41 schrittweise in eine Richtung gedreht wird, da eine vorzugsweise ebenfalls in den Zahnkranz 51 eingreifende Sperrklinke 52 ein Rückdrehen verhindert.

Im Transport- bzw. Auslieferungszustand ist der Inhalator vorzugsweise zusammengeschobenen. Insbesondere sind hierzu die Gehäuseteile 14 und 15 über mindestens einen nicht dargestellten Schapphaken o.dgl. im zusammen geschobenen Zustand vorzugsweise rastend miteinander verbunden. Durch manuelle Betätigung ist der Eingriff lösbar. Die Feder 49 bewirkt dann das insbesondere in Längsrichtung erfolgende Abrücken der beiden Gehäuseteile 14 und 15 voneinander, so daß der in Fig. 34 und 35 gezeigte Zustand wieder eingenommen wird.

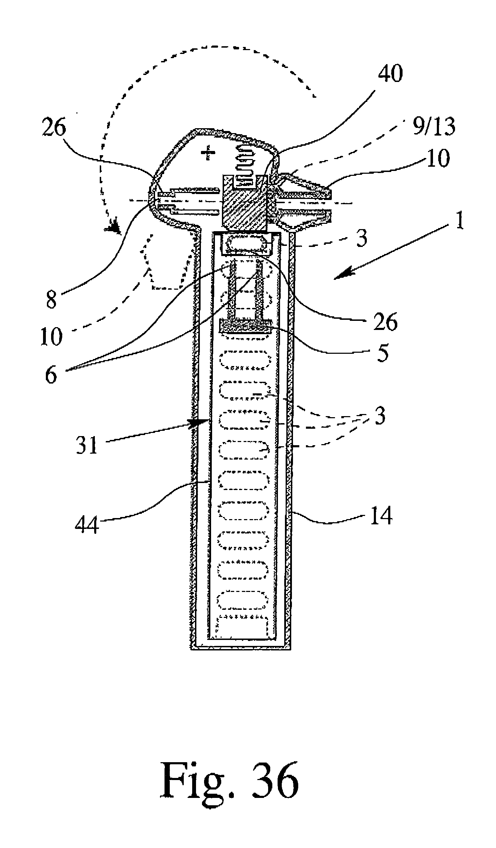

Fig. 36 zeigt in einem schematischen Schnitt einen zu der vorherigen Ausführungsform ähnlichen Inhalator 1. Beim Darstellungsbeispiel sind die Kapseln 3 vom Band 44 bzw. in Aufnahmen 33 oder Blistertaschen 48 aufgenommen und insbesondere einzelweise entnehmbar.

Im Gegensatz zur vorherigen Ausführungsform mit axialer Ausgaberichtung ist hier eine zu der Längsrichtung des Inhalators 1 bzw. des vorzugsweise länglichen bzw. stabartigen Gehäuseunterteils 14 vorzugsweise quer bzw. radial verlaufende Ausgaberichtung und Ausrichtung des Mundstücks 10 für die Inhalation vorgesehen.

Das Mundstück 10 ist vorzugsweise aus der dargestellten Inhalationsposition in die gestrichelte Lage weg- bzw. abklappbar. Diese Klappbewegung wird vorzugsweise zur Betätigung bzw. zum Antrieb des Inhaltors 1 bzw. der Öffnungseinrichtung 5, der Fördereinrichtung 34, eines Antriebsrads 41 oder zum Weiterbebewegen des Bands 44 - beispeilsweise über das nicht dargestellte Getriebe 16 - verwendet.

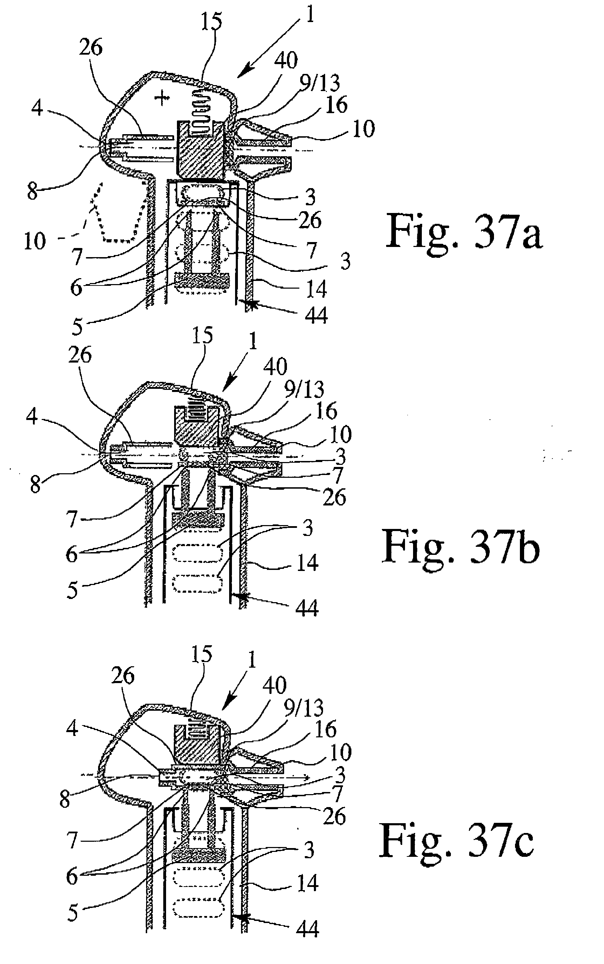

Fig. 37a bis 37c zeigen in schematischen, ausschnittsweisen Schnitten verschiedene Zustände des bevorzugten Funktionsablaufs.

Fig. 37a zeigt einen ersten Zustand. Noch befindet sich keine Kapsel 3 in der Inhalationsposition bzw. in der Kapselkammer 4. Ein erstes Kapselkammerteil 26 ist entgegen der Ausgaberichtung bzw. Hauptströmungsrichtung von der Kapselkammer 4 abgerückt. Die Kapselkammer 4 ist seitlich geöffnet.

Fig. 37b zeigt einen zweiten Zustand. Die Öffnungseinrichtung 5 hat die für die nächste Inhalation vorgesehene Kapsel 3 zusammen mit einem zweiten, insbesondere balkenartigen Kapselkammerteil 26 aus der Blistertasche 48 in die Kapselkammer 4 bzw. in die Verlängerung des sich in der Inhalationsposition befindenden Mundstücks 10 gegen die Kraft des vorzugsweise federbelasteten Rückholelements 40 bewegt bzw. verschoben und geöffnet, insbesondere seitlich mit den Anstechelementen 6 angestochen.

Fig. 37c zeigt einen dritten Zustand. Die Öffnungseinrichtung 5 ist mit ihren Anstechelementen 6 aus der Kapsel 3 zurückgezogen, insbesondere nur soweit, daß in dem zweiten Kapselkammerteil 26 gebildete Anstechöffnungen 7 von den Anstechelementen 6 verschlossen bleiben, um die gewünschte Luftführung (längs durch die Kapselkammer 4) sicherzustellen. Weiter ist das erste Kapselkammerteil 26 vorgeschoben. Die Kapselkammer 4 ist damit geschlossen. Der Inhalator 1 ist zur Inhalation bereit.

Wenn die Öffnungseinrichtung 5 wieder ganz in ihre Ausgangslage zurückbewegt wird und die Kapselkammer 4 geöffnet wird, fördert das vorzugsweise federbelastete Rückholelement 40 die entleerte Kapsel 3 wieder in die leere Blistertasche 48 bzw. Aufnahme 33 im Band 44 zurück. Anschließend kann das Band 44 zur nächsten Kapsel 3 weiterbewegt werden.

Beim Darstellungsbeispiel ist in jeder Aufnahme 33 bzw. Blistertasche 48 ein zweites Kapselkammerteil 26 vorgesehen. Alternativ kann auch nur eine einziges, dann vorzugsweise nicht von den Anstechelementen 6 lösbares zweites Kapselkammerteil 26 vorgesehen sein.

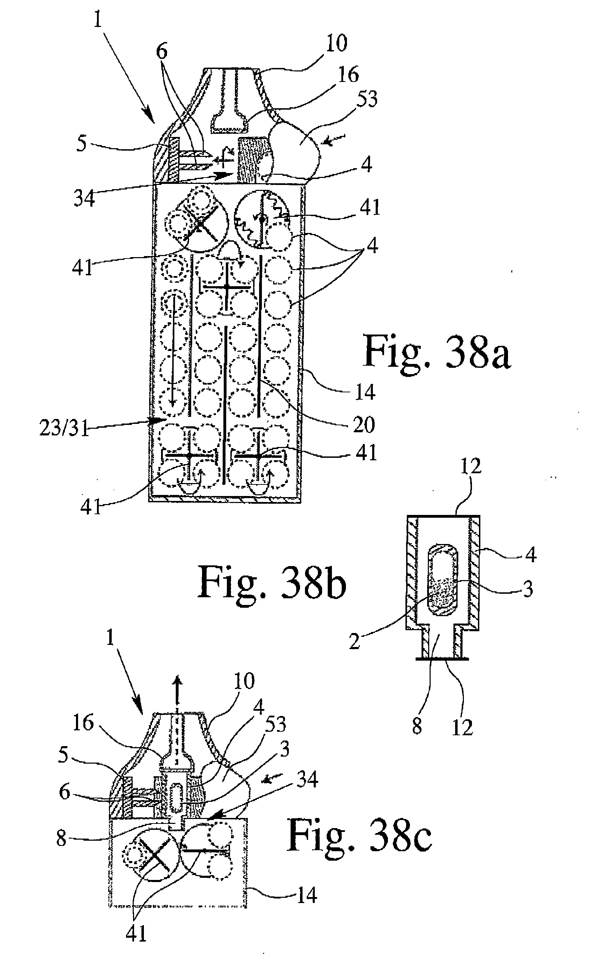

Fig. 38a zeigt eine weitere Ausführungsform des Inhalators 1 mit einem Aufnahmeraum 23 oder Kapselreservoir 31 in einem schematischen Schnitt. Jede Kapsel 3 ist hier in eine Kapselkammer 4 vorkonfektioniert, wie im Schnitt in Fig. 38b gezeigt.

Die Kapselkammern 4 mit den Kapseln 3 sind insbesondere mäanderförmig oder in sonstiger Weise in dem Inhalator 1 bzw. Aufnahmeraum 23 oder Reservoir 31 aufgenommen und vorzugsweise mittels mindestens einem, insbesondere vier Transport- oder Antriebsrädern 41 einzelweise zum Mundstück 10 förderbar.

Die für die nächste Inhalation vorgesehene Kapselkammer 4 wird vorzugsweise mittels der Fördereinrichtung 34, insbesondere einem manuell betätigbaren, hier eindrückbaren Betätigungselement 53, aufgenommen, in die gewünschte Ausrichtung axial zum Mundstück 10 gedreht, ein- und auslaßseitig geöffnet, gegen die Öffnungseinrichtung 5 zum Öffnen bzw. Anstechen der Kapsel 3 bewegt und/oder schließlich in an ein dem Mundstück 10 zugeordnetes Anschlußstück 16 auslaßseitig angeschlossen, wie in dem vergrößerten Ausschnitt in Fig. 38c dargestellt. dann die Ausgabe der Formulierung 2 bzw. die Inhalation erfolgen.

Nach der Inhalation wird die benutzte Kapselkammer 4 mit der entleerten Kapsel 3 wieder von der Fördereinrichtung bzw. dem Aufnahmeraum 23 oder Reservoir 31 aufgenommen, insbesondere mittels des nächsten Transport- oder Antriebsrads 41.

Fig. 39 zeigt eine weitere Ausführungsform mit einem teilschnittartig dargestellten Kapselreservoir 31. Das Kapselreservoir 31 ist rohrförmig ausgebildet. Die Kapseln 3 sind vorzugsweise axial hintereinander und über den Umfang verteilt vom Kapselreservoir 31 aufgenommen.

Das Kapselreservoir 31 ist insbesondere zur gruppenweise Ausgabe von Kapseln 3, beispeilsweise jeweils sechs Kapseln 3, zur Aufnahme in einem entsprechend mit beispielsweise sechs Kapselkammern 4 versehenen Träger 11 ausgebildet. Es weist eine vorzugsweise blendenartige Schleuse oder Ausgabe 54 mit Durchbrechungen 55 entsprechend der Anzahl an gleichzeitig auszugebenden Kapseln 3 auf. Die Schleuse ist vorzugsweise gegen die Kraft einer Feder 56 derart verdrehbar, daß die Durchbrechungen 55 zu den Kapseln 3 in Verlängerung bzw. axial ausrichtbar sind, so daß die Kapseln 3 durch die Durchbrechungen 55 hindurch ausgegeben und von dem darunter angeordneten Magazin bzw. Träger 11 aufgenommen werden. Nach der Ausgabe schließt die Schleuse 54 durch die Federkraft bzw. Rückdrehen wieder selbsttätig.

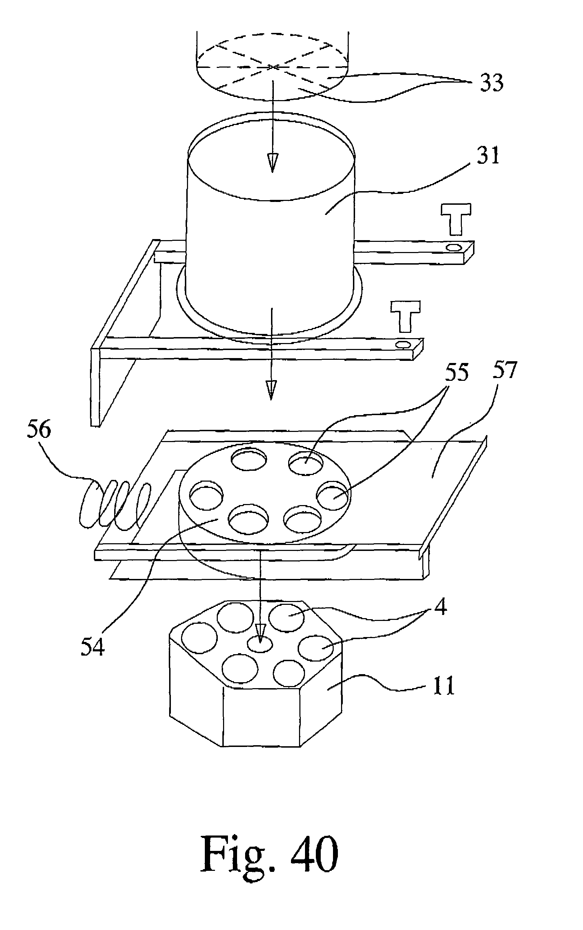

Fig. 40 bis 42 zeigen ähnliche Ausführungsformen des Kapselreservoirs 31.

Bei der Ausführungsform gemäß Fig. 40 ist die Schleuse 54 von einer zugeordneten schieberartigen Blende 57 verschließbar, die zur Kapselausgabe, also zum Öffnen der Schleuse 54 bzw. Freigeben der Durchbrechungen 55, quer bzw. radial gegen die Kraft der Feder 56 verschiebbar ist. In Fig. 40 sind aus Vereinfachungsgründen keine Kapseln 3 dargestellt.

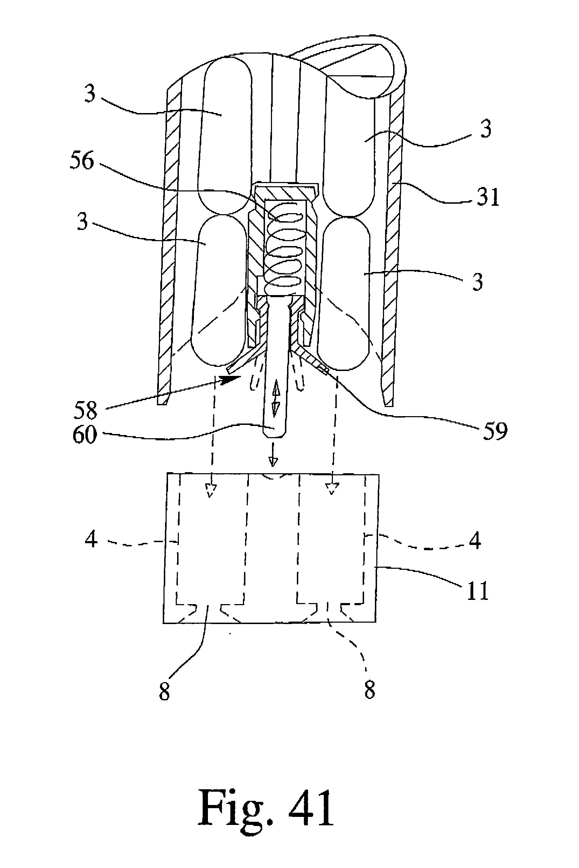

Bei der Ausführungsform gemäß Fig. 41 weist das Kapselreservoir 31 eine vorzugsweise zentrale Halteeinrichtung 58 auf, die die Kapseln 3 mittels sich im gesperrten Zustand radial spreizender Arme 59 gegen eine axiale Bewegung blockiert. Wenn der Träger 11 axial auf einen Stift 60 der Halteeinrichtung 58 drückt, gibt diese die Kapseln 3 frei, indem die Arme 59 radial an den Stift 60 geklappt werden, wie in Fig. 41 gestrichelt dargestellt. Die nächste Gruppe von Kapseln 3 kann dann in den darunter befindlichen Träger 11 bzw. dessen Kapselkammern 4 rutschen. Wenn der Träger entfernt wird, erfolgt eine Rückstellung durch die Feder 56, spreizen sich die Arme 59 und sperrt die Halteeinrichtung 58 eine weitere Ausgabe der Kapseln 3.

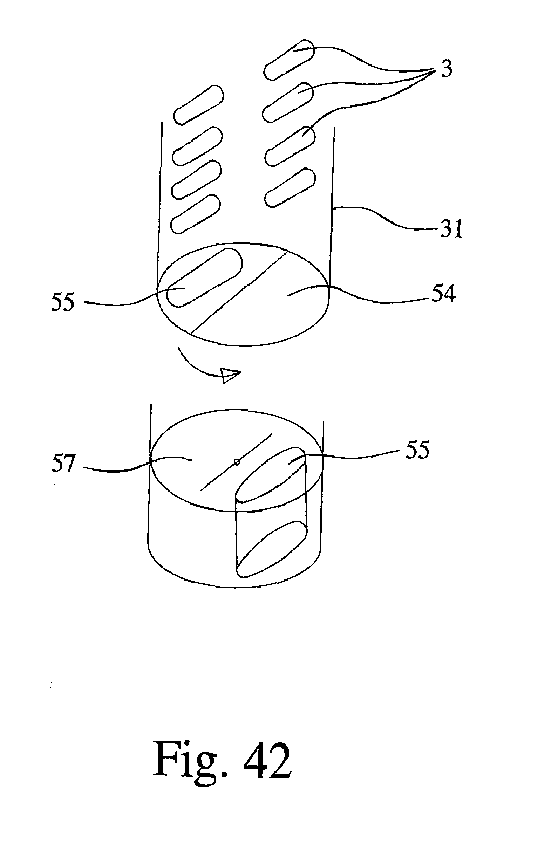

Bei der Ausführungsform gemäß Fig. 42 nimmt das Kapselreservoir 31 die Kapseln 3 vorzugsweise mit einer Ausrichtung quer zur Längserstreckung des Kapselreservoirs 31 auf Die Kapseln 3 sind entweder in zwei Reihen oder Stapeln in axialer Richtung des Kapselreservoirs 31 oder entlang einer Wendel bzw. Schraubenlinie im Kapselreservoir 31 angeordnet. Die Schleuse 54 weist insbesondere nur eine Durchbrechnung 55 auf. Bei Verdrehen oder schrittweisem Drehen der Blende 57, so daß deren Durchbrechung 55 mit der Durchbrechung 55 der Schleuse 54 in Deckung gebracht wird, werden die Kapseln 3 vorzugsweise nur einzelweise ausgegeben. Insbesondere ist jeweils eine Kapsel 3 in einer bestimmten Drehlage der Blende 57 ausgebbar. In der entgegengesetzten bzw. demgegenüber verdrehten, in Fig. 42 gezeigten und insbesondere um 180° verdrehten Drehlage ist das Kapselreservoir 31 vorzugsweise dicht verschlossen, insbesondere um die Kapseln 3 vor übermäßigen Klimaschwankungen zu schützen und/oder ein übermäßiges Austrocknen der Kapseln 3 zu verhindern.

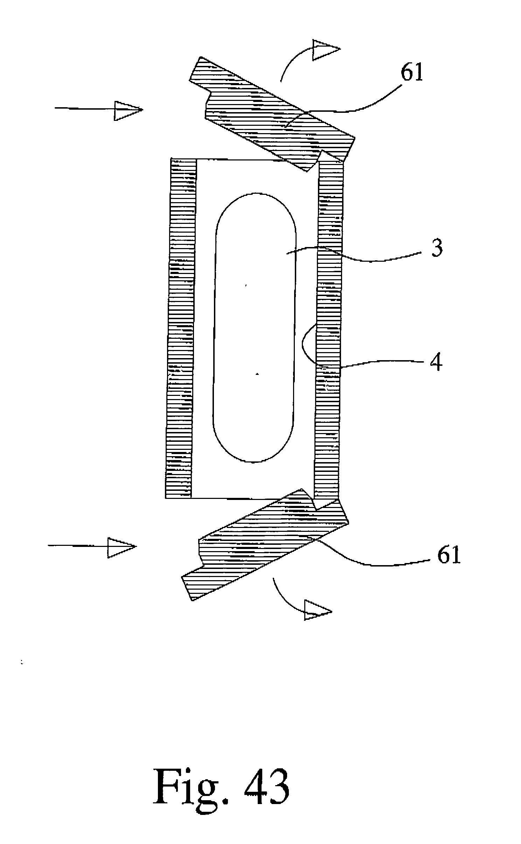

Fig. 43 zeigt einen Schnitt einer Ausführungsform, bei der die Kapselkammer 4 durch entfernbare Stopfen 61 oder sonstige Verschlußelemente, wie Kappen, Deckel o.dgl., ein- und/oder auslaßseitig verschließbar bzw. verschlossen ist.

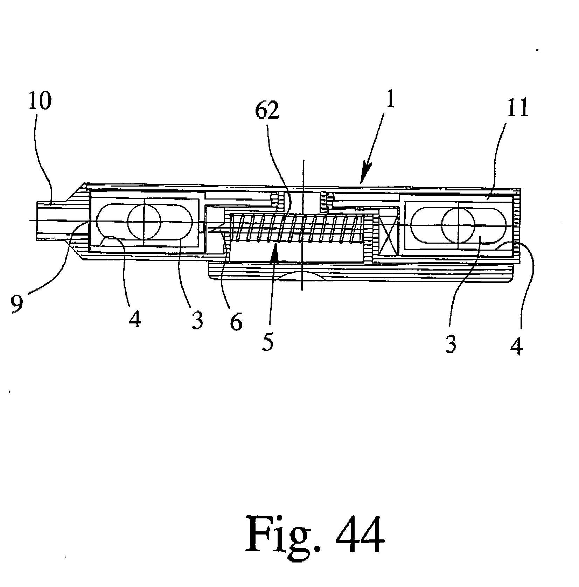

Fig. 44 zeigt in einem schematischen Schnitt eine weitere Ausführungsform des Inahalators 1. Die Kapselkammern 4 und Kapseln 3 sind in dem drehbaren, ringförmigen Träger 11 radial ausgerichtet. Das Anstechen der Kapseln 3 durch die Öffnungseinrichtung 5 in der Inhalationsposition erfolgt ebenfalls radial, also axial bzw. strinseitig bezüglich der Kapseln 3. Die Öffnungseinrichtung 5 weist hier eine vorzugsweise das Anstechelement 6 umgebende Rückstellfeder 62 auf und ist insbesondere schieberartig gegen die Kraft der Rückstellfeder 62 betätigbar.

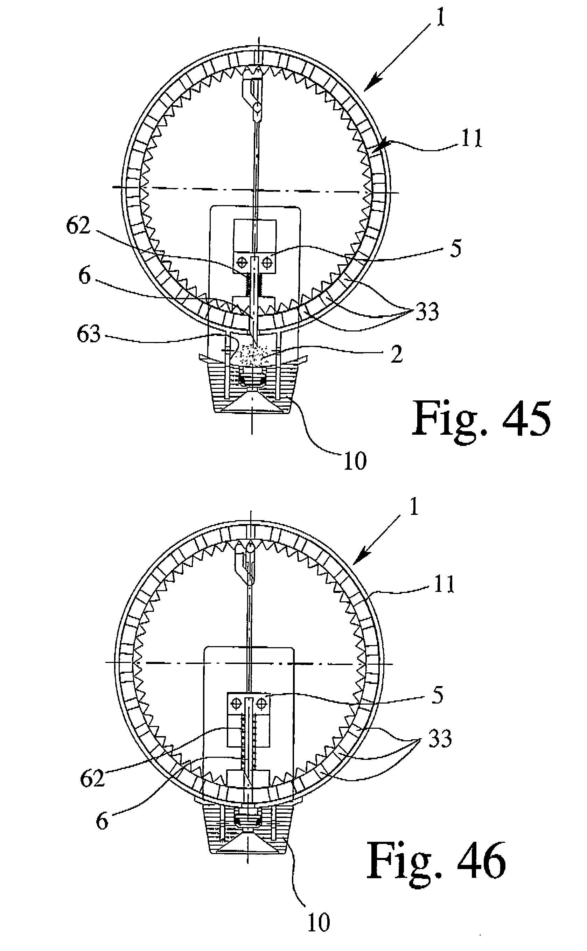

Fig. 45 und 46 zeigen schematisch eine weitere Ausführungsform des Inahalators 1. Anstelle von separaten Kapseln 3 ist hier die Formulierung 2 direkt in Aufnahmen 33 im Träger 11 vordosiert. Hier bilden also die Aufnahmen 33 vorzugsweise Kapseln im Sinne der vorliegenden Erfindung. Der Träger 11 bildet vorzugsweise einen Ring, wobei die Aufnahmen 33 über den Umfang verteilt und verschlossen sind.

Die Aufnahmen 33 sind einzelweise mittels der wieder vorzugsweise radial wirkenden Öffnungseinrichtung 5 durch radiales Herausziehen des Mundstücks 10 öffenbar. Hierdurch wird die mit dem Mundstück 10 insbesondere über einen Schlitten gekoppelte Öffnungseinrichtung 5 ebenfalls radial verschoben, so daß das Anstechelement 6 in die sich in der Inhalationsposition befindende bzw. zum Anstechelement 6 ausgerichtete Aufnahme 33 eingreift und die darin befindliche Formulierung 2 radial nach außen in die sich anschließende Kammer 63 austrägt bzw. ausschiebt. Dieser Zustand ist in Fig. 45 dargestellt.

Anschließend wird bei der Inhalation die Formulierung 2 aus der Kammer 63 über das Mundstück 10 durch den Luftstrom ausgetragen. Danach kann das Mundstück 10 wieder radial zurückgeschoben werden. Dieser Zustand ist in Fig. 46 gezeigt. Weiter wird dadurch das Anstechelement 6 aus der zuvor angestochenen bzw. entleerten Aufnahme 33 wieder zurückgezogen.

Schließlich wird der Träger 11 um eine Aufnahme 33 weiterbewegt bzw. weitergedreht. Dies kann beispielsweise über die gezeigte Innenverzahnung beim vollständigen Einschieben und/oder anfänglichen Herausziehen des Mundstücks 10 erfolgen.

Generell wird insbesondere ein Inhalator 1 zur Inhalation einer Formulierung 2 aus Kapseln 3 vorgeschlagen, die jeweils eine Dosis der Formulierung 2 enthalten. Die Kapseln 3 werden vorzugsweise jeweils in einer Kapselkammer 4 dadurch entleert, daß sie von einem durch die Kapselkammer 4 strömenden Gas- bzw. Luftstrom in Bewegung versetzt werden. Der Gas- bzw. Luftstrom kann durch das Einatmen eines Benutzers bzw. Patienten und/oder aktiv durch den Inhalator 1 erzeugt werden. Zur einfachen Handhabung weist der Inhalator 1 insbesondere eine Einrichtung zur insbesondere selbsttätigen Befüllung, Entleerung und/oder Reinigung der Kapselkammer 4 auf, wenn diese mehrfach verwendet wird. Alternativ weist der Inhalator 1 eine Vielzahl von Kapselkammem 4 auf, die jeweils vorzugsweise bereits eine Kapsel 3 enthalten und vorzugsweise nur einmal verwendet werden.

Das vorzugsweise vorgesehene Dispergierverfahren (Bewegung der geöffneten Kapsel 3 im Gas- bzw. Luftstrom zur Ausgabe und Dispersion der Formulierung 2 im Gas- bzw. Luftstrom) hat verschiedene Vorteile. Es wird eine sehr gleichmäßige Dispersion mit guter Deagglomaration der Partikel ermöglicht. Es wird eine relative Flußratenunabhängigkeit ermöglicht. Insbesondere beginnt die Kapselbewegung bzw. -vibration und damit die Ausgabe der Formulierung erst ab etwa einer Flußrate von 10 oder 20 l/min. Weiter kann ein für den Benutzer wahrnehmbares akustisches Signal durch die Kapselbewegung - also beim Inhalieren - erzeugt werden.

Weiter kann der Inhalator 1 mit einer Vorrichtung zur Reinigung des Mundstücks 10 versehen sein. Alternativ oder zusätzlich kann das Mundstück 10 insbesondere zu Reinigungszwecken demontierbar sein.

Bei mehreren Kapselkammern 4 kann der Inhalator 1 eine nicht dargestellte Halte- oder Klemmvorrichtung aufweisen, um die Kapselkammern 4 in der Inhalationsposition - kraft- und/oder formschlüssig - fixieren zu können. Die Halte- oder Klemmvorrichtung kann beispielsweise längsseitig an der Kapselkammer 4 angreifen, so daß der Einlaß 8 und Auslaß 9 für die Luftzuführung bzw. Luftabführung erhalten bleibt. Alternativ oder zusätzlich kann die Kapselkammer 4 auch an ihren axialen Endbereichen - insbesondere am Einlaß 8 und Auslaß 9 - vorzugsweise klemmend, kraft- und/oder formschlüssig gehalten werden.

Die beim Darstellungsbeispiel gezeigten Kapseln 3 sind vorzugsweise zylindrisch bzw. länglich, insbesondere mit abgerundeten Enden ausgebildet. Grundsätzlich können die Kapseln 3 aber jede beliebige Form aufweisen bzw. annehmen.

Bedarfsweise kann das Kapselreservoir 31 auch drehbar ausgebildet sein, insbesondere wenn es diskrete Aufnahmekammern für die Kapseln 3 oder entsprechende Mitnehmer für die Kapseln 3 aufweist. Die Kapseln 3 können dann entsprechend in eine Ausgabeposition überführt werden, aus der sie insbesondere einzelweise in die zugeordnete Kapselkammer 4 oder in eine sonstige Handhabungs- bzw. Fördereinrichtung ausgegeben werden können.

Bei den beschriebenen Ausführungsformen und -varianten erfolgt vorzugsweise ein automatisiertes Öffnen der Kapselkammern 4, des Trägers 11, des Kapselreservoirs 31 oder dgl. Zusätzlich oder alternativ ist es auch möglich, daß eine Abdeckung, Versiegelung oder dgl. manuell - insbesondere bei erstmaliger Benutzung des Inhalators 1 - geöffnet werden muß, beispielsweise durch seitliches Herausziehen.

Gemäß einer weiteren, nicht dargestellten Ausführungsvariante kann das Reservoir 31 für die Kapseln 3 oder Kapselkammern 4 auch derart ausgebildet sein, daß es an den Inhalator 1 zum Nachfüllen des Inhalators 1 ansetzbar bzw. ankoppelbar ist. Insbesondere erfolgt dann ein automatisiertes Öffnen des Reservoirs 31 zur Übergabe der Kapseln 3 bzw. Kapselkammern 4 an den Inhalator 1.

Zur Betätigung des Inhalators 1 ist auch möglich, daß das Mundstück 10 vorzugsweise axial bzw. in Ausgangrichtung, insbesondere manuell bewegbar ist. Dies kann zum Befüllen bzw. Entleeren der zugeordneten Kapselkammer 4, zum insbesondere axialen bzw. in Längsrichtung erfolgenden Öffnen oder Anstechen der Kapsel 3 und/oder zum Öffnen/Schließen eines vorzugsweise im Mundstück 10 angeordneten Auslaßventils o.dgl. verwendet werden.

Zum Fördern bzw. Weitertransport der Kapseln 3 und/oder Kapselkammern 4 kann generell ein beliebiger Antrieb eingesetzt werden. Besonders bevorzugt erfolgt ein rein mechanischer, insbesondere manuell betätigter Antrieb. Bedarfsweise sind Federmittel einsetzbar. So ist es beispielsweise möglich, eine vorgespannte Feder, wie eine Spiralfeder, Uhrenfeder oder dgl., zum Weiterfördern des Trägers 11 oder zum Fördern und/oder Aufwickeln der insbesondere ein Band oder eine Kette bildenden Kapseln 3, Kapselkammern 4 oder Führungselemente 21 einzusetzen.

Der vorschlagsgemäße Inhalator 1 ist vorzugsweise mit einer Sperr- oder Sicherungseinrichtung versehen, um eine unbefugte Benutzung verhindern zu können, insbesondere eine Kindersicherung oder dgl. zu realisieren.

Weiter kann der Inhalator 1 auch einen Sperrmechanismus, wie ein Ratschenmechanismus oder dgl., aufweisen, um eine entgegengesetzte Förderung der Kapseln 3, der Kapselkammern 4, des Trägers 11, der Führungselemente 21 oder einer davon gebildeten Kette oder eines davon gebildeten Bandes oder dgl. zu verhindern.

Der vorschlagsgemäße Inhalator 1 ist insbesondere zur Aufnahme von mindestens 30 Kapseln oder mehr ausgebildet. Bei täglicher Inhalation einer Dosis kann der Inhalator dann einen Monatsbedarf abdecken.

Einzelne Merkmale und Aspekte der verschiedenen Ausfühnmgsformen können auch beliebig miteinander kombiniert oder bei sonstigen Konstruktionen von Inhalatoren eingesetzt werden.

Die vorliegende Erfindung ist nicht auf Inhalatoren beschränkt, sondern kann entsprechend auch bei sonstigen Zerstäubern eingesetzt werden. Dementsprechend ist der Begriff "Inhalator" vorzugsweise in einem weiteren Sinne auch dahingehend zu verstehen, daß er auch sonstige Spender oder Zerstäuber, insbesondere für medizinische oder sonstige therapeutische Zwecke, umfaßt.

Nachfolgend werden bevorzugte Bestandteile und/oder Zusammensetzungen der vorzugsweise medizinischen Formulierung 2 aufgeführt. Wie bereits erwähnt, handelt es sich insbesondere um Pulver, oder um Flüssigkeiten im weitesten Sinne. Besonders bevorzugt sind in der Formulierung 2 enthalten:

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Besonders bevorzugt sind in diesem Zusammenhang Arzneimittel, die ausgewählt sind aus der Gruppe bestehend aus Anticholinergika, Betamimetika, Steroiden, Phosphodiesterase IV-Inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika. Im Fall von Kombinationen weist vorzugsweise wenigstens einer der Wirkstoffe chemisch gebundenes Wasser auf. Bevorzugt werden Anticholinergika-haltige Wirkstoffe eingesetzt, als Monopräparate oder in Form von Kombinationspräparaten.

Im einzelnen seien als Beispiele für die wirksamen Bestandteile oder deren Salze genannt:

Zur Anwendung gelangende Anticholinergika sind bevorzugt ausgewählt aus der Gruppe bestehend aus Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Metbobromid, 2,2-Diphenylpropionsäurecyclopropyltropinester -Methobromid, 9-Hydroxy-xanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester - Methobromid, 9-Methyl-xanthen-9-carbonsäurescopinester -Methobromid, 9-Ethyl-xanthen-9-carbonsäuretropenolester Methobromid, 9-Difluormethylxanthen-9-carbonsäuretropenolester -Methobromid und 9-Hydroxymethylxanthen-9-carbonsäurescopinester -Methobromid, gegebenenfalls in Form ihrer Racemate, Enantiomere oder Diastereomere und gegebenenfalls in Form ihrer Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacaterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1*H*-quinolin-2-on, 4-hydroxy-7-[2-{[2-{[3-(2-phenylethoxy)propyl]sulphonyl}ethyl]-ammo}ethyl]-2(3H)-benzothiazolon, 1-(2-Fluoro-4-hydroxyphenyl)-2-[4-(1-benzmidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol , 1-[2H-5-hydroxy-3-oxo-4H-1,4-benzoxazm-8-yl]-2-[3-(4-metboxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol , 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-Inhibitoren sind bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a, 10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbansäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]aceta, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2- hydroxy-2-propyl)phenyl)thio)methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methyl-ethyl)phenyl)pro-pyl)thio)methyl)cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenylethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-(R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)ammo]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-pipendin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonylpiperidin-4-yloxy)-7-(2-methoxy-ethoxy)-chinazolin, 4-[(3-Chlor-4-fluorphenyl)amino]-6-[1-(2-methoxy-acetyl)-pipefidin-4-yloxy]-7-(2-methoxyethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopynrolidin-1-yl)ethyl]-piperidin-4-yloxy)-7-methoxy-chinazolin, 4-[(3-Ethinylphenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{ 1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethylamino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methylamino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylaminocyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methylamino}-cyclohexan-1-yloxy)-7-methoxy-chinazolin, 4-[(3-Chlor-4-fluor-phenyl)amino]-6-[2-(2,2-dimetbyl-6-oxo-morpholin-4-yl)-ethoxy]-7-[(*S*)-(tetrahydrofuran-2-yl)tnethoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-eyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydrolnethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Antiallergika: Dinatriumcromoglicat, Nedocromil.

Als Derivate der Mutterkornalkaloide: Dihydroergotamin, Ergotamin.

Für die Inhalation kommen Arzneimittel, Arzneimittelformulierungen und -mischungen mit den o.g. Wirkstoffen in Betracht, sowie deren Salze, Ester sowie die Kombination dieser Wirkstoffe, Salze und Ester.

### Bezugszeichentiste

| | | | |
|---|---|---|---|
| 1 | Inhalator | 27 | Mechanik |
| 2 | Formulierung | 28 | Steuerschieber |
| 3 | Kapsel | 29 | Schwenkeinrichtung |
| 4 | Kapselkammer | 29a | Sonnenrad |
| 5 | Öffnungseinrichtung | 29b | Zahnrad |
| 6 | Anstechelement | 30 | Schwenkachse |
| 6a | zusätzliches Anstechelement | 31 | Kapselreservoir |
| 7 | Anstechöffnung | 32 | Reinigungseinrichtung |
| 8 | Einlaß | 33 | Aufnahme |
| 9 | Auslaß | 34 | Fördereinrichtung |
| 10 | Mundstück | 35 | Steuerhülse |
| 11 | Träger | 36 | Getriebe |
| 12 | Abdeckung | 37 | Stellrad |
| 12a | Abdeckungsstück | 38 | Führung |
| 13 | Gitter | 39 | Stößel |
| 14 | Gehäuseunterteil | 40 | Rückholelement |
| 15 | Gehäuseoberteil | 41 | Antriebsrad |
| 16 | Anschlußstück | 42 | Trennwand |
| 17 | Feder (Anschlußstück) | 43 | Abdeckkappe |
| 18 | Einsatz | 44 | Band |
| 18a | erste Rolle | 45 | Kanal |
| 18b | zweite Rolle | 46 | Zähleinrichtung |
| 18c | Ring | 47 | Ring/Segment |
| 18d | Öffnungselement | 48 | Blistertasche |
| 18e | Auffangbehälter | 49 | Feder (Gehäuse) |
| 18f | Nut | 49a | Stoßdom |
| 18g | Kulisse | 50 | Eingriffshaken |
| 18h | Vorsprung | 51 | Zahnkranz |
| 18i | Steuerkurve | 52 | Sperrklinke |
| 19 | Einsatz | 53 | Betätigungselement |
| 20 | Führung | 54 | Schleuse |
| 20a | Laufbahn | 55 | Durchbrechung |
| 21 | Führungselement | 56 | Feder (Schleuse) |
| 21a | Eingriffselement | 57 | Blende |
| 22 | Verjüngung | 58 | Halteeinrichtung |
| 23 | Erster Aufnahmeraum | 59 | Arm |
| 24 | Zweiter Aufnahmeraum | 60 | Stift |
| 25 | Befülleinrichtung | 61 | Stopfen |
| 25a | Förderelement | 62 | Rückstellfeder |
| 26 | Kapselkammerteil | 63 | Kammer |

## Patentansprüche

1. Inhalator (1) zur Inhalation einer Formulierung (2) aus Kapseln (3), die jeweils eine Dosis der Formulierung (2) enthalten, wobei der Inhalator (1) vorzugsweise nur eine Kapselkammer (4) zur einzelweisen Aufnahme von Kapseln (3) nacheinander zu deren Entleerung bei der Inhalation aufweist,
**dadurch gekennzeichnet,**
**daß** die Kapselkammer (4) zu ihrer Befüllung, Entleerung und/oder Reinigung bewegbar, schwenkbar oder insbesondere längsseitig öffenbar ist und/oder
**daß** der Inhalator (1) eine Einrichtung (25, 32) zur insbesondere selbsttätigen Befüllung, Entleerung und/oder Reinigung der Kapselkammer (4) aufweist.

2. Inhalator nach Anspruch 1, **dadurch gekennzeichnet, daß** der Inhalator eine Öffnungseinrichtung (5) zum insbesondere selbsttätigen oder zwangsweisen Öffnen der jeweiligen Kapsel (3) insbesondere beim Bewegen, beim Schwenken oder beim Transport aus oder von einem Kapselreservoir (31) zu der oder in die Kapselkammer (4) aufweist.

3. Inhalator nach Anspruch 1 oder 2, **dadurch gekennzeichnet, daß** der Inhalator eine Öffnungseinrichtung (5) zum seitlichen, radialen, axialen oder endseitigen Öffnen, insbesondere Anstechen oder Aufschneiden, der jeweiligen, vorzugsweise länglichen Kapsel (3) - vorzugsweise zum Öffnen der Kapsel (3) in der Kapselkammer (4) - aufweist.

4. Inhalator nach Anspruch 3, **dadurch gekennzeichnet, daß** die Kapselkammer (4) mindestens eine Öffnung (7) zum Öffnen, insbesondere Anstechen, der Kapsel (3) aufweist, wobei die Öffnung (7) wiederverschließbar ist, insbesondere mittels eines Anstechelements (6), einer Abdeckung (12), eines Abdeckungsstücks (12a), eines Septums oder eines vorzugsweise federbelasteten oder elastischen Abdeckelements.

5. Inhalator nach einem der voranstehenden Ansprüche, **dadurch gekennzeichnet, daß** die Kapselkammer (4) länglich ausgebildet ist und insbesondere in Längsrichtung von Luft zur Entleerung der jeweiligen Kapsel (3) und/oder zum Austrag der jeweiligen Dosis der Formulierung (2) durchströmbar ist.

6. Inhalator nach einem der voranstehenden Ansprüche, **dadurch gekennzeichnet, daß** die Kapseln (3) jeweils zur Entleerung insbesondere durch einen Luftstrom - insbesondere nach einem Öffnen der jeweiligen Kapsel (3) und/oder bei der Inhalation - in der Kapselkammer (4) bewegbar sind.

7. Inhalator nach einem der voranstehenden Ansprüche, **dadurch gekennzeichnet, daß** die Kapselkammer (4) mehrteilig ausgebildet ist, insbesondere wobei die Kapselkammer (4) aus einem mehrfach verwendbaren Teil und einem einmalig verwendbaren, insbesondere eine Halterung oder Aufnahme (44) einer Kapsel (3) bildenden Teil (26) aufgebaut ist.

8. Inhalator nach einem der voranstehenden Ansprüche, **dadurch gekennzeichnet, daß** die Kapselkammer (4) ein Kapselkammerteil (26) aufweist, das insbesondere einen Zentralabschnitt der Kapselkammer (4) bildet, aus der Kapselkammer (4) herausbewegbar oder -ziehbar ist, und/oder einer Befüllung und Entleerung der Kapselkammer (4) mit den Kapseln (3) dient.

9. Inhalator nach einem der voranstehenden Ansprüche, **dadurch gekennzeichnet, daß** der Inhalator (1) mehrere, vorzugsweise jeweils mehrfach verwendbare Kapselkammern (4) aufweist.

10. Inhalator (1) zur Inhalation einer Formulierung (2) aus Kapseln (3), die jeweils eine Dosis der Formulierung (2) enthalten, wobei der Inhalator (1) mehrere Kapselkammern (4) zur Aufnahme von Kapseln (3) zu deren Entleerung bei der Inhalation aufweist, insbesondere nach einem der voranstehenden Ansprüche,
**dadurch gekennzeichnet,**
**daß** jede Kapselkammer (4) nur einmalig verwendbar ist und/oder
**daß** die Kapselkammern (4) mit den Kapseln (3) vorzugsweise flüssigkeits- oder gasdicht verschlossen, insbesondere versiegelt, und/oder einzelweise öffenbar sind.

11. Inhalator nach Anspruch 10, **dadurch gekennzeichnet, daß** jede Kapselkammer (4) eine Kapsel (3) enthält.

12. Inhalator nach einem der Ansprüche 9 bis 11, **dadurch gekennzeichnet, daß** die Kapselkammern (4) nicht miteinander verbunden sind, insbesondere lose entlang einer Schiene oder Führung (20) geführt oder bewegbar sind.

13. Inhalator nach einem der Ansprüche 9 bis 12, **dadurch gekennzeichnet, daß** die Kapselkammern (4) insbesondere fest, beweglich, flexibel, gelenkig, rastend, klemmend, lösbar oder auftrennbar miteinander und/oder mit einem Träger (11) verbunden sind.

14. Inhalator nach einem der Ansprüche 9 bis 13, **dadurch gekennzeichnet, daß** die Kapselkammern (4) eine Kette oder ein Band (44) bilden.

15. Inhalator nach einem der Ansprüche 9 bis 14, **dadurch gekennzeichnet, daß** die benutzten und/oder unbenutzten Kapselkammern (4) mäanderförmig oder schneckenförmig im Inhalator (1) aufnehmbar oder aufgenommen sind.

16. Inhalator nach einem der Ansprüche 9 bis 15, **dadurch gekennzeichnet, daß** die Kapselkammern (4) einen Ring bilden bzw. ringförmig, insbesondere doppelringförmig, angeordnet sind, insbesondere wobei die Kapselkammern (4) und/oder die Kapseln (3) radial oder axial ausgerichtet sind.

17. Inhalator nach einem der Ansprüche 9 bis 16, **dadurch gekennzeichnet, daß** der Inhalator (1) eine insbesondere revolverartige Schwenkeinrichtung (29) mit mehreren oder allen Kapselkammern (4) aufweist, insbesondere wobei der Inhalator (1) mehrere, vorzugsweise getrieblich gekoppelte Schwenkeinrichtungen (29) aufweist.

18. Inhalator nach einem der Ansprüche 10 bis 17, **dadurch gekennzeichnet, daß** der Inhalator (1) einen vorzugsweise starren und/oder ringförmigen Träger (11) mit den Kapseln (3) und/oder Kapselkammern (4) aufweist.

19. Inhalator nach einem der Ansprüche 10 bis 18, **dadurch gekennzeichnet, daß** die Kapselkammern (4) durch eine vorzugsweise gemeinsame, insbesondere folienartige Abdeckung (12), durch Abdeckungsstücke (12a) oder durch Verschlußelemente, insbesondere Hülsen oder Stopfen (61), zumindest einlaß- und/oder auslaßseitig verschlossen sind.

20. Inhalator nach den Ansprüchen 18 und 19, **dadurch gekennzeichnet, daß** die Abdeckung (12) oder Abdeckungsstücke (12a) am Träger (11) angebracht, insbesondere auflaminiert, ist bzw. sind und/oder Anstechöffnungen (7) der Kapselkammern (4) überdeckt bzw. überdecken.

21. Inhalator nach Anspruch 19 oder 20, **dadurch gekennzeichnet, daß** die Abdeckung (12) oder durch Abdeckungsstücke (12a) zum einzelweisen Öffnen der Kapselkammern (4) abziehbar, abschälbar, ab- bzw. aufwickelbar, durchstoßbar oder auftrennbar ist bzw. sind, insbesondere mittels einer Öffnungseinrichtung (5) oder eines vorzugsweise haken- oder schaufelartigen Öffnungselements (18d) oder über mindestens eine Rolle (18a, 18b).

22. Inhalator nach einem der voranstehenden Ansprüche, **dadurch gekennzeichnet, daß** dem Inhalator (1) ein Kapselreservoir (31) zur Aufnahme mehrerer Kapseln (3) und/oder Kapselkammern (4) zugeordnet, insbesondere in den Inhalator (1) aufnehmbar oder darin gebildet ist.

23. Inhalator (1) zur Inhalation einer Formulierung (2) aus Kapseln (3), die jeweils eine Dosis der Formulierung (2) enthalten, wobei der Inhalator (1) eine Einrichtung, insbesondere eine die Kapseln (3) einzelweise aufnehmende Kapselkammer (4), zur einzelweisen Entleerung der Kapseln (3) bei der Inhalation aufweist und dem Inhalator (1) ein Kapselreservoir (31) zur Aufnahme mehrerer Kapseln (3) zugeordnet ist, das vorzugsweise in den Inhalator (1) aufnehmbar oder darin gebildet ist, insbesondere nach einem der voranstehenden Ansprüche,
**dadurch gekennzeichnet,**
**daß** die Kapseln (3) eine Kette oder ein Band (44) bilden und/oder mäanderförmig, schneckenförmig, umlaufend oder ringförmig angeordnet oder aufnehmbar sind.

24. Inhalator nach Anspruch 22 oder 23, **dadurch gekennzeichnet, daß** die Kapseln (3) nicht miteinander verbunden sind, insbesondere lose entlang einer Schiene oder Führung (20) geführt oder bewegbar sind.

25. Inhalator nach Anspruch 22 oder 23, **dadurch gekennzeichnet, daß** die Kapseln (3) insbesondere fest, beweglich, flexibel, gelenkig, rastend, klemmend, lösbar oder auftrennbar miteinander und/oder mit einem Träger (11) verbunden oder darin aufgenommen sind.

26. Inhalator nach einem der Ansprüche 22 bis 25, **dadurch gekennzeichnet, daß** der Inhalator (1) insbesondere getrennt vom Kapselreservoir (31) mindestens eine Kapselkammer (4) zur Aufnahme der Kapseln (3) zu deren Entleerung bei der Inhalation aufweist.

27. Inhalator nach einem der Ansprüche 22 bis 26, **dadurch gekennzeichnet, daß** das Kapselreservoir (31) für sich oder zusammen mit dem Inhalator (1) insbesondere flüssigkeits- oder gasdicht verschlossen ist und/oder mit einem Trockenmittel versehen ist.

28. Inhalator nach einem der Ansprüche 22 bis 27, **dadurch gekennzeichnet, daß** das Kapselreservoir (31) einzelweise zur Entnahme einzelner Kapseln (3) öffenbar ist und/oder daß das Kapselreservoir (31) nach jeder Entnahme einer Kapsel (3) oder mehrerer Kapseln (3) insbesondere flüssigkeits- oder gasdicht wieder verschließbar ist.

29. Inhalator nach einem der Ansprüche 22 bis 28, **dadurch gekennzeichnet, daß** die Kapseln (3) einzelweise oder gruppenweise vom Kapselreservoir (31) ausgebbar sind.

30. Inhalator nach einem der Ansprüche 22 bis 29, **dadurch gekennzeichnet, daß** das Kapselreservoir (31) eine Schleuse (54) oder sonstige Entnahmeeinrichtung, wie eine Blende (57), einen Schieber oder eine Feder- oder Drehmechanik, zur Ausgabe von Kapseln (3) und/oder temporären Öffnung aufweist.

31. Inhalator nach einem der Ansprüche 22 bis 30, **dadurch gekennzeichnet, daß** das Kapselreservoir (31) magazinartig und/oder rohrförmig ausgebildet, in den Inhalator (1) einsetzbar oder an diesen ankoppelbar, auswechselbar und/oder nachfüllbar ist.

32. Inhalator nach einem der Ansprüche 22 bis 31, **dadurch gekennzeichnet, daß** die Kapseln (3) als Schüttung vom Kapselreservoir (31) aufnehmbar sind.

33. Inhalator nach einem der Ansprüche 22 bis 32, **dadurch gekennzeichnet, daß** der Inhalator (1) länglich oder stabförmig ausgebildet ist und/oder zwei zur Förderung der Kapseln (3) zueinander bewegbare, insbesondere ineinander schiebbare Gehäuseteile (14, 15) aufweist.

34. Inhalator nach einem der Ansprüche 22 bis 34, **dadurch gekennzeichnet, daß** die Kapseln (3) im wesentlichen in einer länglichen, vorzugsweise zweireihigen Anordnung im Inhalator (I) aufnehmbar oder aufgenommen sind, wobei die Längsrichtung der Kapselkammer (4) und/oder der Ausgaberichtung des Inhalators (1) zumindet im wesentlich in Verlängerung oder quer zu der Längsachse der Kapselnordnung, zum Kapselreservoir (31) oder der Transportrichtung der Kapseln (3) verläuft bzw. verlaufen.

35. Inhalator nach einem der voranstehenden Ansprüche, **dadurch gekennzeichnet, daß** die Kapseln (3) einen Ring bilden und/oder ringförmig, insbesondere doppelringförmig, angeordnet sind, insbesondere wobei die Kapseln (3) axial oder radial ausgerichtet oder bewegbar sind.

36. Inhalator nach Anspruch 16, 18 oder 35, **dadurch gekennzeichnet, daß** der Inhalator (1) ein Mundstück (10) aufweist, das quer zur Ringebene, insbesondere in eine Nicht-Gebrauchsstellung in die Mitte, klappbar oder schwenkbar ist.

37. Inhalator nach einem der voranstehenden Ansprüche, **dadurch gekennzeichnet, daß** der Inhalator (1) zur Aufnahme benutzter Kapseln (3) und/oder Kapselkammern (4) ausgebildet ist.

38. Inhalator nach Anspruch 37, **dadurch gekennzeichnet, daß** der Inhalator (1) derart ausgebildet ist, daß benutzte Kapseln (3) bzw. Kapselkammern (4) komprimierbar und/oder zerschneidbar sind.

39. Inhalator nach Anspruch 37 oder 38, **dadurch gekennzeichnet, daß** der Inhalator (1) einen ersten Aufnahmeraum (23) für unbenutzte Kapseln (3) bzw. Kapselkammern (4) und einen zweiten Aufnahmeraum (24) für unbenutzte Kapseln (3) bzw. Kapselkammern (4) oder Teile bzw. Stücke davon aufweist, insbesondere wobei die beiden Aufnahmeräume (23, 24) durch eine vorzugsweise bewegliche, verschiebbare oder verformbare Folie oder Trennwand (42) voneinander getrennt sind.

40. Inhalator nach einem der voranstehenden Ansprüche, **dadurch gekennzeichnet, daß** die Kapseln (3) in einer vorzugsweise geraden Reihe neben- oder hintereinander oder mehrreihig, insbesondere mit zueinander parallelen oder unterschiedlichen Ausrichtungen der Reihen, angeordnet und insbesondere in einem Träger (11) aufgenommen sind.

41. Inhalator nach einem der voranstehenden Ansprüche, **dadurch gekennzeichnet, daß** die Kapseln (3) - gegebenenfalls zusammen mit zugeordneten Kapselkammern (4) - von vorzugsweise gondelartigen Führungselementen (21) aufgenommen und/oder förderbar sind.

42. Inhalator nach einem der voranstehenden Ansprüche, **dadurch gekennzeichnet, daß** die Kapseln (3) oder diesen zugeordnete Kapselkammern (4) oder Führungselemente (21) bewegbar sind, insbesondere in einer Bewegungsrichtung oder Bewegungsebene, vorzugsweise wobei diese von innen, außen, unten oder oben - insbesondere mittels eines Antriebsrads (41) - bewegbar oder antreibbar sind.

43. Inhalator nach einem der voranstehenden Ansprüche, **dadurch gekennzeichnet, daß** der Inhalator (1) zumindest im wesentlichen nierenförmig ausgebildet ist und/oder eine Verjüngung (22) - insbesondere in einem mittleren Gehäusebereich - aufweist.

44. Inhalator nach den Ansprüchen 39 und 43, **dadurch gekennzeichnet, daß** der erste Aufnahmeraum (23) und der zweite Aufnahmeraum (24) über die Verjüngung (22) miteinander verbunden sind und/oder daß zwischen dem ersten Aufnahmeraum (23) und dem zweiten Aufnahmeraum (24) mindestens eine Kapselkammer (4), eine Öffnungseinrichtung (5), ein Mundstück (10) und/oder Befülleinrichtung (25) angeordnet ist bzw. sind.

45. Inhalator nach einem der voranstehenden Ansprüche, **dadurch gekennzeichnet, daß** die Formulierung (2) pulverförmig ist.

46. Inhalator nach einem der voranstehenden Ansprüche, **dadurch gekennzeichnet, daß** der Inhalator (1) tragbar ausgebildet ist.

47. Inhalator nach einem der voranstehenden Ansprüche, **dadurch gekennzeichnet, daß** der Inhalator (1) nur mechanisch arbeitet.

48. Inhalator nach einem der voranstehenden Ansprüche, **dadurch gekennzeichnet, daß** der Inhalator (1) mittels vorzugsweise nur eines Betätigungselements, insbesondere eines Mundstücks (10), und/oder vorzugsweise nur durch Drehen, insbesondere immer in die gleiche Richtung, betätigbar ist.

49. Inhalator nach einem der voranstehenden Ansprüche, **dadurch gekennzeichnet, daß** der Inhalator (1) eine Zähleinrichtung (46) aufweist.
